# EUROPEAN PATENT SPECIFICATION

(11) **EP 1 966 392 B1**
(45) Date of publication and mention of the grant of the patent: **20.11.2013**
(21) Application number: 06835408.3
(22) Date of filing: 26.12.2006
(51) Int. Cl.: C12Q 1/68

(54) **PRIMERS, PROBES, MICROARRAY, AND METHOD FOR SPECIFIC DETECTION OF NINE RESPIRATORY DISEASE-ASSOCIATED BACTERIAL SPECIES**
PRIMER, SONDEN, MIKROARRAY UND VERFAHREN ZUM SPEZIFISCHEN NACHWEIS VON NEUN MIT ATEMWEGSERKRANKUNGEN ASSOZIIERTEN BAKTERIENSPEZIES
AMORCES, SONDES, MICRORESEAU, ET PROCEDE DE DETECTION SPECIFIQUE DE NEUF ESPECES BACTERIENNES ASSOCIEES A DES MALADIES RESPIRATOIRES

(30) Priority: 27.12.2005 KR 20050130610
(43) Date of publication of application: 10.09.2008
(73) Proprietor: Samsung Electronics Co., Ltd., Suwon-si, Gyeonggi-do, 443-742 (KR)
(72) Inventor: OH, Ji-Young, Gyeonggi-do 446-712 (KR); HUH, Nam, Gyeonggi-do 446-712 (KR); PAEK, Sang-Hyun, Gyeonggi-do 446-712 (KR); CHUNG, Jong-Suk, Gyeonggi-do 446-712 (KR); MA, Soo-Min, Gyeonggi-do 446-712 (KR)
(74) Representative: Grünecker, Kinkeldey, Stockmair & Schwanhäusser
(86) International application number: PCT/KR2006/005704
(87) International publication number: WO 2007/075026

(56) References cited:
- WO-A-00/52203
- WO-A1-00/73436
- US-A- 5 681 698
- US-A- 2006 073 454
- DATABASE Geneseq [Online] 4 March 1991 (1991-03-04), "Probe 1337 for Staphylococcus aureus." XP002538793 retrieved from EBI accession no. GSN:AAQ06881 Database accession no. AAQ06881 & WO 90/14444 A (GENE TRAK SYSTEMS [US]) 29 November 1990 (1990-11-29)
- ANTHONY R M ET AL: "Rapid Diagnosis of Bacteremia by Universal Amplification of 23S Ribosomal DNA Followed by Hybridization to an Oligonucleotide Array" JOURNAL OF CLINICAL MICROBIOLOGY, WASHINGTON, DC, US, vol. 38, no. 2, 1 February 2000 (2000-02-01), pages 781-788, XP002176098 ISSN: 0095-1137
- GINEVRA C. ET AL.: 'Development and evaluation of Chlamylege, a New Commercial Test Allowing Simultaneous Detection and Identification of Legionella, Chlamydophila pneumoniae, and Mycoplasma pneumoniae in Clinical Respiratory Specimens by Multiplex PCR' JOURNAL OF CLINICAL MICROBIOLOGY vol. 43, no. 7, July 2005, pages 3247 - 3254, XP003014868
- MADICO G. ET AL.: 'Touchdown enzyme time release-PCR for detection and identification of Chlamydia trachomatis, C. pneumoniae, and C. psittaci using the 16S and 16S-23S spacer rRNA genes' JOURNAL OF CLINICAL MICROBIOLOGY vol. 38, no. 3, March 2000, pages 1085 - 1093, XP002427092
- ANTHONY R.M. ET AL.: 'Rapid diagnosis of Bacteremia by universal amplification of 23S ribosomal DNA followed by hybridization to an oligonucleotide array' JOURNAL OF CLINICAL MICROBIOLOGY vol. 38, no. 2, February 2000, pages 781 - 788, XP002176098

<110> Samsung Electronics Co. Lt d.
<120> A primer set for amplifying t ar get sequences of 9 bacterial species causing respiratory diseases, pr obe set specifically hybridizable with the t ar get sequences of the 9 bacterial speci es, a microarray having immobilized the probe set and a method for detecting the presence of one or more of the 9 bacterial species
<130> PN065016
<160> 94
<170> Kopat ent I n 1. 71
<210> 1
   <211> 20
   <212> DNA
   <213> Artificial Sequence
<220>
   <223> primer S01-F : forward primer for amplifying S01
<400> 1
   gt cat ggct t accaaaccga 20
<210> 2
   <211> 23
   <212> DNA
   <213> Artificial Sequence
<220>
   <223> primer S01-R: r ever se primer f or amplifying SO1
<400> 2
   aat gt t gt ct ct ct t gagt g gat 23
<210> 3
   <211> 23
   <212> DNA.
   <213> Artificial Sequence
<220>
   <223> primer A02- F: forward primer f or amplifying A02
<400> 3
   gcgt acct t t tgtataatgg gt c 23
<210> 4
   <211> 19
   <212> DNA
   <213> Artificial Sequence
<220>
   <223> primer A02- R: r ever se primer f or amplifying A02
<400> 4
   cagaccgcca gct aaggtc 19
<210> 5
   <211> 19
   <212> DNA
   <213> Artificial Sequence
<220>
   <223> primer A07- F: forward primer for amplifying A07
<400> 5
   t gt cgggt aa gt t ccgacc 19
<210> 6
   <211> 19
   <212> DNA
   <213> Artificial Sequence
<220>
   <223> primer A07- R: r ever se primer for amplifying A07
<400> 6
   t agt gat ccg gt ggt t cyg 19
<210> 7
   <211> 19
   <212> DNA
   <213> Artificial Sequence
<220>
   <223> primer A07- R2: reverse primer for amplifying A07
<400> 7
   t ggaagggcc at cgct caa 19
<210> 8
   <211> 20
   <212> DNA
   <213> Artificial Sequence
<220>
   <223> primer A17- F: forward primer for amplifying A17
<400> 8
   cacct cgat g t cgr ct cat c 20
<210> 9
   <211> 21
   <212> DNA
   <213> Artificial Sequence
<220>
   <223> primer A17- R: reverse primer for amplifying A17
<400> 9
   t gyt yct agt acgagaggac c 21
<210> 10
   <211> 25
   <212> DNA
   <213> Artificial Sequence
<220>
   <223> pr obe A17- Chl pneurro- re: pr obe specific c f or A17
<400> 10
   gacagt t t gg t ct ct at cct t t gt g 25
<210> 11
   <211> 23
   <212> DNA
   <213> Artificial Sequence
<220>
   <223> probe A02- Hae. influe specific for A02
<400> 11
   accaaagagt gat act cagg aga 23
<210> 12
   <211> 27
   <212> DNA
   <213> Artificial Sequence
<220>
   <223> pr obe A02- Hae. influe1- re specific f or A02
<400> 12
   aaact acgaa t accaaagag t gat act 27
<210> 13
   <211> 22
   <212> DNA
   <213> Artificial Sequence
<220>
   <223> probe A02- H. inf 2-I specific for A02
<400> 13
   caaact acga at accaaaga gt 22
<210> 14
   <211> 22
   <212> DNA
   <213> Ar t i f i ci al Sequence
<220>
   <223> pr obe A02- H. inf 2-A specific for A02
<400> 14
   caaact gcga ataccgaaga gt 22
<210> 15
   <211> 23
   <212> DNA
   <213> Artificial Sequence
<220>
   <223> probe A07- Hae. influe specific for A07
<400> 15
   t aagcaagct t aact gcgag aca 23
<210> 16
   <211> 23
   <212> DNA
   <213> Artificial Sequence
<220>
   <223> pr obe A07- Kl e. pneurro1 - r e speci f i c for A07
<400> 16
   at aagct agc t t gact gcga gcg 23
<210> 17
   <211> 21
   <212> DNA
   <213> Artificial Sequence
<220>
   <223> pr obe A07- Kl e. pneurro3- re specific f or A07
<400> 17
   gcgt ggacgc cagt ct gcgt g 21
<210> 18
   <211> 21
   <212> DNA
   <213> Artificial Sequence
<220>
   <223> pr obe A07- Kl e. pneurro- re
<400> 18
   gt t ggcccct t accggggt t g 21
<210> 19
   <211> 20
   <212> DNA
   <213> Artificial Sequence
<220>
   <223> probe A02 Leg. pneurro
<400> 19
   gt cat ggct t accaaaccga 20
<210> 20
   <211> 23
   <212> DNA
   <213> Ar t i f i ci al Sequence
<220>
   <223> probe A07- Leg. penurro2
<400> 20
   t t ggt ccagt aat cct ggat gag 23
<210> 21
   <211> 24
   <212> DNA
   <213> Artificial Sequence
<220>
   <223> pr obe A02- Mor - catarr1-re specific f or A02
<400> 21
   ccgaat accg at gagt aat a t ccg 24
<210> 22
   <211> 21
   <212> DNA
   <213> Artificial Sequence
<220>
   <223> probe A02- Mor . cat 2- C specific for A02
<400> 22
   t agggggt ca caccgact t a c 21
<210> 23
   <211> 21
   <212> DNA
   <213> Artificial Sequence
<220>
   <223> probe A02- Mor. cat 2- N specific f or A02
<400> 23
   t agggggt ca t cccgact t a c 21
<210> 24
   <211> 24
   <212> DNA
   <213> Artificial Sequence
<220>
   <223> probe A07- Mor . cat ar r - r e specific c f or A07
<400> 24
   t gt t ggggt t ct aact t agg at ca 24
<210> 25
   <211> 24
   <212> DNA
   <213> Artificial Sequence
<220>
   <223> probe A07-Mor-cat 2- C speci f i c f or A07
<400> 25
   ct aact t agg at caacaaat ccaa 24
<210> 26
   <211> 24
   <212> DNA
   <213> Artificial Sequence
<220>
   <223> pr obe A07- Mor - cat 2- N speci f i c for A07
<400> 26
   ct aact t agg at t aacaaat ccaa 24
<210> 27
   <211> 23
   <212> DNA
   <213> Artificial Sequence
<220>
   <223> probe A17- Myc. pneurro specific c f or A17
<400> 27
   acaggt t ggt ccct at ct at t gt 23
<210> 28
   <211> 20
   <212> DNA
   <213> Artificial Sequence
<220>
   <223> pr obe A02- Pse. aer ugi specific c f or A02
<400> 28
   ccact cccgt t gaaaaggt a 20
<210> 29
   <211> 24
   <212> DNA
   <213> Artificial Sequence
<220>
   <223> probe A07- Pse. aer ugi - r e speci f i c f or A07
<400> 29 .
   gct t gaggt t ct aact ct gg t ccg 24
<210> 30
   <211> 22
   <212> DNA
   <213> Artificial Sequence
<220>
   <223> pr obe S01- St a. aur eu1 speci f i c for S01
<400> 30
   acggagt t ac aaaggacgac at 22
<210> 31
   <211> 22
   <212> DNA
   <213> Artificial Sequence
<220>
   <223> pr obe S01- St a. aur eu2 specific for S01
<400> 31
   aaaggacgac at t agacgaa t c 22
<210> 32
   <211> 20
   <212> DNA
   <213> Artificial Sequence
<220>
   <223> pr obe A07- St r . pneurron2- r e
<400> 32 20
   gacgct gt t g ggat act acc 20
<210> 33
   <211> 20
   <212> DNA
   <213> Artificial Sequence
<220>
   <223> pr obe A07- St a. pneurro3 specf i c f or A07
<400> 33
   gccagtttcg aaggagacgc 20
<210> 34
   <211> 20
   <212> DNA
   <213> Artificial Sequence
<220>
   <223> pr obe A07- St r . pneurro3- O- r,e speci f i c f or A07
<400> 34
   t t t cgaagga gacgt t gt t g 20
<210> 35
   <211> 20
   <212> DNA
   <213> Artificial Sequence
<220>
   <223> pr obe A07- St r. pneurro3- P- r e specific c f or A07
<400> 35
   tttcgaagga gacgctgttg 20
<210> 36
   <211> 1024
   <212> DNA
   <213> Chl arrydophi l a pneurroni ae LKK- 1
<400> 36
   ttacctgcag taatcttcgg tgagctggta tagagctatg acccggaggt atccgaatgg     60
   ggcaacccga tagactaata gtctatcatt atatgttgaa tacataggca tataaggcga    120
   cacccgctga actgaaacat cttagtaagc ggaggaaaag aaatcaaaga gattccctgt    180
   gtagcggcga gcgaaagggg aacagcctaa accatatttt taatatgggg ttgtagggtc    240
   gat aacat gg gat ct t aagt t t t agt t gaa t act t ct gga aagt t gaacg at acagggt g 300
   atagtcccgt aaacgaaaaa acaaaagacg ctaatcgata cctgagtagg gctagacacg    360
   tgaaacctag tctgaatctg gggagaccac tctccaaggc taaatactag tcaatgacct    420
   atagtgaacc agtactgtga aggaaaggtg aaaagaaccc ttgttaaggg agtgaaatag    480
   aacct gaaac cagt agct t a t aagcggt cg gagacct at a act ct t cgga gt aat ggt t g 540
   acggcgtgcc ttttgcatga tgagccaggg agttaagtta aacggcgaga ttaagggatt    600
   t acat t ccgg agt cgaagcg aaagcgagt t t t aaaagagc gt t t t agt cg t t t gat t t ag 660
   acacgaaacc aagtgagcta tttatgacca ggttgaagca ttggtaagac tttgtggagg    720
   accgaaccag tacatgttga aaaatgtttg gatgagttgt gaataggggt gaaaggccaa    780
   t caaact t gg agat at ct t g t t ct ct ccga aat aact t t a gggt t agcct cggat at t aa 840
   gt t t t t gggg gt agagcact gaat t ct agc gggggcct ac cggct t acca acggaaat ca 900
   aactccgaat accaaaagcg agtccgggag atagacagcg ggggctaagc ttcgttgtcg    960
   agaggggaac agcccagacc gccgattaag gtccctaatt ttatgctaag tgagtaagga   1020
   agt g 1024
<210> 37
   <211> 1024
   <212> DNA
   <213> Chl arrydophi l a pneurroni ae TW 183
<400> 37
   t acagaccaa gt t gt t aaga gct at t ggcg gat gcct t gg cat t gacagg cgat gaagga 60
   t gcgt t t acc t gcagt aat c t t cggt gagc t ggt at agag ct at gacccg gaggt at ccg 120
   aat ggggcaa cccgat agac t aat agt ct a t cat t at at g t t gaat acat aggcat at aa 180
   ggcgacaccc gctgaactga aacatcttag taagcggagg aaaagaaatc aaagagattc     240
   cctgtgtagc ggcgagcgaa aggggaacag cctaaaccat atttttaata tggggttgta     300
   gggt cgat aa cat gggat ct t aagt t t t ag t t gaat act t ct ggaaagt t gaacgat aca 360
   gggtgatagt cccgtaaacg aaaaaacaaa agacgctaat cgatacctga gtagggctag     420
   acacgt gaaa cct agt ct ga at ct ggggag accact ct cc aaggct aaat act agt caat 480
   gacct at agt gaaccagt ac t gt gaaggaa aggt gaaaag aaccct t gt t aagggagt ga 540
   aat agaacct gaaaccagt a gct t at aagc ggt cggagac ct at aact ct t cggagt aat 600
   ggt t gacggc gt gcct t t t g cat gat gagc cagggagt t a agt t aaacgg cgagat t aag 660
   ggatttacat tccggagtcg aagcgaaagc gagttttaaa agagcgtttt agtcgtttga     720
   t t t agacacg aaaccaagt g agct at t t at gaccaggt t g aagcat t ggt aagact t t gt 780
   ggaggaccga accagtacat gttgaaaaat gtttggatga gttgtgaata ggggtgaaag     840
   gccaat caaa ct t ggagat a t ct t gt t ct c t ccgaaat aa ct t t agggt t agcct cggat 900
   at t aagt t t t t gggggt aga gcact gaat t ct agcggggg cct accggct t accaacgga 960
   aatcaaactc cgaataccaa aagcgagtcc gggagataga cagcgggggc taagcttcgt    1020
   t gt c 1024
<210> 38
   <211> 1024
   <212> DNA
   <213> Haerrophilus influenzae
<400> 38
   ct gcgaaagc t t ggat gagt cgat nagagg cgt t t aat cc nagat gt ccg aat ggggaaa 60
   cccagttggg tgaagaaccc actatcatta tctgaatcca taggataatg aggcgaaccg 120
   ggagaact ga aacat ct aag t accccgagg aaaagaaat c aaccgagat t t cgt t agt ag 180
   cggcgagcga acgcgaagga gcctgttagt gataatgaca gagacagagg aacaagctgg 240
   gaagcttggc gacacagggt gatagccccg tactcgaant ccaggttatg gtactaagct 300
   aacgacaagt aaggcgggac acgtgatatc ctgtttgaag atggggggac catcctccaa 360
   ggctaaatac tcctgattga ccgatagtga accagtactg tgaaggaaag gcgaaaagaa 420
   ccccggt gag gggagt gaaa t agaacct ga aacct t gt ac gt acaagcag t gggagcct g 480
   aaagggt gac t gcgt acct t t t gt at aat g ggt cagcgac t t at at t t t g t agcgaggt t 540
   aaccgaatag gggagccgaa gggaaaccga gtcttaactg ggcgagtagt tgcaaggtat 600
   agacccgaaa cccggtgatc tagccatggg caggttgaag gttgggtaac actaactgga 660
   ggaccgaacc gactaatgtt gaaaaattag cggatgactt gtggctgggg gtgaaaggcc 720
   aat caaaccg ggagat agct ggt t ct cccc gaaat ct at t t aggt agagc ct t gagt gga 780
   cacct t cggg ggt agagcac t gt t t cggct aggggt ccat cccggat t ac caacccgat g 840
   caaactgcga ataccgaaga gtgatactca ggagacacac ggcgggtgct aacgttcgtc 900
   gtggagaggg aaacaaccca gaccgccagc taaggtccca aagtctatat taagtgggaa 960
   acgaagtggg aaggcttaga cagctaggat gttggcttag aagcagccat catttaaaga 1020
   aagc 1024
<210> 39
   <211> 1024
   <212> DNA
   <213> Haerrophilus influenzae
<400> 39
   ct at ct gcga aaagct t gga t gagt cgat a agaggcgt t t aat ccaagat at ccgaat gg 60
   ggaaacccag tagatgaaga atctactatc aacaagtgaa ttcatagctt gttgaggcaa 120
   accgggagaa ctgaaacatc taagtacccc gaggaaaaga aatcaaccga gatttcgtca      180
   gtagtggcga gcgaaagcga aagagccagt aagtgatagc agtatagtta ggagaatgtg      240
   t t gggaaaca caat caaaga gggt gat aat cccgt at ct t aaaaccat at t gt ggt act a 300
   agctaacgag aagtagggcg ggacacgtga tatcctgttt gaagaagggg ggaccatcct      360
   ccaaggctaa atactcctga ttgaccgata gtgaaccagt actgtgaagg aaaggcgaaa      420
   agaaccccgg tgaggggagt gaaatagaac ctgaaacctt gtacgtacaa gcagtgggag      480
   ccccat cact t aat ccgat t ggaaagt gat gcggaaat ag agcgcact t t aaggt aaggc 540
   gaacgaagtg agacaaacct tcccaaaaaa aagcagtggg attaagtgat ggggtgactg      600
   cgt acct t t t gt at aat ggg t cagcgact t at at t t t gt a gcgaggt t aa ccgaat aggg 660
   gagccgaagg gaaaccgagt cttaactggg cgaagagttg caaggtatag acccgaaacc      720
   cggtgatcta gccatgggca ggttgaaggt tgggtaacac taactggagg accgaaccga      780
   ctaatgttga aaattagcgg gatgacttgt ggctgggggt gaaaggccaa tcaaaccggg    840
   agat agct gg t t ct ccccga aat ct at t t a ggt agagcct t gaggt gaca cct t t ggggg 900
   t agagcact g t t t cggct ag gggt ccat cc cggat t acca acccgat gca aact acgaat 960
   accaaagagt gatactcagg agacacacgg cgggtgctaa cgtccgtcgt ggagagggaa     1020
   acaa 1024
<210> 40
   <211> 1024
   <212> DNA
   <213> Haerrophilus influenzae
<400> 40
   ct at ct gcga aaagct t gga t gagt cgat a agaggcgt t t aat ccaagat at ccgaat gg 60
   ggaaacccag tagatgaaga atctactatc aacaagtgaa ttcatagctt gttgaggcaa      120
   accgggagaa ctgaaacatc taagtacccc gaggaaaaga aatcaaccga gatttcgtca     180
   gtagcggcga gcgaaagcga aagagccagt aagtgatagc agtatagtga ggagaatgtg      240
   t t gggaagca caat caaaga gggt gat aat cccgt at ct a aaaaccat at t gt ggt act a 300
   agctaacgag aagtagggcg ggacacgtga tatcctgttt gaagaagggg ggaccatcct      360
   ccaaggctaa atactcctga ttgaccgata gtgaaccagt actgtgaagg aaaggcgaaa      420
   agaaccccgg tgaggggagt gaaatagaac ctgaaacctt gtacgtacaa gcagtgggag      480
   ccccatcact taatccgatt ggaaggtgat gaaaagcaac cgcacttaaa caatggtaag      540
   aggagcgaag cgagtcaaac cacccaaaag aagcagtggg attaagtgat ggggtgactg      600
   cgt acct t t t gt at aat ggg t cagcgact t at at t t t gt a gcgaggt t aa ccgaat aggg 660
   gagccgaagg gaaaccgagt cttaactggg cgaatagttg caaggtatag acccgaaacc      720
   cggtgatcta gccatgggca ggttgaaggt tgggtaacac taactggagg accgaaccga      780
   ctaatgttga aaattagcgg gatgacttgt ggctgggggt gaaaggccaa tcaaaccggg    840
   agat agct gg t t ct ccccga aat ct at t t a ggt agagcct t gaggt gaca cct t t ggggg 900
   t agagcact g t t t cggct ag gggt ccat cc cggat t acca acccgat gca aact acgaat 960
   accaaagagt gatactcagg agacacacgg cgggtgctaa cgtccgtcgt ggagagggaa     1020
   acaa 1024
<210> 41
   <211> 1024
   <212> DNA
   <213> Haerrophi l us i nf l uenzae
<400> 41
   ctatctgcga aaagcttgga tgagtcgata agaggcgttt aatccaagat atccgaatgg 60
   ggaaacccag tagatgaaga atctactatc aacaagtgaa ttcatagctt gttgaggcaa 120
   accgggagaa ctgaaacatc taagtacccc gaggaaaaga aatcaaccga gatttcgtca 180
   gtagcggcga gcgaaagcga aagagccagt aagtgatagc agtatagtga ggagaatgtg 240
   ttgggaagca caatcaaaga gggtgataat cccgtatcta aaaaccatat tgtggtacta 300
   agctaacgag aagtagggcg ggacacgtga tatcctgttt gaagaagggg ggcccatcct 360
   ccaaggctaa atactcctga ttgaccgata gtgaaccagt actgtgaagg aaaggcgaaa 420
   agaaccccgg tgaggggagt gaaatagaac ctgaaacctt gtacgtacaa gcagtgggag 480
   ccccatcact taatccgatt ggaaggtgat gaaaagcaac cgcacttaaa caatggtaag 540
   aggagcgaag cgagtcaaac cacccaaaag aagcagtggg attaagtgat ggggtgactg 600
   cgtacctttt gtataatggg tcagcgactt atattttgta gcgaggttaa ctgaataagg 660
   gagccgaagg gaaaccgagt cttaactggg cgaagagttg caaggtatag acccgaaacc 720
   cggtgatcta gccatgggca ggttgaaggt tgggtaacac taactggagg accgaaccga 780
   ctaatgttga aaaattagcg gatgacttgt ggctgggggt gaaaggccaa tcaaaccggg 840
   agatagctgg ttctccccga aatctattta ggtagagcct tgagcttaca cctttggggg 900
   tagagcactg tttcggctag gggtccatcc cggattacca acccgatgca aactacgaat 960
   accaaagagt gatactcagg agacacacgg cgggtgctaa cgtccgtcgt ggagagggaa 1020
   acaa 1024
<210> 42
   <211> 1024
   <212> DNA
   <213> Haerrophilus influenzae
<400> 42
   ct at ct gcga aaagct t ggg t gagt cgat a agaggcgt t t aat ccaagat at ccgaat gg 60
   ggaaacccag tagatgaaga atctactatc acttactgaa tccataggta agtgaggcaa     120
   accgggagaa ctgaaacatc taagtacccc gaggaaaaga aatcaaccga gatttcgtca     180
   gtagcggcga gcgaaagcga aagagcctgt taatgataac agcagagata gaggaataag     240
   ct gggaagct t agcgacaca gggt gat agc cccgt act cg acat ccaggc t gt ggt act a 300
   agttaacgag aagtagggcg ggacacgtga tatcctgttt gaagaagggg ggaccatcct     360
   ccaaggctaa atactcctga ttgaccgata gtgaaccagt actgtgaagg aaaggcgaaa     420
   agaaccccgg tgaggggagt gaaatagaac ctgaaacctt gtacgtacaa gcagtgggag     480
   cct gaaaggg t gact gcgt a cct t t t gt at aat gggt cag cgact t at at t t t gt agcga 540
   ggttaaccga ataggggagc caaagggaaa ccgagtctta actgggcgac tagttgcaag     600
   gtatagaccc gaaacccggt gatctagcca tgggcaggtt gaaggttggg taacactaac     660
   t ggaggaccg aaccgact aa t gt t gaaaaa t t agcggat g act t gt ggct gggggt gaaa 720
   ggccaatcaa accgggagat agctggttct ccccgaaatc tatttaggta gagccttgag     780
   cggacacct t t gggggt aga gcact gt t t c ggct aggggt ccat cccgga t t accaaccc 840
   gatgcaaact acgaatacca aagagtgata ctcaggagac acacggcggg tgctaacgtc     900
   cgtcgtggag agggaaacaa cccagaccgc cagctaaggt cccaaagtct atattaagtg     960
   ggaaacgaag tgggaaggct tagacagcta ggatgttggc ttagaagcag ccatcattta    1020
   aaga 1024
<210> 43
   <211> 1024
   <212> DNA
   <213> Haerrophilus influenzae
<400> 43
   ct at ct gcga aaagct t gga t gagt cgat a agaggcgt t t aat ccgagat at ccgaat gg 60
   ggaaacccag tagatgaaga atctactatc aacaagtgaa ttcatagctt gttgaggcaa     120
   accgggagaa ctgaaacatc taagtacccc gaggaaaaga aatcaaccga gatttcgtca    180
   gtagcggcga gcgaaagcga aagagccagt aagtgatagc agtatagtga ggagaatgtg     240
   t t gggaagca caat caaaga gggt gat aat cccgt at ct a aaaaccat at t gt ggt act a 300
   agctaacgag aagtagggcg ggacacgtga tatcctgttt gaagaagggg ggaccatcct     360
   ccaaggctaa atactcctga ttgaccgata gtgaaccagt actgtgaagg aaaggcgaaa     420
   agaaccccgg tgaggggagt gaaatagaac ctgaaacctt gtacgtacaa gcagtgggag     480
   ccccatcact taatccgatt ggaaggtgat gaaaagcaac cgcacttaaa caatggtaag     540
   aggagcgaag cgagtcaaac cacccaaaag aagcagtggg attaagtgat ggggtgactg     600
   cgt acct t t t gt at aat ggg t cagcgact t at at t t t gt a gcgaggt t aa ccgaat aggg 660
   gagccgaagg gaaaccgagt cttaactggg cgaatagttg caaggtatag acccgaaacc     720
   cggtgatcta gccatgggca ggttgaaggt tgggtaacac taactggagg accgaaccga     780
   ctaatgttga aaaattagcg gatgacttgt ggctgggggt gaaaggccaa tcaaaccggg    840
   agat agct gg t t ct ccccga aat ct at t t a ggt agagcct t gaggt gaca cct t t ggggg 900
   t agagcact g t t t cggct ag gggt ccat cc cggat t acca acccgat gca aact acgaat 960
   accaaagagt gatactcagg agacacacgg cgggtgctaa cgtccgtcgt ggagagggaa    1020
   acaa 1024
<210> 44
   <211> 1024
   <212> DNA
   <213> Haerrophilus influenzae
<400> 44
   aggggct at c t gcgaaaagc t t ggat gagt cgat aagagg cgt t t aat cc aagat at ccg 60
   aat ggggaaa cccagt agat gaagaat ct a ct at caacaa gt gaat t cat agct t gt t ga 120
   ggcaaaccgg gagaactgaa acatctaagt accccgagga aaagaaatca accgagattt 180
   cgtcagtagc ggcgagcgaa agcgaaagag ccagtaagtg atagcagtat agtgaggaga     240
   at gt gt t ggg aagcacaat c aaagagggt g at aat cccgt at ct aaaaac cat at t gt gg 300
   tactaagcta acgagaagta gggcgggaca cgtgatatcc tgtttgaaga aggggggacc     360
   atcctccaag gctaaatact cctgattgac cgatagtgaa ccagtactgt gaaggaaagg     420
   cgaaaagaac cccggtgagg ggagtgaaat agaacctgaa accttgtacg tacaagcagt     480
   gggagcccca tcacttaatc cgattggaag gtgatgaaaa gcaaccgcac ttaaacaatg     540
   gtaagaggag cgaagcgagt caaaccaccc aaaagaagca gtgggattaa gtgatggggt     600
   gactgcgtac cttttgtata atgggtcagc gacttatatt ttgtagcgag gttaaccgaa     660
   taggggagcc gaagggaaac cgagtcttaa ctgggcgaat agttgcaagg tatagacccg     720
   aaacccggtg atctagccat gggcaggttg aaggttgggt aacactaact ggaggaccga     780
   accgact aat gt t gaaaaat t agcggat ga ct t gt ggct g ggggt gaaag gccaat caaa 840
   ccgggagat a gct ggt t ct c cccgaaat ct at t t aggt ag agcct t gagg t gacacct t t 900
   gggggt agag cact gt t t cg gct aggggt c cat cccggat t accaacccg at gcaaact a 960
   cgaataccaa agagtgatac tcaggagaca cacggcgggt gctaacgtcc gtcgtggaga    1020
   ggga 1024
<210> 45
   <211> 1024
   <212> DNA
   <213> Haerrophilus influenzae
<400> 45
   ct aat ct gcg aaaagct t gg at gagt cgat aagaggcgt t t aat ccaaga t at ccgaat g 60
   gggaaaccca gtagatgaag aatctactat caacaagtga attcatagct tgttgaggca     120
   aaccgggaga actgaaacat ctaagtaccc cgaggaaaag aaatcaaccg agatttcgtc     180
   agtagcggcg agcgaaagcg aaagagccag taagtgatag cagtatagtt aggagaatgt     240
   gt t gggaagc acaat caaag agggt gat aa t cccgt at ct aaaaaccat a t t gt ggt act 300
   aagct aacga gaagt agggc gggacacgt g at at cct gt t t gaagaaggg gggaccat cc 360
   tccaaggcta aatactcctg attgaccgat agtgaaccag tactgtgaag gaaaggcgaa     420
   aagaaccccg gtgaggggag tgaaatagaa cctgaaacct tgtacgtaca agcagtggga     480
   gccccat cac t t aat ccgat t ggaaggt ga t gaaaagcaa ccgcact t aa acaat ggt aa 540
   gaggagcgaa gcgagtcaaa ccacccaaaa gaagcagtgg gattaagtga tggggtgact     600
   gcgt acct t t t gt at aat gg gt cagcgact t at at t t t gt agcgaggt t a accgaat agg 660
   ggagccgaag ggaaaccgag tcttaactgg gcgagtagtt gcaaggtata gacccgaaac     720
   ccggtgatct agccatgggc aggttgaagg ttgggtaaca ctaactggag gaccgaaccg     780
   act aat gt t g aaaaat t agc ggat gact t g t ggct ggggg t gaaaggcca at caaaccgg 840
   gagat agct g gt t ct ccccg aaat ct at t t aggt agagcc t t gaggt gac acct t t gggg 900
   gt agagcact gt t t cggct a gggggccat c ccggct t acc aacccgat gc aaact acgaa 960
   taccaaagag tgatactcag gagacacacg gcgggtgcta acgtccgtcg tggagaggga    1020
   aaca 1024
<210> 46
   <211> 1024
   <212> DNA
   <213> Mycopl asrra pneurroni ae
<400> 46
   t t acct gcga t agct t cggg t aggt ggt aa gaacct caga t ccggagat t t ccgaat gga 60
   gcaat ccggt agt t ggaaac agct at cat t aat t gat gaa t aaat agt ca t at t aaagca 120
   atacgtggtg aagtgaaaca tctcagtagc cacaggaaaa gaaaacgaat gtgattccgt     180
   gtgtagtggc gagcgaaagc ggaacaggcc aaacttatca ttagataggg gttgtagggc     240
   ttgcaatgtg gacttgaaaa cgatagaaga agctgttgga aagcagcgcg caaaagggtg     300
   at agccccgt at t t gaaat t gt t t t cat ac ct agcgagat ccct gagt ag ct cggaaaac 360
   gt t at t t t ga gt gaat ct gc ccagaccat t gggt aagcct aaat act aat t agt gaccga 420
   tagcgaaaca gtaccgtgag ggaaaggtga aaagaaccca gagatgggag tgaaatagat     480
   t ct gaaacca t at gcct aca acgt gt caga gcacat t aat gt gt gat ggc gt gcgt t t t g 540
   aagt at gagc cggcgagt t a t gat agcaag cgt t agt t aa ccaggagat g gggagct gt a 600
   gcgaaagcga gttttaaaag agcgtttgtt tgttattata gacccgaaac gggttgagct     660
   agtcatgagc aggttgaagg ttgagtaaca tcaactggag gaccgaaccg actctcgttg     720
   aaacgat agc ggat gact t g t gat t agggg t gaaat t cca at cgaaat cc gt gat agct g 780
   gt t ct cgt cg aaat agct t t aaggct agcg t gagat caca aat aagt gga ggt aaagct a 840
   ct gaat gt at gat ggcgcca cct aggcgt a ct gaat acaa t t aaact ct g aat gccat t t 900
   attttattct cgcagtcaga cagtggggga taagcttcat tgtcaagagg ggaagagccc     960
   agat cat t aa at aaggt ccc caaaat at ac t aagt ggaaa aggat gt gaa agt gct aaaa 1020
   cagc 1024
<210> 47
   <211> 1024
   <212> DNA
   <213> Kl ebsi el l a pneurroni ae
<400> 47
   ctatctgcga aaagcgtcgg taggtgatat gaaccgttat aaccggcgat gtccgaatgg      60
   ggaaacccag tgcaattcgt tgcactatcg ttaactgaat acataggtta acgaggcgaa     120
   ccgggggaac tgaaacatct aagtaccccg aggaaaagaa atcaaccgag attcccccag     180
   tagcggcgag cgaacgggga gcagcccaga gtctgaatca gcttgtgtgt tagtggaacg     240
   gt ct ggaaag t ccgacggt a cagggt gat a gt cccgt aca ccaaaat gca caggt t gt ga 300
   act cgaagag t agggcggga cacgt ggt at cct gt ct gaa t at gggggga ccat cct cca 360
   aggctaaata ctcctgactg accgatagtg aaccagtacc gtgagggaaa ggcgaaaaga     420
   accccggcga ggggagtgaa aaagaacctg aaaccgtgta cgtacaagca gtgggagcac     480
   ct t cgggt gt gact gcgt ac ct t t t gt at a at gggt cagc gact t at at t ct gt agcaag 540
   gttaaccgta taggggagcc gcagggaaac cgagtcttaa ctgggcgtta agttgcaggg     600
   t at agacccg aaacccggt g at ct agccat gggcaggt t g aaggt t gggt aacact aact 660
   ggaggaccga accgactaat gttgaaaaat tagcggatga cttgtggctg ggggtgaaag     720
   gccaatcaaa ccgggagata gctggttctc cccgaaagct atttaggtag cgcctcgtga     780
   at t cat ct t c gggggt agag cact gt t t cg gct agggggt cat cccgact t accaacccg 840
   atgcaaacta cgaataccga agaatgttat cacgggagac acacggcggg tgctaacgtc     900
   cgtcgtgaag agggaaacaa cccagaccgc cagctaaggt cccaaagtca tggttaagtg     960
   ggaaacgatg tgggaaggca cagacagcca ggatgttggc ttagaagcag ccatcattta    1020
   aaga 1024
<210> 48
   <211> 1024
   <212> DNA
   <213> Klebsiella pneumoniae
<400> 48
   ctatctgcga aaagcgtcgg taggtgatat gaaccgttat aaccggcgat gtccgaatgg      60
   ggaaacccag tgcaattcgt tgcactatcg ttaactgaat acataggtta acgaggcgaa     120
   ccgggggaac tgaaacatct aagtaccccg aggaaaagaa atcaaccgag attcccccag     180
   tagcggcgag cgaacgggga gcagcccaga gtctgaatca gcttgtgtgt tagtggaacg     240
   gt ct ggaaag t ccgacggt a cagggt gat a gt cccgt aca ccaaaat gca caggt t gt ga 300
   act cgaagag t agggcggga cacgt ggt at cct gt ct gaa t at gggggga ccat cct cca 360
   aggctaaata ctcctgactg accgatagtg aaccagtacc gtgagggaaa ggcgaaaaga     420
   accccggcga ggggagtgaa aaagaacctg aaaccgtgta cgtacaagca gtgggagcac     480
   ct t cgggt gt gact gcgt ac ct t t t gt at a at gggt cagc gact t at at t ct gt agcaag 540
   gttaaccgta taggggagcc gcagggaaac cgagtcttaa ctgggcgtta agttgcaggg     600
   t at agacccg aaacccggt g at ct agccat gggcaggt t g aaggt t gggt aacact aact 660
   ggaggaccga accgactaat gttgaaaaat tagcggatga cttgtggctg ggggtgaaag     720
   gccaatcaaa ccgggagata gctggttctc cccgaaagct atttaggtag cgcctcgtga     780
   act cat ct t c gggggt agag cact gt t t cg gct agggggt cat cccgact t accaacccg 840
   atgcaaacta cgaataccga agaatgttat cacgggagac acacggcggg tgctaacgtc     900
   cgtcgtgaag agggaaacaa cccagaccgc cagctaaggt cccaaagtca tggttaagtg     960
   ggaaacgatg tgggaaggca cagacagcca ggatgttggc ttagaagcag ccatcattta    1020
   aaga 1024
<210> 49
   <211> 1024
   <212> DNA
   <213> Klebsiella pneumoniae
<400> 49
   ctatctgcga aaagcgtcgg taggtgatat gaaccgttat aaccggcgat gtccgaatgg     60
   ggaaacccag tgcaattcgt tgcactatcg ttaactgaat acataggtta acgaggcgaa    120
   ccgggggaac tgaaacatct aagtaccccg aggaaaagaa atcaaccgag attcccccag    180
   tagcggcgag cgaacgggga gcagcccaga gtctgaatca gcttgtgtgt tagtggaacg    240
   gtctggaaag tccgacggta cagggtgata gtcccgtaca ccaaaatgca caggttgtga    300
   actcgaagag tagggcggga cacgtggtat cctgtctgaa tatgggggga ccatcctcca    360
   aggctaaata ctcctgactg accgatagtg aaccagtacc gtgagggaaa ggcgaaaaga    420
   accccggcga ggggagtgaa aaagaacctg aaaccgtgta cgtacaagca gtgggagcac    480
   cttcgggtgt gactgcgtac cttttgtata atgggtcagc gacttatatt ctgtagcaag    540
   gttaaccgta taggggagcc gcagggaaac cgagtcttaa ctgggcgtta agttgcaggg    600
   tatagacccg aaacccggtg atctagccat gggcaggttg aaggttgggt aacactaact    660
   ggaggaccga accgactaat gttgaaaaat tagcggatga cttgtggctg ggggtgaaag    720
   gccaatcaaa ccgggagata gctggttctc cccgaaagct atttaggtag cgcctcgtga    780
   actcatcttc gggggtagag cactgtttcg gctagggggt catcccgact taccaacccg    840
   atgcaaacta cgaataccga agaatgttat cacgggagac acacggcggg tgctaacgtc    900
   cgtcgtgaag agggaaacaa cccagaccgc cagctaaggt cccaaagtca tggttaagtg    960
   ggaaacgatg tgggaaggca cagacagcca ggatgttggc ttagaagcag ccatcattta   1020
   aaga                                                                1024
<210> 50
   <211> 1024
   <212> DNA
   <213> Klebsiella pneumoniae
<400> 50
   ctatctgcga aaagcgtcgg taggtgatat gaaccgttat aaccggcgat gtccgaatgg     60
   ggaaacccag tgcaattcgt tgcactatcg ttaactgaat acataggtta acgaggcgaa    120
   ccgggggaac tgaaacatct aagtaccccg aggaaaagaa atcaaccgag attcccccag    180
   tagcggcgag cgaacgggga gcagcccaga gtctgaatca gcttgtgtgt tagtggaacg    240
   gt ct ggaaag t ccgacggt a cagggt gat a gt cccgt aca ccaaaat gca caggt t gt ga 300
   actcgaagag tagggcggga cacgtggtat cctgtctgaa tatgggggga ccatcctcca    360
   aggctaaata ctcctgactg accgatagtg aaccagtacc gtgagggaaa ggcgaaaaga    420
   accccggcga ggggagtgaa aaagaacctg aaaccgtgta cgtacaagca gtgggagcac    480
   ct t cgggt gt gact gcgt ac ct t t t gt at a at gggt cagc gact t at at t ct gt agcaag 540
   gttaaccgta taggggagcc gcagggaaac cgagtcttaa ctgggcgtta agttgcaggg    600
   t at agacccg aaacccggt g at ct agccat gggcaggt t g aaggt t gggt aacact aact 660
   ggaggaccga accgactaat gttgaaaaat tagcggatga cttgtggctg ggggtgaaag    720
   gccaatcaaa ccgggagata gctggttctc cccgaaagct atttaggtag cgcctcgtga    780
   act cat ct t c gggggt agag cact gt t t cg gct agggggt cat cccgact t accaacccg 840
   atgcaaacta cgaataccga agaatgttat cacgggagac acacggcggg tgctaacgtc    900
   cgtcgtgaag agggaaacaa cccagaccgc cagctaaggt cccaaagtca tggttaagtg    960
   ggaaacgatg tgggaaggca cagacagcca ggatgttggc ttagaagcag ccatcattta   1020
   aaga 1024
<210> 51
   <211> 1024
   <212> DNA
   <213> Kl ebsiella pneumoniae
<400> 51
   ctatctgcga aaagcgtcgg taggtgatat gaaccgttat aaccggcgat gtccgaatgg      60
   ggaaacccag tgcaattcgt tgcactatcg ttaactgaat acataggtta acgaggcgaa     120
   ccgggggaac tgaaacatct aagtaccccg aggaaaagaa atcaaccgag attcccccag     180
   tagcggcgag cgaacgggga gcagcccaga gtctgaatca gcttgtgtgt tagtggaacg     240
   gtctggaaag tccgacggta cagggtgata gtcccgtaca ccaaaatgca caggttgtga     300
   actcgaagag tagggcggga cacgtggtat cctgtctgaa tatgggggga ccatcctcca     360
   aggctaaata ctcctgactg accgatagtg aaccagtacc gtgagggaaa ggcgaaaaga     420
   accccggcga ggggagtgaa aaagaacctg aaaccgtgta cgtacaagca gtgggagcac     480
   cttcgggtgt gactgcgtac cttttgtata atgggtcagc gacttatatt ctgtagcaag     540
   gttaaccgta taggggagcc gcagggaaac cgagtcttaa ctgggcgtta agttgcaggg     600
   tatagacccg aaacccggtg atctagccat gggcaggttg aaggttgggt aacactaact     660
   ggaggaccga accgactaat gttgaaaaat tagcggatga cttgtggctg ggggtgaaag     720
   gccaatcaaa ccgggagata gctggttctc cccgaaagct atttaggtag cgcctcgtga     780
   attcatcttc gggggtagag cactgtttcg gctagggggt catcccgact taccaacccg     840
   atgcaaacta cgaataccga agaatgttat cacgggagac acacggcggg tgctaacgtc     900
   cgtcgtgaag agggaaacaa cccagaccgc cagctaaggt cccaaagtca tggttaagtg     960
   ggaaacgatg tgggaaggca cagacagcca ggatgttggc ttagaagcag ccatcattta    1020
   aaga                                                                 1024
<210> 52
   <211> 1024
   <212> DNA
   <213> Kl ebsi el l a pneurroni ae
<400> 52
   ctatctgcga aaagcgtcgg taggtgatat gaaccgttac aaccggcgat gtccgaatgg      60
   ggaaacccag tgcaattcgt tgcactatcg ttaactgaat acataggtta acgaggcgaa     120
   ccgggggaac tgaaacatct aagtaccccg aggaaaagaa atcaaccgag attcccccag     180
   tagcggcgag cgaacgggga gcagcccaga gtctgaatca gcttgtgtgt tagtggaacg     240
   gtctggaaag tccgacggta cagggtgata gtcccgtaca ccaaaatgca caggctgtga     300
   act cgaagag t agggcggga cacgt ggt at cct gt ct gaa t at gggggga ccat cct cca 360
   aggctaaata ctcctgactg accgatagtg aaccagtacc gtgagggaaa ggcgaaaaga      420
   accccggcga ggggagtgaa aaagaacctg aaaccgtgta cgtacaagca gtgggagcac      480
   ct t cgggt gt gact gcgt ac ct t t t gt at a at gggt cagc gact t at at t ct gt agcaag 540
   gttaaccgta taggggagcc gcagggaaac cgagtcttaa ctgggcgtta agttgcaggg      600
   t at agacccg aaacccggt g at ct agccat gggcaggt t g aaggt t gggt aacact aact 660
   ggaggaccga accgactaat gttgaaaaat tagcggatga cttgtggctg ggggtgaaag      720
   gccaatcaaa ccgggagata gctggttctc cccgaaagct atttaggtag cgcctcgtga      780
   act cat ct t c gggggt agag cact gt t t cg gct agggggt cat cccgact t accaacccg 840
   atgcaaacta cgaataccga agaatgttat cacgggagac acacggcggg tgctaacgtc      900
   cgtcgtgaag agggaaacaa cccagaccgc cagctaaggt cccaaagtca tggttaagtg      960
   ggaaacgatg tgggaaggca cagacagcca ggatgttggc ttagaagcag ccatcattta     1020
   aaga 1024
<210> 53
   <211> 1024
   <212> DNA
   <213> Kl ebsiella pneumoniae
<400> 53
   ctatctgcga aaagcgtcgg taggtgatat gaaccgttat aaccggcgat gtccgaatgg       60
   ggaaacccag tgcaattcgt tgcactatcg ttaactgaat acataggtta acgaggcgaa      120
   ccgggggaac tgaaacatct aagtaccccg aggaaaagaa atcaaccgag attcccccag      180
   tagcggcgag cgaacgggga gcagcccaga gtctgaatca gcttgtgtgt tagtggaacg      240
   gt ct ggaaag t ccgacggt a cagggt gat a gt cccgt aca ccaaaat gca caggct gt ga 300
   act cgaagag t agggcggga cacgt ggt at cct gt ct gaa t at gggggga ccat cct cca 360
   aggctaaata ctcctgactg accgatagtg aaccagtacc gtgagggaaa ggcgaaaaga      420
   accccggcga ggggagtgaa aaagaacctg aaaccgtgta cgtacaagca gtgggagcac      480
   ct t cgggt gt gact gcgt ac ct t t t gt at a at gggt cagc gact t at at t ct gt agcaag 540
   gttaaccgta taggggagcc gcagggaaac cgagtcttaa ctgggcgtta agttgcaggg      600
   t at agacccg aaacccggt g at ct agccat gggcaggt t g aaggt t gggt aacact aact 660
   ggaggaccga accgactaat gttgaaaaat tagcggatga cttgtggctg ggggtgaaag      720
   gccaatcaaa ccgggagata gctggttctc cccgaaagct atttaggtag cgcctcgtga      780
   act cat ct t c gggggt agag cact gt t t cg gct agggggt cat cccgact t accaacccg 840
   atgcaaacta cgaataccga agaatgttat cacgggagac acacggcggg tgctaacgtc      900
   cgtcgtgaag agggaaacaa cccagaccgc cagctaaggt cccaaagtca tggttaagtg      960
   ggaaacgatg tgggaaggca cagacagcca ggatgttggc ttagaagcag ccatcattta     1020
   aaga 1024
<210> 54
   <211> 1024
   <212> DNA
   <213> Kl ebsiella pneumoniae
<400> 54
   ctatctgcga aaagcgtcgg taggtgatat gaaccgttat aaccggcgat gtccgaatgg     60
   ggaaacccag tgcaattcgt tgcactatcg ttaactgaat acataggtta acgaggcgaa    120
   ccgggggaac tgaaacatct aagtaccccg aggaaaagaa atcaaccgag attcccccag    180
   tagcggcgag cgaacgggga gcagcccaga gtctgaatca gcttgtgtgt tagtggaacg    240
   gtctggaaag tccgacggta cagggtgata gtcccgtaca ccaaaatgca caggttgtga    300
   actcgaagag tagggcggga cacgtggtat cctgtctgaa tatgggggga ccatcctcca    360
   aggctaaata ctcctgactg accgatagtg aaccagtacc gtgagggaaa ggcgaaaaga    420
   accccggcga ggggagtgaa aaagaacctg aaaccgtgta cgtacaagca gtgggagcac    480
   cttcgggtgt gactgcgtac cttttgtata atgggtcagc gacttatatt ctgtagcaag    540
   gttaaccgta taggggagcc gcagggaaac cgagtcttaa ctgggcgtta agttgcaggg    600
   tatagacccg aaacccggtg atctagccat gggcaggttg aaggttgggt aacactaact    660
   ggaggaccga accgactaat gttgaaaaat tagcggatga cttgtggctg ggggtgaaag     720
   gccaatcaaa ccgggagata gctggttctc cccgaaagct atttaggtag cgcctcgtga    780
   attcatcttc gggggtagag cactgtttcg gctagggggt catcccgact taccaacccg    840
   atgcaaacta cgaataccga agaatgttat cacgggagac acacggcggg tgctaacgtc    900
   cgtcgtgaag agggaaacaa cccagaccgc cagctaaggt cccaaagtca tggttaagtg    960
   ggaaacgatg tgggaaggca cagacagcca ggatgttggc ttagaagcag ccatcattta   1020
   aaga                                                                1024
<210> 55
   <211> 1024
   <212> DNA
   <213> Kl ebsiella pneumoniae
<400> 55
   ggttaagcga ctaagcgtac acggtggatg ccctggcagt cagaggcgat gaaggacgtg      60
   ct aat ct gcg aaaagcgt cg gt aaggt gat at gaaccgt t at aaccggcg at gt ccgaat 120
   ggggaaaccc agtgcaattc gttgcactat cgttaactga atacataggt taacgaggcg     180
   aaccggggga actgaaacat ctaagtaccc cgaggaaaag aaatcaaccg agattccccc     240
   agtagcggcg agcgaacggg gagcagccca gagtctgaat cagcttgtgt gttagtggaa     300
   cggt ct ggaa agt ccgacgg t acagggt ga t agt cccgt a caccaaaat g cacaggct gt 360
   gaact cgaag agt agggcgg gacacgt ggt at cct gt ct g aat at ggggg gaccat cct c 420
   caaggctaaa tactcctgac tgaccgatag tgaaccagta ccgtgaggga aaggcgaaaa     480
   gaaccccggc gaggggagtg aaaaagaacc tgaaaccgtg tacgtacaag cagtgggagc     540
   acct t cgggt gt gact gcgt acct t t t gt a t aat gggt ca gcgact t at a t t ct gt agca 600
   aggttaaccg tataggggag ccgcagggaa accgagtctt aactgggcgt taagttgcag     660
   ggtatagacc cgaaacccgg tgatctagcc atgggcaggt tgaaggttgg gtaacactaa     720
   ct ggaggacc gaaccgact a at gt t gaaaa at t agcggat gact t gt ggc t gggggt gaa 780
   aggccaatca aaccgggaga tagctggttc tccccgaaag ctatttaggt agcgcctcgt     840
   gaact cat ct t cgggggt ag agcact gt t t cggct agggg gt cat cccga ct t accaacc 900
   cgatgcaaac tacgaatacc gaagaatgtt atcacgggag acacacggcg ggtgctaacg     960
   tccgtcgtga agagggaaac aacccagacc gccagctaag gtcccaaagt catggttaag    1020
   t ggg 1024
<210> 56
   <211> 1024
   <212> DNA
   <213> Kl ebsiella pneumoniae
<400> 56
   gct aat ct gc gaaaagcgt c ggt aaggt ga t at gaaccgt t at aaccggc gat gt ccgaa 60
   t ggggaaacc cagt gt gat t cgt cgcact a t cgt t aact g aat acat agg t t aacgaggc 120
   gaaccggggg aactgaaaca tctaagtacc ccgaggaaaa gaaatcaacc gagattcccc 180
   cagtagcggc gagcgaacgg ggagcagccc agagtctgaa tcagcttgtg tgttagtgga 240
   acggtctgga aagtccgacg gtacagggtg atagtcccgt acaccaaaat gcacaggttg 300
   t gaact cgaa gagt agggcg ggacacgt gg t at cct gt ct gaat at gggg ggaccat cct 360
   ccaaggctaa atactcctga ctgaccgata gtgaaccagt accgtgaggg aaaggcgaaa 420
   agaaccccgg cgaggggagt gaaaaagaac ctgaaaccgt gtacgtacaa gcagtgggag 480
   cacct t cggg t gt gact gcg t acct t t t gt at aat gggt c agcgact t at at t ct gt agc 540
   aaggttaacc gtatagggga gccgcaggga aaccgagtct taactgggcg ttaagttgca 600
   gggtatagac ccgaaacccg gtgatctagc catgggcagg ttgaaggttg ggtaacacta 660
   actggaggac cgaaccgact aatgttgaaa aattagcgga tgacttgtgg ctgggggtga 720
   aaggccaatc aaaccgggag atagctggtt ctccccgaaa gctatttagg tagcgcctcg 780
   t gaant cat c t t cgggggt a gagcact gt t t cggct aggg ggt cat cccg act t accaac 840
   ccgatgcaaa ctacgaatac cgaagaatgt tatcacggga gacacacggc gggtgctaac 900
   gtccgtcgtg aagagggaaa caacccagac cgccagctaa ggtcccaaag tcatggttaa 960
   gtgggaaacg atgtgggaag gcacagacag ccaggatgtt ggcttagaag cagccatcat 1020
   t t aa 1024
<210> 57
   <211> 1024
   <212> DNA
   <213> Kl ebsiella pneumoniae
<400> 57
   t ct gcgaaag cgt cggt aag gt gat at gaa ccgt t anagc cggcgat gt c cgaat gggga 60
   aacccagtgt gattcgtcac actatcgtta actgaataca taggttaacg aggcgaaccg 120
   ggggaactga aacatctaag taccccgagg aaaagaaatc aaccgagatt cccccagtag 180
   cggcgagcga acggggagca gcccagagtc tgaatcagct tgtgtgttag tggaacggtc 240
   tggaaagtcc gacggtacag ggtgatagtc ccgtacacca aaatgcacag gctgtgaact 300
   cgaagagtag ggcgggacac gtggtatcct gtctgaatat ggggggacca tcctccaagg 360
   ctaaatactc ctgactgacc gatagtgaac cagtaccgtg agggaaaggc gaaaagaacc 420
   ccggcgaggg gagtgaaaaa gaacctgaaa ccgtgtacgt acaagcagtg ggncacttcg 480
   tggtgtgact gcgntacctt ttgtataatg ggtcagcgac ttatattctg tagcaaggtt 540
   aaccgtatag gggagccgaa gggaaaccga gtcttaactg ggcgttaagt tgcagggtat 600
   agacccgaaa cccggtgatc tagccatggg caggttgaag gttgggtaac actaactgga 660
   ggaccgaacc gactaatgtt gaaaaattag cggatgactt gtggctgggg gtgaaaggcc 720
   aatcaaaccg ggagatagct ggttctcccc gaaagctatt taggtagcgc ctcgtgaact 780
   catcttcggg ggtagagcac tgtttcggct agggggccat cccggcttac caacccgatg 840
   caaactacga ataccgaaga atgttatcac gggagacaca cggcgggtgc taacgtccgt 900
   cgtgaagagg gaaacaaccc agaccgccag ctaaggtccc aaagtcatgg ttaagtggga 960
   aacgatgtgg gaaggcacag acagccagga tgttggctta gaagcagcca tcatttaaag 1020
   aaag 1024
<210> 58
   <211> 1024
   <212> DNA
   <213> Klebsiella pneumoniae
<400> 58
   ggttaagcga ctaagcgtac acggtggatg ccctggcagt cagaggcgat gaaggacgtg      60
   ct aat ct gcg aaaagcgt cg gt aaggt gat at gaaccgt t at aaccggcg at gt ccgaat 120
   ggggaaaccc agtgcaattc gttgcactat cgttaactga atacataggt taacgaggcg     180
   aaccggggga actgaaacat ctaagtaccc cgaggaaaag aaatcaaccg agattccccc     240
   agtagcggcg agcgaacggg gagcagccca gagtctgaat cagcttgtgt gttagtggaa     300
   cggt ct ggaa agt ccgacgg t acagggt ga t agt cccgt a caccaaaat g cacaggct gt 360
   gaact cgaag agt agggcgg gacacgt ggt at cct gt ct g aat at ggggg gaccat cct c 420
   caaggctaaa tactcctgac tgaccgatag tgaaccagta ccgtgaggga aaggcgaaaa     480
   gaaccccggc gaggggagtg aaaaagaacc tgaaaccgtg tacgtacaag cagtgggagc     540
   acct t cgggt gt gact gcgt acct t t t gt a t aat gggt ca gcgact t at a t t ct gt agca 600
   aggttaaccg tataggggag ccgcagggaa accgagtctt aactgggcgt taagttgcag     660
   ggtatagacc cgaaacccgg tgatctagcc atgggcaggt tgaaggttgg gtaacactaa     720
   ctggaggacc gaaccgacta atgttgaaaa attagcggat gacttgtggc tgggggtgaa     780
   aggccaatca aaccgggaga tagctggttc tccccgaaag ctatttaggt agcgcctcgt     840
   gaact cat ct t cgggggt ag agcact gt t t cggct agggg gt cat cccga ct t accaacc 900
   cgatgcaaac tacgaatacc gaagaatgtt atcacgggag acacacggcg ggtgctaacg     960
   tccgtcgtga agagggaaac aacccagacc gccagctaag gtcccaaagt catggttaag    1020
   t ggg 1024
<210> 59
   <211> 1024
   <212> DNA
   <213> Kl ebsiella pneumoniae
<400> 59
   gctaatctgc gaaaagcgcc ggcgaggtga tatgaacctt tgacccggcg atgtccgaat      60
   ggggaaaccc agtgtgattc gtcacactat cgttaactga atacataggt taacgaggcg     120
   aaccggggga actgaaacat ctaagtaccc cgaggaaaag aaatcaaccg agattccccc     180
   agtagcggcg agcgaacggg gagcagccca gagtctgaat cagcttgtgt gttagtggaa     240
   gcgtctggaa agtcgcacgg tacagggtga cagtcccgta cacgaaanng cacaggttgt     300
   gaact cgaag agt agggcgg gacacgt ggt at cct gt ct g aat at ggggg gaccat cct c 360
   caaggctaaa tactcctgac tgaccgatag tgaaccagta ccgtgaggga aaggcgaaaa     420
   gaaccccggc gaggggagtg aaanagaacc tgaaaccgtg tacgtacaag cagtgggagc     480
   acct t cgt gg t gt gact gcg t acct t t t gt at aat gggt c agcgact t at at t ct gt agc 540
   aaggttaacc gtatagggga gccgaaggga aaccgagtct taactgggcg ttaagttgca     600
   gggtatagac ccgaaacccg gtgatctagc catgggcagg ttgaaggttg ggtaacacta     660
   actggaggac cgaaccgact aatgttgaaa aattagcgga tgacttgtgg ctgggggtga     720
   aaggccaatc aaaccgggag atagctggtt ctccccgaaa gctatttagg tagcgcctcg     780
   t gaact cat c t t cgggggt a gagcact gt t t cggct aggg ggccat cccg gct t accaac 840
   ccgatgcaaa ctacgaatac cgaanaatgt tatcacggga gacacacggc gggtgctaac     900
   gtccgtcgtg aagagggaaa caacccagac cgccagctaa ggtcccaaag tcatggttaa     960
   gtgggaaacg atgtgggaag gcacagacag ccaggatgtt ggcttagaag cagccatcat    1020
   t t aa 1024
<210> 60
   <211> 1024
   <212> DNA
   <213> Kl ebsiella pneumoniae
<400> 60
   ctaatctgcg aaaagcgtcg gtaaggtgat atgaaccgtt anagccggcg atgtccgaat      60
   ggggaaaccc agtgtgattc gtcncactat cgttaactga atacataggt taacgaggcg     120
   aaccggggga actgaaacat ctaagtaccc cgaggaaaag aaatcaaccg agattccccc     180
   agtagcggcg agcgaacggg gagcagccca gagtctgaat cagcttgtgt gttagtggaa     240
   cggt ct ggaa agt ccgacgg t acagggt ga t agt cccgt a caccaaaat g cacaggct gt 300
   gaact cgaag agt agggcgg gacacgt ggt at cct gt ct g aat at ggggg gaccat cct c 360
   caaggctaaa tactcctgac tgaccgatag tgaaccagta ccgtgaggga aaggcgaaaa 420
   gaaccccggc gaggggagtg aaaaagaacc tgaaaccgtg tacgtacaag cagtgggagc 480
   acct t cgggt gt gact gcgt acct t t t gt a t aat gggt ca gcgact t at a t t ct gt agca 540
   aggttaaccg tataggggag ccgcagggaa accgagtctt aactgggcgt taagttgcag 600
   ggtatagacc cgaaacccgg tgatctagcc atgggcaggt tgaaggttgg gtaacactaa 660
   ct ggaggacc gaaccgact a at gt t gaaaa at t agcggat gact t gt ggc t gggggt gaa 720
   aggccaatca aaccgggaga tagctggttc tccccgaaag ctatttaggt agcgcctcgt 780
   gaat t cat ct t cgggggt ag agcact gt t t cggct agggg gt cat cccga ct t accaacc 840
   cgatgcaaac tacgaatacc gaagaatgtt atcacgggag acacacggcg ggtgctaacg 900
   tccgtcgtga agagggaaac aacccagacc gccagctaag gtcccaaagt catggttaag 960
   tgggaaacga tgtgggaagg cacagacagc caggatgttg gcttagaagc agccatcatt 1020
   t aaa 1024
<210> 61
   <211> 1024
   <212> DNA
   <213> Klebsiella pneumoniae
<400> 61
   ct aat ct gcg aaaagcgt cg gt aaggt gat at gaaccgt t at aaccggcg at gt ccgaat 60
   ggggaaaccc agtgcaattc gttgcactat cgttaactga atacataggt taacgaggcg 120
   aaccggggga actgaaacat ctaagtaccc cgaggaaaag aaatcaaccg agattccccc 180
   agtagcggcg agcgaacggg gagcagccca gagtctgaat cagcttgtgt gttagtggaa 240
   cggt ct ggaa agt ccgacgg t acagggt ga t agt cccgt a caccaaaat g cacaggnt gt 300
   gaact cgaag agt agggcgg gacacgt ggt at cct gt ct g aat at ggggg gaccat cct c 360
   caaggctaaa tactcctgac tgaccgatag tgaaccagta ccgtgaggga aaggcgaaaa 420
   gaaccccggc gaggggagtg aaaaagaacc tgaaaccgtg tacgtacaag cagtgggagc 480
   acct t cgggt gt gact gcgt acct t t t gt a t aat gggt ca gcgact t at a t t ct gt agca 540
   aggttaaccg tataggggag ccgcagggaa accgagtctt aactgggcgt taagttgcag 600
   ggtatagacc cgaaacccgg tgatctagcc atgggcaggt tgaaggttgg gtaacactaa 660
   ct ggaggacc gaaccgact a at gt t gaaaa at t agcggat gact t gt ggc t gggggt gaa 720
   aggccaatca aaccgggaga tagctggttc tccccgaaag ctatttaggt agcgcctcgt 780
   gaact cat ct t cgggggt ag agcact gt t t cggct agggg gt cat cccga ct t accaacc 840
   cgatgcaaac tacgaatacc gaagaatgtt atcacgggag acacacggcg ggtgctaacg 900
   tccgtcgtga agagggaaac aacccagacc gccagctaag gtcccaaagt catggttaag 960
   tgggaaacga tgtgggaagg cacagacagc caggatgttg gcttanaanc agccatcatt 1020
   t aaa 1024
<210> 62
   <211> 1024
   <212> DNA
   <213> Legi onel l a pneumophila
<400> 62
   ggaacgagcg ttgagccata gatgtccgaa tgggggaacc cggctgcagc gatgcggtca      60
   t t t gcat t t g aat acat agg at gcaaaggc gaact cgggg aact gaaaca t ct aagt acc 120
   cgaaggaaaa gaaatcaaga gagattctcc aagtagcggc gagcgaacgg ggaggagcct     180
   ggcgt gat t t at t at t gaac t gagt agaac aat t t gggaa agt t ggccgt agagggt gaa 240
   agccccgtat acgaaggttt gatgaggaac taggcacgcg aacaagtagg ccgggacacg     300
   t gaaat cct g gt t gaagat g ggt ggaccat cat ccaaggc t aaat act ac t t act gaccg 360
   atagtgaacc agtaccgtga gggaaaggtg aaaagaaccc cggagagggg agtgaaatag     420
   aat ct gaaac cgt t t gcgt a caagcagt gg gagcat agt t t aggct gt gt gact gcgt ac 480
   ct t t t gt at a at gggt cagc gagt t act t t cagt ggcgag gt t aact gaa t aagggagcc 540
   gtagagaaat cgagtctgaa tagggcgata gtcgctggga gtagacccga aaccgggcga     600
   t ct agccat g t gcaggat ga aggt t gggt a acaccaact g gaggt ccgaa ccgggt aat g 660
   t t gaaaaat t at cggat gac gt gt ggct ag gagt gaaagg ct aat caagc ccggagat ag 720
   ct ggt t ct cc ccgaaagct a t t t aggt agc gcct cgt gaa t gat t act gg gggt agagca 780
   ctgtttcggc tagggggctg tcatggctta ccaaaccgat gcaaactccg aataccggct     840
   aattgaatca cgggagacac acggcgggtg ctaacgtccg tcgtggagag ggaaacaacc     900
   cagaccgcca gctaaggtcc ccaagtacta gttaagtggg aaacgatgtg ggaaggcata     960
   gacagccagg aggttggctt agaagcagcc accctttaaa gaaagcgtaa tagctcactg    1020
   gt cg 1024
<210> 63
   <211> 1024
   <212> DNA
   <213> Legi onel l a pneumophila
<400> 63
   ggaacgagcg ttgagccata gatgtccgaa tgggggaacc cggctgcagc gatgcggtca 60
   tttgcatttg aatacatagg atgcaaaggc gaactcgggg aactgaaaca tctaagtacc 120
   cgaaggaaaa gaaatcaaga gagattctcc aagtagcggc gagcgaacgg ggaggagcct 180
   ggcgtgattt attattgaac tgagtagaac aatttgggaa agttggccgt agagggtgaa 240
   agccccgtat acgaaggttt gatgaggaac taggcacgcg aacaagtagg ccgggacacg 300
   tgaaatcctg gttgaagatg ggtggaccat catccaaggc taaatactac ttactgaccg 360
   atagtgaacc agtaccgtga gggaaaggtg aaaagaaccc cggagagggg agtgaaatag 420
   aatctgaaac cgtttgcgta caagcagtgg gagcatagtt taggctgtgt gactgcgtac 480
   cttttgtata atgggtcagc gagttacttt cagtggcgag gttaactgaa taagggagcc 540
   gtagagaaat cgagtctgaa tagggcgata gtcgctggga gtagacccga aaccgggcga 600
   tctagccatg tgcaggatga aggttgggta acaccaactg gaggtccgaa ccgggtaatg 660
   ttgaaaaatt atcggatgac gtgtggctag gagtgaaagg ctaatcaagc ccggagatag 720
   ctggttctcc ccgaaagcta tttaggtagc gcctcgtgaa tgattactgg gggtagagca 780
   ctgtttcggc tagggggctg tcatggctta ccaaaccgat gcaaactccg aataccggct 840
   aattgaatca cgggagacac acggcgggtg ctaacgtccg tcgtggagag ggaaacaacc 900
   cagaccgcca gctaaggtcc ccaagtacta gttaagtggg aaacgatgtg ggaaggcata 960
   gacagccagg aggttggctt agaagcagcc accctttaaa gaaagcgtaa tagctcactg 1020
   gtcg 1024
<210> 64
   <211> 1024
   <212> DNA
   <213> Legionella pneumophila
<400> 64
   ggaacgagcg ttgagccata gatgtccgaa tgggggaacc cggctgcagc gatgcggtca 60
   tttgcatttg aatacatagg atgcaaaggc gaactcgggg aactgaaaca tctaagtacc 120
   cgaaggaaaa gaaatcaaga gagattctcc aagtagcggc gagcgaacgg ggaggagcct 180
   ggcgtgattt attattgaac tgagtagaac aatttgggaa agttggccgt agagggtgaa 240
   agccccgtat acgaaggttt gatgaggaac taggcacgcg aacaagtagg ccgggacacg 300
   tgaaatcctg gttgaagatg ggtggaccat catccaaggc taaatactac ttactgaccg 360
   atagtgaacc agtaccgtga gggaaaggtg aaaagaaccc cggagagggg agtgaaatag 420
   aatctgaaac cgtttgcgta caagcagtgg gagcatagtt taggctgtgt gactgcgtac 480
   cttttgtata atgggtcagc gagttacttt cagtggcgag gttaactgaa taagggagcc 540
   gtagagaaat cgagtctgaa tagggcgata gtcgctggga gtagacccga aaccgggcga 600
   tctagccatg tgcaggatga aggttgggta acaccaactg gaggtccgaa ccgggtaatg 660
   ttgaaaaatt atcggatgac gtgtggctag gagtgaaagg ctaatcaagc ccggagatag 720
   ctggttctcc ccgaaagcta tttaggtagc gcctcgtgaa tgattactgg gggtagagca 780
   ctgtttcggc tagggggctg tcatggctta ccaaaccgat gcaaactccg aataccggct 840
   aattgaatca cgggagacac acggcgggtg ctaacgtccg tcgtggagag ggaaacaacc 900
   cagaccgcca gctaaggtcc ccaagtacta gttaagtggg aaacgatgtg ggaaggcata 960
   gacagccagg aggttggctt agaagcagcc accctttaaa gaaagcgtaa tagctcactg 1020
   gtcg 1024
<210> 65
   <211> 1024
   <212> DNA
   <213> Mor axel l a cat ar r hal i s
<400> 65
   gcgatagcgt cggtgaggtg gcaatatcct gtgacccggc gatttctgaa tggggaaacc 60
   caaccaacat aagttggtta ttacacagtt tactgtgtaa ggcaaaccgg gagaagtgaa     120
   acatctcagt accccgagga aaagacatca aatgagattc cgtaagtagc ggcgagcgaa     180
   cacggaggag ccgatcaatt ttacagtagc aaaatggcgt gggaaagcca accatagtag     240
   gtgatagtcc tgtatgcgaa actgtttaag cgacatatta agtagggcgg aacacgagaa     300
   at t ct gt ct g aagat ggggg gaccat cct c caaggct aaa t act cct gac t gaccgat ag 360
   tgaaccagta ccgtgaggga aaggcgaaaa gaacccctgt taggggagtg aaatagaacc     420
   tgaaaccgtg tgcatacaag cagtcggagc ccgaccactt aatcgttttg aggataacaa     480
   tgaattttta ctacgtctat gtcatacaaa acgtggataa tcatgatgag ttttatgttg     540
   gt t t t accac caat t t aaaa caaagaat t g at gaccat aa t aat ggcaca act t act caa 600
   ccagagcaag aacatggcgt attgtttatt gtgaagccta cattaatgaa caagtggcaa     660
   gaaaacgaga gcgaaccata aaaaataatg gtcgtatgag aactttcttg atgaatcgtg     720
   t caaat caca at t t gacaat cagaacgat t aagt ggt cgg gt gacggcgt acct t t t gt a 780
   t aat gggt ca gcgact t at a t t ct gt agca aggt t aaccg aat aggggag ccgt agggaa 840
   accgagtctt aatagggcga ttaagttgca gggtatagac ccgaaaccga gtgatctatc     900
   catgagcagg ttgaaagtgc cgtaacaggc accggaggac cgaacccact gtcgttgaaa     960
   agccagggga tgacttgtgg ataggggtga aaggctaatc aaactcggtg atagctggtt    1020
   ct cc 1024
<210> 66
   <211> 1024
   <212> DNA
   <213> Mor axel l a cat ar r hal i s
<400> 66
   gcgat agcgt  cggt gaggt g  gcaat at cct  gt gacccggc  gat t t ct gaa  t ggggaaacc      60
   caaccaacat aagttggtta ttacacagtt tactgtgtaa ggcaaaccgg gagaagtgaa     120
   acatctcagt accccgagga aaagacatca aatgagattc cgtaagtagc ggcgagcgaa     180
   cacggaggag ccgatcaatt ttacagtagc aaaatggcgt gggaaagcca accatagtag     240
   gtgatagtcc tgtatgcgaa actgtttaag cgacatatta agtagggcgg aacacgagaa     300
   at t ct gt ct g  aagat ggggg  gaccat cct c  caaggct aaa  t act cct gac  t gaccgat ag     360
   tgaaccagta ccgtgaggga aaggcgaaaa gaacccctgt taggggagtg aaatagaacc     420
   t gaaaccgt g  t gcat acaag  cagt cggagc  ccgaccact t  aat cgt t t t g  aggat aacaa     480
   t gaat t t t t a  ct acgt ct at  gt cat acaaa  acgt ggat aa  t cat gat gag  t t t t at gt t g     540
   gct t t accac  caat t t aaaa  caaagaat t g  at gaccat aa  t aat ggcaca  act t act caa     600
   ccagagcaag aacatggcgt attgtttatt gtgaagccta cattaatgaa caagtggcaa     660
   gaaaacgaga gcgaaccata aaaaataatg gtcgtatgag aactttcttg atgaatcgtg     720
   t caaat caca  at t t gacaat  cagaacgat t  aagt ggt cgg  gt gacggcgt  acct t t t gt a     780
   t aat gggt ca  gcgact t at a  t t ct gt agca  aggt t aaccg  aat aggggag  ccgt agggaa     840
   accgagtctt aatagggcga ttaagttgca gggtatagac ccgaaaccga gtgatctatc     900
   catgagcagg ttgaaagtgc cgtaacaggc accggaggac cgaacccact gtcgttgaaa     960
   agccagggga tgacttgtgg ataggggtga aaggctaatc aaactcggtg atagctggtt   1020
   ct cc                                                                       1024
<210> 67
   <211> 1024
   <212> DNA
   <213> Mor axel l a cat ar r hal i s
<400> 67
   gcgat agcgt cggt gaggt g gcaat at cct gt gacccggc gat t t ct gaa t ggggaaacc 60
   caaccaacat aagttggtta ttacacagtt tactgtgtaa ggcaaaccgg gagaagtgaa     120
   acatctcagt accccgagga aaagacatca aatgagattc cgtaagtagc ggcgagcgaa     180
   cacggaggag ccgatcaatt ttacagtagc aaaatggcgt gggaaagcca accatagtag     240
   gtgatagtcc tgtatgcgaa actgtttaag cgacatatta agtagggcgg aacacgagaa     300
   at t ct gt ct g aagat ggggg gaccat cct c caaggct aaa t act cct gac t gaccgat ag 360
   tgaaccagta ccgtgaggga aaggcgaaaa gaacccctgt taggggagtg aaatagaacc     420
   tgaaccgtgg tgcatacaag cagtcggagc ccgaccactt aatcgttttg aggataacaa     480
   t gaat t t t t a ct acgt ct at gt cat acaaa acgt ggat aa t cat gat gag t t t t at gt t g 540
   gt t t t accac caat t t aaaa caaagaat t g at gaccat aa t aat ggcaca act t act caa 600
   ccagagcaag aacatggcgt attgtttatt gtgaagccta cattaatgaa caagtggcaa     660
   gaaaacgaga gcgaaccata aaaaataatg gtcgtatgag aactttcttg atgaatcgtg     720
   t caaat caca at t t gacaat cagaacgat t aagt ggt cgg gt gacggcgt acct t t t gt a 780
   t aat gggt ca gcgact t at a t t ct gt agca aggt t aaccg aat aggggag ccgt agggaa 840
   accgagtctt aatagggcga atgagttgca gggtatagac ccgaaaccga gtgatctatc     900
   catgagcagg ttgaaagtgc cgtaacaggc accggaggac cgaacccact gtcgttgaaa     960
   agccagggga tgacttgtgg ataggggtga aaggctaatc aaactcggtg atagctggtt    1020
   ct cc 1024
<210> 68
   <211> 1024
   <212> DNA
   <213> Pseudorronas aer ugi nosa
<400> 68
   cct gcgaaag ct t cggggag t cggcaaaca gact t t gat c cggagat ct c t gaat ggggg 60
   aacccaccta ggataaccta ggtatcttgt actgaatcca taggtgcaag aggcgaacca     120
   ggggaact ga aacat ct aag t accct gagg aaaagaaat c aaccgagat t ccct t agt ag 180
   t ggcgagcga acggggat t a gccct t aagc t t cat t gat t t t agcggaac gct ct ggaaa 240
   gtgcggccat agtgggtgat agccccgtac gcgaaaggat ctttgaagtg aaatcgagta     300
   ggacggagca cgagaaactt tgtctgaaca tggggggacc atcctccaag gctaaatact     360
   actgactgac cgatagtgaa ccagtaccgt gagggaaagg cgaaaagaac cccggagagg    420
   ggagt gaaat agaacct gaa accgt at gcg t acaagcagt gggagcct ac t t gt t aggt g 480
   act gcgt acc t t t t gt at aa t gggt cagcg act t at at t c agt ggcaagc t t aaccgt at 540
   agggt aggcg t agcgaaagc gagt ct t aat agggcgt t t a gt cgct gggt at agacccga 600
   aaccgggcga tctatccatg agcaggttga aggttaggta acactgactg gaggaccgaa    660
   cccact cccg t t gaaaaggt aggggat gac t t gt ggat cg gagt gaaagg ct aat caagc 720
   t cggagat ag ct ggt t ct cc t cgaaagct a t t t aggt agc gcct cat gt a t cact ct ggg 780
   gggtagagca ctgtttcggc tagggggtca tcccgactta ccaaaccgat gcaaactccg    840
   aatacccaga agtgccgagc atgggagaca cacggcgggt gctaacgtcc gtcgtgaaaa    900
   gggaaacaac ccagaccgcc agctaaggtc ccaaagttgt ggttaagtgg taaacgatgt    960
   gggaaggct t agacagct ag gaggt t ggct t agaagcagc cat cct t t aa agaaagcgt a 1020
   at ag 1024
<210> 69
   <211> 1024
   <212> DNA
   <213> Pseudomonas aer ugi nosa
<400> 69
   cct gcgaaag ct t cggggag t cggcaaaca gact t t gat c cggagat ct c t gaat ggggg 60
   aacccaccta ggataaccta ggtatcttgt actgaatcca taggtgcaag aggcgaacca    120
   ggggaact ga aacat ct aag t accct gagg aaaagaaat c aaccgagat t ccct t agt ag 180
   t ggcgagcga acggggat t a gccct t aagc t t cat t gat t t t agcggaac gct ct ggaaa 240
   gtgcggccat agtgggtgat agccccgtac gcgaaaggat ctttgaagtg aaatcgagta    300
   ggacggagca cgagaaactt tgtctgaaca tggggggacc atcctccaag gctaaatact    360
   actgactgac cgatagtgaa ccagtaccgt gagggaaagg cgaaaagaac cccggagagg    420
   ggagt gaaat agaacct gaa accgt at gcg t acaagcagt gggagcct ac t t gt t aggt g 480
   act gcgt acc t t t t gt at aa t gggt cagcg act t at at t c agt ggcaagc t t aaccgt at 540
   agggt aggcg t agcgaaagc gagt ct t aat agggcgt t t a gt cgct gggt at agacccga 600
   aaccgggcga tctatccatg agcaggttga aggttaggta acactgactg gaggaccgaa    660
   cccact cccg t t gaaaaggt aggggat gac t t gt ggat cg gagt gaaagg ct aat caagc 720
   t cggagat ag ct ggt t ct cc t cgaaagct a t t t aggt agc gcct cat gt a t cact ct ggg 780
   gggtagagca ctgtttcggc tagggggtca tcccgactta ccaaaccgat gcaaactccg    840
   aatacccaga agtgccgagc atgggagaca cacggcgggt gctaacgtcc gtcgtgaaaa    900
   gggaaacaac ccagaccgcc agctaaggtc ccaaagttgt ggttaagtgg taaacgatgt    960
   gggaaggct t agacagct ag gaggt t ggct t agaagcagc caccct t t aa agaaagcgt a 1020
   at ag 1024
<210> 70
   <211> 1024
   <212> DNA
   <213> Pseudomonas aer ugi nosa
<400> 70
   cct gcgaaag ct t cggggag t cggcaaaca gact t t gat c cggagat ct c t gaat ggggg 60
   aacccaccta ggataaccta ggtatcttgt actgaatcca taggtgcaag aggcgaacca    120
   ggggaact ga aacat ct aag t accct gagg aaaagaaat c aaccgagat t ccct t agt ag 180
   t ggcgagcga acggggat t a gccct t aagc t t cat t gat t t t agcggaac gct ct ggaaa 240
   gtgcggccat agtgggtgat agccccgtac gcgaaaggat ctttgaagtg aaatcgagta    300
   ggacggagca cgagaaactt tgtctgaaca tggggggacc atcctccaag gctaaatact    360
   actgactgac cgatagtgaa ccagtaccgt gagggaaagg cgaaaagaac cccggagagg    420
   ggagt gaaat agaacct gaa accgt at gcg t acaagcagt gggagcct ac t t gt t aggt g 480
   act gcgt acc t t t t gt at aa t gggt cagcg act t at at t c agt ggcaagc t t aaccgt at 540
   agggt aggcg t agcgaaagc gagt ct t aat agggcgt t t a gt cgct gggt at agacccga 600
   aaccgggcga tctatccatg agcaggttga aggttaggta acactgactg gaggaccgaa    660
   cccact cccg t t gaaaaggt aggggat gac t t gt ggat cg gagt gaaagg ct aat caagc 720
   t cggagat ag ct ggt t ct cc t cgaaagct a t t t aggt agc gcct cat gt a t cact ct ggg 780
   gggtagagca ctgtttcggc tagggggtca tcccgactta ccaaaccgat gcaaactccg    840
   aatacccaga agtgccgagc atgggagaca cacggcgggt gctaacgtcc gtcgtgaaaa    900
   gggaaacaac ccagaccgcc agctaaggtc ccaaagttgt ggttaagtgg taaacgatgt    960
   gggaaggct t agacagct ag gaggt t ggct t agaagcagc caccct t t aa agaaagcgt a 1020
   at ag 1024
<210> 71
   <211> 1024
   <212> DNA
   <213> Pseudorronas aer ugi nosa
<400> 71
   cctgcgaaag cttcggggag tcggcaaaca gactttgatc cggagatctc tgaatggggg 60
   aacccaccta ggataaccta ggtatcttgt actgaatcca taggtgcaag aggcgaacca 120
   ggggaactga aacatctaag taccctgagg aaaagaaatc aaccgagatt cccttagtag 180
   tggcgagcga acggggatta gcccttaagc ttcattgatt ttagcggaac gctctggaaa 240
   gtgcggccat agtgggtgat agccccgtac gcgaaaggat ctttgaagtg aaatcgagta 300
   ggacggagca cgagaaactt tgtctgaaca tggggggacc atcctccaag gctaaatact 360
   actgactgac cgatagtgaa ccagtaccgt gagggaaagg cgaaaagaac cccggagagg 420
   ggagtgaaat agaacctgaa accgtatgcg tacaagcagt gggagcctac ttgttaggtg 480
   actgcgtacc ttttgtataa tgggtcagcg acttatattc agtggcaagc ttaaccgtat 540
   agggtaggcg tagcgaaagc gagtcttaat agggcgttta gtcgctgggt atagacccga 600
   aaccgggcga tctatccatg agcaggttga aggttaggta acactgactg gaggaccgaa 660
   cccactcccg ttgaaaaggt aggggatgac ttgtggatcg gagtgaaagg ctaatcaagc 720
   tcggagatag ctggttctcc tcgaaagcta tttaggtagc gcctcatgta tcactctggg 780
   gggtagagca ctgtttcggc tagggggtca tcccgactta ccaaaccgat gcaaactccg 840
   aatacccaga agtgccgagc atgggagaca cacggcgggt gctaacgtcc gtcgtgaaaa 900
   gggaaacaac ccagaccgcc agctaaggtc ccaaagttgt ggttaagtgg taaacgatgt 960
   gggaaggctt agacagctag gaggttggct tagaagcagc catcctttaa agaaagcgta 1020
   atag 1024
<210> 72
   <211> 1024
   <212> DNA
   <213> Pseudomonas aeruginosa
<400> 72
   cct gcgaaag ct t cggggag t cggcaaaca gact t t gat c cggagat ct c t gaat ggggg 60
   aacccaccta ggataaccta ggtatcttgt actgaatcca taggtgcaag aggcgaacca     120
   ggggaact ga aacat ct aag t accct gagg aaaagaaat c aaccgagat t ccct t agt ag 180
   t ggcgagcga acggggat t a gccct t aagc t t cat t gat t t t agcggaac gct ct ggaaa 240
   gtgcggccat agtgggtgat agccccgtac gcgaaaggat ctttgaagtg aaatcgagta     300
   ggacggagca cgagaaactt tgtctgaaca tggggggacc atcctccaag gctaaatact     360
   actgactgac cgatagtgaa ccagtaccgt gagggaaagg cgaaaagaac cccggagagg     420
   ggagt gaaat agaacct gaa accgt at gcg t acaagcagt gggagcct ac t t gt t aggt g 480
   act gcgt acc t t t t gt at aa t gggt cagcg act t at at t c agt ggcaagc t t aaccgt at 540
   agggt aggcg t agcgaaagc gagt ct t aat agggcgt t t a gt cgct gggt at agacccga 600
   aaccgggcga tctatccatg agcaggttga aggttaggta acactgactg gaggaccgaa     660
   cccact cccg t t gaaaaggt aggggat gac t t gt ggat cg gagt gaaagg ct aat caagc 720
   t cggagat ag ct ggt t ct cc t cgaaagct a t t t aggt agc gcct cat gt a t cact ct ggg 780
   gggtagagca ctgtttcggc tagggggtca tcccgactta ccaaaccgat gcaaactccg     840
   aatacccaga agtgccgagc atgggagaca cacggcgggt gctaacgtcc gtcgtgaaaa     900
   gggaaacaac ccagaccgcc agctaaggtc ccaaagttgt ggttaagtgg taaacgatgt     960
   gggaaggct t agacagct ag gaggt t ggct t agaagcagc cat cct t t aa agaaagcgt a 1020
   at ag 1024
<210> 73
   <211> 1024
   <212> DNA
   <213> Pseudorronas aer ugi nosa
<400> 73
   cct gcgaaag ct t cggggag t cggcaaaca gact t t gat c cggagat ct c t gaat ggggg 60
   aacccaccta ggataaccta ggtatcttgt actgaatcca taggtgcaag aggcgaacca     120
   ggggaact ga aacat ct aag t accct gagg aaaagaaat c aaccgagat t ccct t agt ag 180
   t ggcgagcga acggggat t a gccct t aagc t t cat t gat t t t agcggaac gct ct ggaaa 240
   gtgcggccat agtgggtgat agccccgtac gcgaaaggat ctttgaagtg aaatcgagta    300
   ggacggagca cgagaaactt tgtctgaaca tggggggacc atcctccaag gctaaatact    360
   actgactgac cgatagtgaa ccagtaccgt gagggaaagg cgaaaagaac cccggagagg    420
   ggagt gaaat agaacct gaa accgt at gcg t acaagcagt gggagcct ac t t gt t aggt g 480
   act gcgt acc t t t t gt at aa t gggt cagcg act t at at t c agt ggcaagc t t aaccgt at 540
   agggtaggcg tagcgaaagc gagtcttaat agggcgttta gtcgctgggt atagacccga    600
   aaccgggcga tctatccatg agcaggttga aggttaggta acactgactg gaggaccgaa    660
   cccact cccg t t gaaaaggt aggggat gac t t gt ggat cg gagt gaaagg ct aat caagc 720
   t cggagat ag ct ggt t ct cc t cgaaagct a t t t aggt agc gcct cat gt a t cact ct ggg 780
   gggtagagca ctgtttcggc tagggggtca tcccgactta ccaaaccgat gcaaactccg    840
   aatacccaga agtgccgagc atgggagaca cacggcgggt gctaacgtcc gtcgtgaaaa    900
   gggaaacaac ccagaccgcc agctaaggtc ccaaagttgt ggttaagtgg taaacgatgt    960
   gggaaggct t agacagct ag gaggt t ggct t agaagcagc cat cct t t aa agaaagcgt a 1020
   at ag 1024
<210> 74
   <211> 1024
   <212> DNA
   <213> Pseudomonas aer ugi nosa
<400> 74
   cctgcgaaag cttcggggag tcggcaaaca gactttgatc cggagatctc tgaatggggg 60
   aacccaccta ggataaccta ggtatcttgt actgaatcca taggtgcaag aggcgaacca 120
   ggggaactga aacatctaag taccctgagg aaaagaaatc aaccgagatt cccttagtag 180
   tggcgagcga acggggatta gcccttaagc ttcattgatt ttagcggaac gctctggaaa 240
   gtgcggccat agtgggtgat agccccgtac gcgaaaggat ctttgaagtg aaatcgagta 300
   ggacggagca cgagaaactt tgtctgaaca tggggggacc atcctccaag gctaaatact 360
   actgactgac cgatagtgaa ccagtaccgt gagggaaagg cgaaaagaac cccggagagg 420
   ggagtgaaat agaacctgaa accgtatgcg tacaagcagt gggagcctac ttgttaggtg 480
   actgcgtacc ttttgtataa tgggtcagcg acttatattc agtggcaagc ttaaccgtat 540
   agggtaggcg tagcgaaagc gagtcttaat agggcgttta gtcgctgggt atagacccga 600
   aaccgggcga tctatccatg agcaggttga aggttaggta acactgactg gaggaccgaa 660
   cccactcccg ttgaaaaggt aggggatgac ttgtggatcg gagtgaaagg ctaatcaagc 720
   tcggagatag ctggttctcc tcgaaagcta tttaggtagc gcctcatgta tcactctggg 780
   gggtagagca ctgtttcggc tagggggtca tcccgactta ccaaaccgat gcaaactccg 840
   aatacccaga agtgccgagc atgggagaca cacggcgggt gctaacgtcc gtcgtgaaaa 900
   gggaaacaac ccagaccgcc agctaaggtc ccaaagttgt ggttaagtgg taaacgatgt 960
   gggaaggctt agacagctag gaggttggct tagaagcagc catcctttaa agaaagcgta 1020
   atag 1024
<210> 75
   <211> 1024
   <212> DNA
   <213> Pseudomonas aeruginosa
<400> 75
   cct gcgaaag ct t cggggag t cggcaaaca gact t t gat c cggagat ct c t gaat ggggg 60
   aacccaccta ggataaccta ggtatcttgt actgaatcca taggtgcaag aggcgaacca     120
   ggggaact ga aacat ct aag t accct gagg aaaagaaat c aaccgagat t ccct t agt ag 180
   t ggcgagcga acggggat t a gccct t aagc t t cat t gat t t t agcggaac gct ct ggaaa 240
   gtgcggccat agtgggtgat agccccgtac gcgaaaggat ctttgaagtg aaatcgagta     300
   ggacggagca cgagaaactt tgtctgaaca tggggggacc atcctccaag gctaaatact     360
   actgactgac cgatagtgaa ccagtaccgt gagggaaagg cgaaaagaac cccggagagg     420
   ggagt gaaat agaacct gaa accgt at gcg t acaagcagt gggagcct ac t t gt t aggt g 480
   act gcgt acc t t t t gt at aa t gggt cagcg act t at at t c agt ggcaagc t t aaccgt at 540
   agggt aggcg t agcgaaagc gagt ct t aat agggcgt t t a gt cgct gggt at agacccga 600
   aaccgggcga tctatccatg agcaggttga aggttaggta acactgactg gaggaccgaa     660
   cccact cccg t t gaaaaggt aggggat gac t t gt ggat cg gagt gaaagg ct aat caagc 720
   t cggagat ag ct ggt t ct cc t cgaaagct a t t t aggt agc gcct cat gt a t cact ct ggg 780
   gggtagagca ctgtttcggc tagggggtca tcccgactta ccaaaccgat gcaaactccg     840
   aatacccaga agtgccgagc atgggagaca cacggcgggt gctaacgtcc gtcgtgaaaa     900
   gggaaacaac ccagaccgcc agctaaggtc ccaaagttgt ggttaagtgg taaacgatgt     960
   gggaaggct t agacagct ag gaggt t ggct t agaagcagc cat cct t t aa agaaagcgt a 1020
   at ag 1024
<210> 76
   <211> 1024
   <212> DNA
   <213> Pseudorronas aer ugi nosa
<400> 76
   ggtcaagtga agaagcgcat acggtggatg ccttggcagt cagaggcgat gaaagacgtg      60
   gt agcct gcg aaaagct t cg gggagt cggc aaacagact t t gat ccggag at ct ct gaat 120
   gggggaaccc acctaggata acctaggtat cttgtactga atccataggt gcaagaggcg     180
   aaccacgggg aactgaaaca tctaagtacc ctgaggaaaa gaaatcaacc gagattccct     240
   tagtagtggc gagcgaacgg ggattagccc ttaagcttca ttgattttag cggaacgctc     300
   t ggaaagt gc ggccat agt g ggt gat agcc ccgt acgcga aaggat ct t t gaagt gaaat 360
   cgagtaggac ggagcacgag aaactttgtc tgaacatggg gggaccatcc tccaaggcta    420
   aatactactg actgaccgat agtgaaccag taccgtgagg gaaaggcgaa aagaaccccg    480
   gagaggggag tgaaatagaa cctgaaaccg tatgcgtaca agcagtggga gcctacttgt    540
   t aggt gact g cgt acct t t t gt at aat ggg t cagcgact t at at t cagt g gcaagct t aa 600
   tcgtataggg taggcgtagc gaaaagcgag tcttaatagg gcgtttagtc gctgggtata    660
   gacccgaaac cgggcgatct atccatgagc aggttgaagg ttaggtaaca ctgactggag    720
   gaccgaaccc actcccgttg aaaaggtagg ggatgacttg tggatcggag tgaaaggcta    780
   at caagct cg gagat agct g gt t ct cct cg aaagct at t t aggt agcgcc t cat gt at ca 840
   ct ct gggggg t agagcaact gt t t cggct a gggggt cat c ccgact t acc aaaccgat gc 900
   aaactccgaa tacccagaag tgccgagcat gggagacaca cggcgggtgc taacgtccgt    960
   cgtgaaaagg gaaacaaccc agaccgccag ctaaggtccc aaagttgtgg ttaagtggta   1020
   aacg 1024
<210> 77
   <211> 1024
   <212> DNA
   <213> Staphylococcus aureus
<400> 77
   t gt aagt aag ct t t gat cca gagat t t ccg aat ggggaaa cccagcat ga gt t at gt cat 60
   gttatcgata tgtgaataca tagcatatca gaaggcacac ccggagaact gaaacatctt    120
   agtacccgga ggaagagaaa gaaaattcga ttcccttagt agcggcgagc gaaacgggaa    180
   gagcccaaac caacaagctt gcttgttggg gttgtaggac actctatacg gagttacaaa    240
   ggacgacat t agacgaat ca t ct ggaaaga t gaat caaag aaggt aat aa t cct gt agt c 300
   gaaaat gt t g t ct ct ct t ga gt ggat cct g agt acgacgg agcacgt gaa at t ccgt cgg 360
   aat ct gggag gaccat ct cc t aaggct aaa t act ct ct ag t gaccgat ag t gaaccagt a 420
   ccgtgaggga aaggtgaaaa gcaccccgga aggggagtga aatagaacct gaaaccgtgt    480
   gct t acaagt agt cagagcc cgt t aat ggg t gat ggcgt g cct t t t gt ag aat gaaccgg 540
   cgagt t acga t t t gat gcaa ggt t aagcag t aaat gt gga gccgt agcga aagcgagt ct 600
   gaatagggcg tttagtattt ggtcgtagac ccgaaaccag gtgatctacc cttggtcagg    660
   ttgaagttca ggtaacactg aatggaggac cgaaccgact tacgttgaaa agtgagcgga    720
   t gaact gagg gt agcggaga aat t ccaat c gaacct ggag at agct ggt t ct ct ccgaaa 780
   t agct t t agg gct agcct ca agt gat gat t at t ggaggt a gagcact gt t t ggacgaggg 840
   gcccct ct cg ggt t accgaa t t cagacaaa ct ccgaat gc caat t aat t t aact t gggag 900
   tcagaacatg ggtgataagg tccgtgttcg aaagggaaac agcccagacc accagctaag    960
   gtcccaaaat atatgttaag tggaaaagga tgtggcgttg cccagacaac taggatgttg   1020
   gct t 1024
<210> 78
   <211> 1024
   <212> DNA
   <213> Staphylococcus aureus
<400> 78
   tgtaagtaag ctttgatcca gagatttccg aatggggaaa cccagcatga gttatgtcat     60
   gttatcgata tgtgaataca tagcatatca gaaggcacac ccggagaact gaaacatctt    120
   agtacccgga ggaagagaaa gaaaattcga ttcccttagt agcggcgagc gaaacgggaa    180
   gagcccaaac caacaagctt gcttgttggg gttgtaggac actctatacg gagttacaaa    240
   ggacgacatt agacgaatca tctggaaaga tgaatcaaag aaggtaataa tcctgtagtc    300
   gaaaatgttg tctctcttga gtggatcctg agtacgacgg agcacgtgaa attccgtcgg    360
   aatctgggag gaccatctcc taaggctaaa tactctctag tgaccgatag tgaaccagta    420
   ccgtgaggga aaggtgaaaa gcaccccgga aggggagtga aatagaacct gaaaccgtgt    480
   gcttacaagt agtcagagcc cgttaatggg tgatggcgtg ccttttgtag aatgaaccgg    540
   cgagttacga tttgatgcaa ggttaagcag taaatgtgga gccgtagcga aagcgagtct    600
   gaatagggcg tttagtattt ggtcgtagac ccgaaaccag gtgatctacc cttggtcagg    660
   ttgaagttca ggtaacactg aatggaggac cgaaccgact tacgttgaaa agtgagcgga    720
   tgaactgagg gtagcggaga aattccaatc gaacctggag atagctggtt ctctccgaaa    780
   tagctttagg gctagcctca agtgatgatt attggaggta gagcactgtt tggacgaggg    840
   gcccctctcg ggttaccgaa ttcagacaaa ctccgaatgc caattaattt aacttgggag    900
   tcagaacatg ggtgataagg tccgtgttcg aaagggaaac agcccagacc accagctaag    960
   gtcccaaaat atatgttaag tggaaaagga tgtggcgttg cccagacaac taggatgttg   1020
   gctt                                                                1024
<210> 79
   <211> 1024
   <212> DNA
   <213> Staphylococcus aureus
<400> 79
   tgtaagtaag ctttgatcca gagatttccg aatggggaaa cccagcatga gttatgtcat 60
   gttatcgata tgtgaataca tagcatatca gaaggcacac ccggagaact gaaacatctt 120
   agtacccgga ggaagagaaa gaaaattcga ttcccttagt agcggcgagc gaaacgggaa 180
   gagcccaaac caacaagctt gcttgttggg gttgtaggac actctatacg gagttacaaa 240
   ggacgacatt agacgaatca tctggaaaga tgaatcaaag aaggtaataa tcctgtagtc 300
   gaaaatgttg tctctcttga gtggatcctg agtacgacgg agcacgtgaa attccgtcgg 360
   aatctgggag gaccatctcc taaggctaaa tactctctag tgaccgatag tgaaccagta 420
   ccgtgaggga aaggtgaaaa gcaccccgga aggggagtga aatagaacct gaaaccgtgt 480
   gcttacaagt agtcagagcc cgttaatggg tgatggcgtg ccttttgtag aatgaaccgg 540
   cgagttacga tttgatgcaa ggttaagcag taaatgtgga gccgtagcga aagcgagtct 600
   gaatagggcg tttagtattt ggtcgtagac ccgaaaccag gtgatctacc cttggtcagg 660
   ttgaagttca ggtaacactg aatggaggac cgaaccgact tacgttgaaa agtgagcgga 720
   tgaactgagg gtagcggaga aattccaatc gaacctggag atagctggtt ctctccgaaa 780
   tagctttagg gctagcctca agtgatgatt attggaggta gagcactgtt tggacgaggg 840
   gcccctctcg ggttaccgaa ttcagacaaa ctccgaatgc caattaattt aacttgggag 900
   tcagaacatg ggtgataagg tccgtgttcg aaagggaaac agcccagacc accagctaag 960
   gtcccaaaat atatgttaag tggaaaagga tgtggcgttg cccagacaac taggatgttg 1020
   gctt 1024
<210> 80
   <211> 1024
   <212> DNA
   <213> Staphylococcus aureus
<400> 80
   gattaagtta ttaagggcgc acggtggatg ccttggcact agaagccgat gaaggacgtt      60
   act aacgacg at at gct t t g gggagct gt a agt aagct t t gat ccagaga t t t ccgaat g 120
   gggaaaccca gcatgagtta tgtcatgtta tcgatatgtg aatacatagc atatcagaag     180
   gcacacccgg agaactgaaa catcttagta cccggaggaa gagaaagaaa attcgattcc     240
   cttagtagcg gcgagcgaaa cgggaagagc ccaaaccaac aagcttgctt gttggggttg     300
   t aggacact c t at acggagt t acaaaggac gacat t agac gaat cat ct g gaaagat gaa 360
   t caaagaagg t aat aat cct gt agt cgaaa at gt t gt ct c t ct t gagt gg at cct gagt a 420
   cgacggagca cgtgaaattc cgtcggaatc tgggaggacc atctcctaag gctaaatact     480
   ctctagtgac cgatagtgaa ccagtaccgt gagggaaagg tgaaaagcac cccggaaggg     540
   gagt gaaat a gaacct gaaa ccgt gt gct t acaagt agt c agagcccgt t aat gggt gat 600
   ggcgtgcctt ttgtagaatg aaccggcgag ttacgatttg atgcaaggtt aagcagtaaa     660
   t gt ggagccg t agcgaaagc gagt ct gaat agggcgt t t a gt at t t ggt c gt agacccga 720
   aaccaggtga tctacccttg gtcaggttga agttcaggta acactgaatg gaggaccgaa     780
   ccgact t acg t t gaaaagt g agcggat gaa ct gagggt ag cggagaaat t ccaat cgaac 840
   ct ggagat ag ct ggt t ct ct ccgaaat agc t t t agggct a gcct caagt g at gat t at t g 900
   gaggtagagc actgtttgga cgaggggccc ctctcgggtt accgaattca gacaaactcc     960
   gaat gccaat t aat t t aact t gggagt cag aacat gggt g at aaggt ccg t gt t cgaaag 1020
   ggaa 1024
<210> 81
   <211> 1024
   <212> DNA
   <213> Staphylococcus aureus
<400> 81
   cgacgat at g ct t t ggggag ct gt aagt aa gct t t gat cc agagat nt cc gaat ggggaa 60
   acccagcatg agttatgtca tgttatcgat atgtgaatac atagcatatc agaaggcaca     120
   cccggagaac tgaaacatct tagtacccgg aggaagagaa agaaaattcg attcccttag 180
   tagcggcgag cgaaacggga agagcccaaa ccaacaagct tgcttgttgg ggttgtagga 240
   cactctatac ggagttacaa aggacgacat tagacgaatc atctggaaag atgaatcaaa 300
   gaaggtaata atcctgtagt cgaaaatgtt gtctctcttg agtggatcct gagtacgacg 360
   gagcacgtga aattccgtcg gaatctggga ggaccatctc ctaaggctaa atactctcta 420
   gtgaccgata gtgaaccagt accgtgaggg aaaggtgaaa agcaccccgg aaggggagtg 480
   aaatagaacc tgaaaccgtg tgcttacaag tagtcagagc ccgttaatgg gtgatggcgt 540
   gccttttgta gaatgaaccg gcgagttacg atttgatgca aggttaagca gtaaatgtgg 600
   agccgtagcg aaagcgagtc tgaatagggc gtttagtatt tggtcgtaga cccgaaacca 660
   ggtgatctac ccttggtcag gttgaagttc aggtaacact gaatggagga ccgaaccgac 720
   ttacgttgaa aagtgagcgg atgaactgag ggtagcggag aaattccaat cgaacctgga 780
   gatagctggt tctctccgaa atagctttag ggctagcctc aagtgatgat tattggaggt 840
   agagcactgt ttggacgagg ggcccctctc gggttaccga attcagacaa actccgaatg 900
   ccaattaatt taacttggga gtcagaacat gggtgataag gtccgtgttc gaaagggaaa 960
   cagcccagac caccagctaa ggtcccaaaa tatatgttaa gtggaaaagg atgtggcgtt 1020
   gccc 1024
<210> 82
   <211> 1024
   <212> DNA
   <213> Staphylococcus aureus
<400> 82
   cgacgat at g ct t t ggggag ct gt aagt aa gct t t gat cc agagat t t cc gaat ggggaa 60
   acccagcatg agttatgtca tgttatcgat atgtgaatac atagcatatc agaaggcaca     120
   cccggagaac tgaaacatct tagtacccgg aggaagagaa agaaaattcg attcccttag     180
   tagcggcgag cgaaacggga agagcccaaa ccaacaagct tgcttgttgg ggttgtagga     240
   cactctatac ggagttacaa aggacgacat tagacgaatc atctggaaag atgaatcaaa     300
   gaaggt aat a at cct gt agt cgaaaat gt t gt ct ct ct t g agt ggat cct gagt acgacg 360
   gagcacgt ga aat t ccgt cg gaat ct ggga ggaccat ct c ct aaggct aa at act ct ct a 420
   gtgaccgata gtgaaccagt accgtgaggg aaaggtgaaa agcaccccgg aaggggagtg     480
   aaat agaacc t gaaaccgt g t gct t acaag t agt cagagc ccgt t aat gg gt gat ggcgt 540
   gcct t t t gt a gaat gaaccg gcgagt t acg at t t gat gca aggt t aagca gt aaat gt gg 600
   agccgtagcg aaagcgagtc tgaatagggc gtttagtatt tggtcgtaga cccgaaacca     660
   ggtgatctac ccttggtcag gttgaagttc aggtaacact gaatggagga ccgaaccgac     720
   t t acgt t gaa aagt gagcgg at gaact gag ggt agcggag aaat t ccaat cgaacct gga 780
   gat agct ggt t ct ct ccgaa at agct t t ag ggct agcct c aagt gat gat t at t ggaggt 840
   agagcact gt t t ggacgagg ggcccct ct c gggt t accga at t cagacaa act ccgaat g 900
   ccaattaatt taacttggga gtcagaacat gggtgataag gtccgtgttc gaaagggaaa     960
   cagcccagac caccagctaa ggtcccaaaa tatatgttaa gtggaaaagg atgtggcgtt    1020
   gccc 1024
<210> 83
   <211> 1024
   <212> DNA
   <213> Staphylococcus aureus
<400> 83
   t gt aagt aag ct t t gat cca gagat t t ccg aat ggggaaa cccagcat ga gt t at gt cat 60
   gttatcgata tgtgaataca tagcatatca gaaggcacac ccggagaact gaaacatctt 120
   agtacccgga ggaagagaaa gaaaattcga ttcccttagt agcggcgagc gaaacgggaa 180
   gagcccaaac caacaagctt gcttgttggg gttgtaggac actctatacg gagttacaaa 240
   ggacgacat t agacgaat ca t ct ggaaaga t gaat caaag aaggt aat aa t cct gt agt c 300
   gaaaat gt t g t ct ct ct t ga gt ggat cct g agt acgacgg agcacgt gaa at t ccgt cgg 360
   aat ct gggag gaccat ct cc t aaggct aaa t act ct ct ag t gaccgat ag t gaaccagt a 420
   ccgtgaggga aaggtgaaaa gcaccccgga aggggagtga aatagaacct gaaaccgtgt 480
   gct t acaagt agt cagagcc cgt t aat ggg t gat ggcgt g cct t t t gt ag aat gaaccgg 540
   cgagt t acga t t t gat gcaa ggt t aagcag t aaat gt gga gccgt agcga aagcgagt ct 600
   gaatagggcg tttagtattt ggtcgtagac ccgaaaccag gtgatctacc cttggtcagg 660
   ttgaagttca ggtaacactg aatggaggac cgaaccgact tacgttgaaa agtgagcgga 720
   t gaact gagg gt agcggaga aat t ccaat c gaacct ggag at agct ggt t ct ct ccgaaa 780
   t agct t t agg gct agcct ca agt gat gat t at t ggaggt a gagcact gt t t ggacgaggg 840
   gcccct ct cg ggt t accgaa t t cagacaaa ct ccgaat gc caat t aat t t aact t gggag 900
   tcagaacatg ggtgataagg tccgtgttcg aaagggaaac agcccagacc accagctaag 960
   gtcccaaaat atatgttaag tggaaaagga tgtggcgttg cccagacaac taggatgttg 1020
   gct t 1024
<210> 84
   <211> 1024
   <212> DNA
   <213> Streptococcus pneurroni ae
<400> 84
   ggttaagtta ataagggcgc acggtggatg ccttggcact aggagccgac gaaggacgtg 60
   acaaacgacg atatgccttg ggtagctgta agtaagcgat gatccatgga tttccgaatg 120
   ggggaaccca acaggtaata cctgttaccc acatctgtta aggatgtgag gaggaagacg 180
   cagtgaactg aaacatctaa gtagctgcag gaagagaaag caaaagcgat tgccttagta 240
   gcggcgagcg aaacggcaga agggcaaacc gaagagttta ctcttcgggg ttgtaggact 300
   gcaat gt gga ct caaagat t at agaagaat gat t t gggaa gat cagccaa agagagt aat 360
   agcct cgt at t t aaaat agt ct t t gt acct agcagt at cc t gagt acggc gggacacgt g 420
   aaat cccgt c ggaat ct ggg aggaccat ct cccaaccct a aat act ccct agt gaccgat 480
   agtgaaccag taccgtgagg gaaaggtgaa aagcaccccg ggaggggagt gaaatagaac     540
   ct gaaaccgt gt gcct acaa caagt t cgag cccgt t aat g ggt gagagcg t gcct t t t gt 600
   agaat gaacc ggcgagt t ac gt t at gat gc gaggt t aagt t gaagagacg gagccgt agg 660
   gaaaccgagt ctgaataggg cgccttagta tcatgacgta gacccgaaac catgtgacct     720
   acccatgagc aggttgaagg tgcggtaaga cgcactggag gaccgaacca gggcacgttg     780
   aaaagt gct t ggat gact t g t gggt agcgg agaaat t cca aacgaact t g gagat agct g 840
   gt t ct ct ccg aaat agct t t agggct agcg t cgacat t ag agat t ct t gg aggt agagca 900
   ct gt t t gggt gaggggt cca t cccggat t a ccaat ct cag at aaact ccg aat gccaat g 960
   aattatggtc ggcagtcaga ctgcgagtgc taagatccgt agtcgaaagg gaaacagccc    1020
   agac 1024
<210> 85
   <211> 1024
   <212> DNA
   <213> Streptococcus pneumoniae
<400> 85
   cgacgat at g cct t gggt ag ct gt aagt aa gcgat gat cc agggat t t cc gaat ggggga 60
   acccaacagg taatacctgt tacccacatc tgttaaggat gtgaggagga agacgcagtg     120
   aactgaaaca tctaagtagc tgcaggaaga gaaagcaaaa gcgattgcct tagtagcggc     180
   gagcgaaacg gcagaagggc aaaccgaaga gtttactctt cggggttgta ggactgcaat     240
   gtggactcaa agattataga agaatgattt gggaagatca gccaaagaga gtaatagcct     300
   cgtatttaaa atagtctttg tacctagcag tatcctgagt acggcgggac acgtgaaatc     360
   ccgt cggaat ct gggaggac cat ct cccaa ccct aaat ac t ccct agt ga ccgat agt ga 420
   accagtaccg tgagggaaag gtgaaaagca ccccgggagg ggagtgaaat agaacctgaa     480
   accgt gt gcc t acaacaagt t cgagcccgt t aat gggt ga gagcgt gcct t t t gt agaat 540
   gaaccggcga gttacgttat gatgcgaggt taagttgaag agacggagcc gtagggaaac     600
   cgagt ct gaa t agggcgcct t agt at cat g acgt agaccc gaaaccat gt gacct accca 660
   tgagcaggtt gaaggtgcgg taagacgcac tggaggaccg aaccagggca cgttgaaaag     720
   tgcttggatg acttgtgggt agcggagaaa ttccaaacga acttggagat agctggttct     780
   ct ccgaaat a gct t t agggc t agcgt cgac at t agagat t ct t ggaggt a gagcact gt t 840
   t gggt gaggg gt ccat cccg gat t accaat ct cagat aaa ct ccgaat gc caat gaat t a 900
   tggtcggcag tcagactgcg agtgctaaga tccgtagtcg aaagggaaac agcccagacc     960
   accagctaag gtcccaaaat aattgttaag tggaaaagga tgtggggttg cacagacaac    1020
   t agg 1024
<210> 86
   <211> 1024
   <212> DNA
   <213> Streptococcus pneumoniae
<400> 86
   cgacgat at g  cct t gggt ag  ct gt aagt aa  gcgat gat cc  agggat t t cc  gaat ggggga  60
   acccaacagg taatacctgt tacccacatc tgttaaggat gtgaggagga agacgcagtg     120
   aactgaaaca tctaagtagc tgcaggaaga gaaagcaaaa gcgattgcct tagtagcggc     180
   gagcgaaacg gcagaagggc aaaccgaaga gtttactctt cggggttgta ggactgcaat     240
   gtggactcaa agattataga agaatgattt gggaagatca gccaaagaga gtaatagcct     300
   cgtatttaaa atagtctttg tacttagcag tatcctgagt acggcgggac acgtgaaatc     360
   ccgt cggaat  ct gggaggac  cat ct cccaa  ccct aaat ac  t ccct agt ga  ccgat agt ga  420
   accagt accg  t gagggaaag  gt gaaaagca  ccccgt gagg  ggagt gaaat  agaacct gaa  480
   accgt gt gcc  t acaacaagt  t cgagcccgt  t aat gggt ga  gagcgt gcct  t t t gt agaat  540
   gaaccggcga gttacgttat gatgcgaggt taagttgaag agacggagcc gtagggaaac     600
   cgagt ct gaa  t agggcgcct  t agt at cat g  acgt agaccc  gaaaccat gt  gacct accca  660
   tgagcaggtt gaaggtgcgg taagacgcac tggaggaccg aaccagggca cgttgaaaag     720
   tgcttggatg acttgtgggt agcggagaaa ttccaaacga acttggagat agctggttct     780
   ct ccgaaat a  gct t t agggc  t agcgt cgac  at t agagat t  ct t ggaggt a  gagcact gt t  840
   t gggt gaggg  gt ccat cccg  gat t accaat  ct cagat aaa  ct ccgaat gc  caat gaat t a  900
   tggtcggcag tcagactgcg agtgctaaga tccgtagtcg aaagggaaac agcccagacc     960
   accagctaag gtcccaaaat aattgttaag tggaaaagga tgtggggttg cacagacaac    1020
   t agg  1024
<210> 87
   <211> 1024
   <212> DNA
   <213> Streptococcus pneumoniae
<400> 87
   cgacgatatg ccttgggtag ctgtaagtaa gcgatgatcc agggatttcc gaatggggga     60
   acccaacagg taatacctgt tacccacatc tgttaaggat gtgaggagga agacgcagtg    120
   aactgaaaca tctaagtagc tgcaggaaga gaaagcaaaa gcgattgcct tagtagcggc    180
   gagcgaaacg gcagaagggc aaaccgaaga gtttactctt cggggttgta ggactgcaat    240
   gtggactcaa agattataga agaatgattt gggaagatca gccaaagaga gtaatagcct    300
   cgtatttaaa atagtctttg tacctagcag tatcctgagt acggcgggac acgtgaaatc    360
   ccgtcggaat ctgggaggac catctcccaa ccctaaatac tccctagtga ccgatagtga    420
   accagtaccg tgagggaaag gtgaaaagca ccccgggagg ggagtgaaat agaacctgaa    480
   accgtgtgcc tacaacaagt tcgagcccgt taatgggtga gagcgtgcct tttgtagaat    540
   gaaccggcga gttacgttat gatgcgaggt taagttgaag agacggagcc gtagggaaac    600
   cgagtctgaa cagggcgaat tagtatcatg acgtanaccc gaaaccatgt gacctaccca    660
   tgagcaggtt gaaggtgcgg taagacgcac tggaggaccg aaccagggca cgttgaaaag    720
   tgcttggatg acttgtgggt agcggagaaa ttccaaacga acttggagat agctggttct    780
   ctccgaaata gctttagggc tagcgtcgac attagagatt cttggaggta gagcactgtt    840
   tgggtgaggg gtccatcccg gattaccaat ctcagataaa ctccgaatgc caatgaatta    900
   tggtcggcag tcagactgcg agtgctaaga tccgtagtcg aaagggaaac agcccagacc    960
   accagctaag gtcccaaaat aattgttaag tggaaaagga tgtggggttg cacagacaac   1020
   tagg                                                                1024
<210> 88
   <211> 1024
   <212> DNA
   <213> Streptococcus pneumoniae
<400> 88
   cgacgat at g cct t gggt ag ct gt aagt aa gcgat gat cc agggat t t cc gaat ggggga 60
   acccaacagg taatacctgt tacccacatc tgttaaggat gtgaggagga agacgcagtg     120
   aactgaaaca tctaagtagc tgcaggaaga gaaagcaaaa gcgattgcct tagtagcggc     180
   gagcgaaacg gcagaagggc aaaccgaaga gtttactctt cggggttgta ggactgcaat     240
   gtggactcaa agattataga agaatgattt gggaagatca gccaaagaga gtaatagcct     300
   cgtatttaaa atagtctttg tacttagcag tatcctgagt acggcgggac acgtgaaatc     360
   ccgt cggaat ct gggaggac cat ct cccaa ccct aaat ac t ccct agt ga ccgat agt ga 420
   accagtaccg tgagggaaag gtgaaaagca ccccgggagg ggagtgaaat agaacctgaa     480
   accgt gt gcc t acaacaagt t cgagcccgt t aat gggt ga gagcgt gcct t t t gt agaat 540
   gaaccggcga gttacgttat gatgcgaggt taagttgaag agacggagcc gtagggaaac     600
   cgagt ct gaa t agggcgcct t agt at cat g acgt agaccc gaaaccat gt gacct accca 660
   tgagcaggtt gaaggtgcgg taagacgcac tggaggaccg aaccagggca cgttgaaaag     720
   tgcttggatg acttgtgggt agcggagaaa ttccaaacga acttggagat agctggttct     780
   ct ccgaaat a gct t t agggc t agcgt cgac at t agagat t ct t ggaggt a gagcact gt t 840
   t gggt gaggg gt ccat cccg gat t accaat ct cagat aaa ct ccgaat gc caat gaat t a 900
   tggtcggcag tcagactgcg agtgctaaga tccgtagtcg aaagggaaac agcccagacc     960
   accagctaag gtcccaaaat aattgttaag tggaaaagga tgtggggttg cacagacaac    1020
   t agg 1024
<210> 89
   <211> 1024
   <212> DNA
   <213> Streptococcus pneumoniae
<400> 89
   cgacgat at g cct t gggt ag ct gt aagt aa gcgat gat cc agggat t t cc gaat ggggga 60
   acccaacagg taatacctgt tacccacatc tgttaaggat gtgaggagga agacgcagtg     120
   aactgaaaca tctaagtagc tgcaggaaga gaaagcaaaa gcgattgcct tagtagcggc     180
   gagcgaaacg gcagaagggc aaaccgaaga gtttactctt cggggttgta ggactgcaat    240
   gtggactcaa agattataga agaatgattt gggaagatca gccaaagaga gtaatagcct    300
   cgtatttaaa atagtctttg tacctagcag tatcctgagt acggcgggac acgtgaaatc    360
   ccgt cggaat ct gggaggac cat ct cccaa ccct aaat ac t ccct agt ga ccgat agt ga 420
   accagtaccg tgagggaaag gtgaaaagca ccccgggagg ggagtgaaat agaacctgaa    480
   accgt gt gcc t acaacaagt t cgagcccgt t aat gggt ga gagcgt gcct t t t gt agaat 540
   gaaccggcga gttacgttat gatgcgaggt taagttgaag agacggagcc gtagggaaac    600
   cgagt ct gaa t agggcgcct t agt at cat g acgt agaccc gaaaccat gt gacct accca 660
   tgagcaggtt gaaggtgcgg taagacgcac tggaggaccg aaccagggca cgttgaaaag    720
   tgcttggatg acttgtgggt agcggagaaa ttccaaacga acttggagat agctggttct    780
   ct ccgaaat a gct t t agggc t agcgt cgac at t agagat t ct t ggaggt a gagcact gt t 840
   t gggt gaggg gt ccat cccg gat t accaat ct cagat aaa ct ccgaat gc caat gaat t a 900
   tggtcggcag tcagactgcg agtgctaaga tccgtagtcg aaagggaaac agcccagacc    960
   accagctaag gtcccaaaat aattgttaag tggaaaagga tgtggggttg cacagacaac   1020
   t agg 1024
<210> 90
   <211> 1024
   <212> DNA
   <213> Streptococcus pneumoniae
<400> 90
   cgacgat at g cct t gggt ag ct gt aagt aa gcgat gat cc agggat t t cc gaat ggggga 60
   acccaacagg taatacctgt tacccacatc tgttaaggat gtgaggagga agacgcagtg     120
   aactgaaaca tctaagtagc tgcaggaaga gaaagcaaaa gcgattgcct tagtagcggc     180
   gagcgaaacg gcaggagggc aaaccgaaga gtttactctt cggggttgta ggactgcaat     240
   gtggactcaa agattataga agaatgattt gggaagatca gccaaagaga gtaatagcct     300
   cgtatttaaa atagtctttg tacttagcag tatcctgagt acggcgggac acgtgaaatc     360
   ccgt cggaat ct gggaggac cat ct cccaa ccct aaat ac t ccct agt ga ccgat agt ga 420
   accagtaccg tgagggaaag gtgaaaagca ccccgggagg ggagtgaaat agaacctgaa     480
   accgt gt gcc t acaacaagt t cgagcccgt t aat gggt ga gagcgt gcct t t t gt agaat 540
   gaaccggcga gttacgttat gatgcgaggt taagttgaag agacggagcc gtagggaaac     600
   cgagt ct gaa t agggcgcct t agt at cat g acgt agaccc gaaaccat gt gacct accca 660
   tgagcaggtt gaaggtgcgg taagacgcac tggaggaccg aaccagggca cgttgaaaag     720
   tgcttggatg acttgtgggt agcggagaaa ttccaaacga acttggagat agctggttct     780
   ct ccgaaat a gct t t agggc t agcgt cgac at t agagat t ct t ggaggt a gagcact gt t 840
   t gggt gaggg gt ccat cccg gat t accaat ct cagat aaa ct ccgaat gc caat gaat t a 900
   tggtcggcag tcagactgcg agtgctaaga tccgtagtcg aaagggaaac agcccagacc     960
   accagctaag gtcccaaaat aattgttaag tggaaaagga tgtggggttg cacagacaac    1020
   t agg 1024
<210> 91
   <211> 1024
   <212> DNA
   <213> Streptococcus pneumoniae
<400> 91
   cgacgat at g  cct t gggt ag  ct gt aagt aa  gcgat gat cc  agggat t t cc  gaat ggggga     60
   acccaacagg taatacctgt tacccacatc tgttaaggat gtgaggagga agacgcagtg 120
   aactgaaaca tctaagtagc tgcaggaaga gaaagcaaaa gcgattgcct tagtagcggc 180
   gagcgaaacg gcaggagggc aaaccgaaga gtttactctt cggggttgta ggactgcaat 240
   gtggactcaa agattataga agaatgattt gggaagatca gccaaagaga gtaatagcct 300
   cgtatttaaa atagtctttg tacttagcag tatcctgagt acggcgggac acgtgaaatc 360
   ccgt cggaat  ct gggaggac  cat ct cccaa  ccct aaat ac  t ccct agt ga  ccgat agt ga    420
   accagtaccg tgagggaaag gtgaaaagca ccccgggagg ggagtgaaat agaacctgaa 480
   accgt gt gcc  t acaacaagt  t cgagcccgt  t aat gggt ga  gagcgt gcct  t t t gt agaat    540
   gaaccggcga gttacgttat gatgcgaggt taagttgaag agacggagcc gtagggaaac 600
   cgagt ct gaa  t agggcgcct  t agt at cat g  acgt agaccc  gaaaccat gt  gacct accca    660
   tgagcaggtt gaaggtgcgg taagacgcac tggaggaccg aaccagggca cgttgaaaag 720
   tgcttggatg acttgtgggt agcggagaaa ttccaaacga acttggagat agctggttct 780
   ct ccgaaat a  gct t t agggc  t agcgt cgac  at t agagat t  ct t ggaggt a  gagcact gt t    840
   t gggt gaggg  gt ccat cccg  gat t accaat  ct cagat aaa  ct ccgaat gc  caat gaat t a    900
   tggtcggcag tcagactgcg agtgctaaga tccgtagtcg aaagggaaac agcccagacc 960
   accagctaag gtcccaaaat aattgttaag tggaaaagga tgtggggttg cacagacaac 1020
   t agg                                                                     1024
<210> 92
   <211> 1024
   <212> DNA
   <213> Streptococcus pneumoniae
<400> 92
   cgacgat at g  cct t gggt ag  ct gt aagt aa  gcgat gat cc  agggat t t cc  gaat ggggga     60
   acccaacagg taatacctgt tacctacatc tgttaaggat gtgaggagga agacgcagtg 120
   aactgaaaca tctaagtagc tgcaggaaga gaaagcaaaa gcgattgcct tagtagcggc 180
   gagcgaaacg gcagaagggc aaaccgaaga gtttactctt cggggttgta ggactgcaat 240
   gtggactcaa agattataga agaatgattt gggaagatca gccaaagaga gtaatagcct 300
   cgtatttaaa atagtctttg tacttagcag tatcctgagt acggcgggac acgtgaaatc 360
   ccgt cggaat  ct gggaggac  cat ct cccaa  ccct aaat ac  t ccct agt ga  ccgat agt ga    420
   accagtaccg tgagggaaag gtgaaaagca ccccgggagg ggagtgaaat agaacctgaa     480
   accgt gt gcc t acaacaagt t cgagcccgt t aat gggt ga gagcgt gcct t t t gt agaat 540
   gaaccggcga gttacgttat gatgcgaggt taagttgaag agacggagcc gtagggaaac     600
   cgagt ct gaa t agggcgcct t agt at cat g acgt agaccc gaaaccat gt gacct accca 660
   tgagcaggtt gaaggtgcgg taagacgcac tggaggaccg aaccagggca cgttgaaaag     720
   tgcttggatg acttgtgggt agcggagaaa ttccaaacga acttggagat agctggttct     780
   ct ccgaaat a gct t t agggc t agcgt cgac at t agagat t ct t ggaggt a gagcact gt t 840
   t gggt gaggg gt ccat cccg gat t accaat ct cagat aaa ct ccgaat gc caat gaat t a 900
   tggtcggcag tcagactgcg agtgctaaga tccgtagtcg aaagggaaac agcccagacc     960
   accagctaag gtcccaaaat aattgttaag tggaaaagga tgtggggttg cacagacaac    1020
   t agg 1024
<210> 93
   <211> 1024
   <212> DNA
   <213> Streptococcus pneumoniae
<400> 93
<210> 94
   <211> 1024
   <212> DNA
   <213> Streptococcus pneumoniae
<400> 94
   cgacgatatg ccttgggtgc tgtaagtaag cgatgatcca gggatttccg aatgggggaa     60
   cccaacaggt aatacctgtt tacccacatc tgttaaggat gtgaggagga agacgcagtg    120
   aactgaaaca tctaagtagc tgcnggaaga gaaagcaaaa gcgattgcct tagtagcggc    180
   gagcgaaacg gcagaagggc aaaccgaaga gtttactctt cggggttgta ggactgcaat    240
   gtggactcaa agattataga agaatgattt gggaagatca gccaaagaga gtaatagcct    300
   cgtatttaaa atagtctttg tacctagcag tatcctgagt acggcgggac acgtgaaatc    360
   ccgtcggaat ctgggaggac catctcccaa ccctaaatac tccctagtga ccgatagtga    420
   accagtaccg tgagggaaag gtgaaaagca ccccgggagg ggagtgaaat agaacctgaa    480
   accgtgtgcc tacaacaagt tcgagcccgt taatgggtga gagcgtgcct tttgtagaat    540
   gaaccggcga gttacgttat gatgcgaggt taagttgaag agacggagcc gtagggaaac    600
   cgagtctgaa tagggcgcct tagtatcatg acgtagaccc gaaaccatgt gacctaccca    660
   tgagcaggtt gaaggtgcgg taagacgcac tggaggaccg aaccagggca cgttgaaaag    720
   tgcttggatg acttgtgggt agcggagaaa ttccaaacga acttggagat agctggttct    780
   ctccgaaata gctttagggc tagcgtcgac attagagatt cttggaggta gagcactgtt    840
   tgggtgaggg gtccatcccg gattaccaat ctcagataaa ctccgaatgc caatgaatta    900
   tggtcggcag tcagactgcg agtgctaaga tccgtagtcg aaagggaaac agcccagacc    960
   accagctaag gtcccaaaat aattgttaag tggaaaagga tgtggggttg cacagacaac   1020
   tagg                                                                1024

## Description

### Technical Field

This application claims priority to Korean Patent Application No. 10-2005-0130610, filed December 27, 2005.

The present invention relates to a primer set for amplifying target sequence(s) of nine respiratory disease-associated bacterial species, and a method of detecting the nine respiratory disease-associated bacterial species using the probe set.

### Background Art

Typically, two sufficiently complementary single-stranded nucleic acids can hybridize to form a double helical structure in which the two antiparallel nucleic acid chains are held together by hydrogen bonds between complementary bases under conditions that promote their hybridization. Under appropriate conditions, DNA/DNA, RNA/DNA, or RNA/RNA hybrids may be formed.

A 'probe' is a single-stranded nucleic acid sequence that is complementary to some particular degree with a nucleic acid sequence ('target') to be detected. When needed, a probe may be labeled. The use of nucleic acid hybridization as a procedure for the detection of particular nucleic acid sequences is disclosed in U.S. Patent No. 4,851,330, and No. 5,288,611.

Broadly, there are two fundamental nucleic acid hybridization procedures. In one procedure, known as 'in-solution' hybridization, both a 'probe' nucleic acid and a 'target' nucleic acid in a test sample are free in solution. In the other procedure, one nucleic acid is immobilized in or on a solid substrate and the second nucleic acid is free in solution. For example, the position of a target nucleic acid present in a gel after electrophoresis may be located by using a solution of labeled probe nucleic acid that can hybridize with the target nucleic acid in the gel under appropriate conditions.

Probes for the detection of a few respiratory disease-associated bacteria are currently known. For example, U.S. Patent No. 5,830,654 discloses hybridization assay probes for *Haemophilus influenzae* comprising oligonucleotides of about 14-18 nucleotides. U.S. Patent No. 5,525,718 discloses oligonucleotides selectively hybridizing with a specific gene (e.g., the entE gene) of *Staphylococcus aureus.* U.S. Patent No. 6,001,564 discloses primers or probes specific to *Escherichia coli, Klebsiella pneumoniae, Pseudomonas aeruginosa, Proteus mirabilis, Streptococcus pneumoniae, Staphylococcus aureus, Staphylococcus epidermis, Haemophilus influenzae,* and *Moraxella catarrhalis.*

Anthony et al. (Journal of Clinical Microbiology, Feb. 2000, p. 781-788) discloses an oligonucleotide primer set specific for bacterial 23S rDNA and suitable for amplifying a conserved region of said bacterial 23S rDNA from most different bacterial species followed by detection of the different species by microarray hybridization.

However, no primer set capable of amplifying target sequences commonly found in the 23S rRNA genes of nine bacterial species (*Chlamydophila pneumoniae, Haemophilus influenza, Klebsiella pneumoniae, Legionella pneumophila, Moraxella catarrhalis, Mycoplasma pneumoniae, Pseudomonas aeruginosa, Staphylococcus aureus,* and *Streptococcus pneumoniae*) known to be associated with respiratory disease is reported. Furthermore, no probe(s) specific to the target sequence(s) of the 23S rRNA genes of the nine bacterial species is reported. The probes and primers are used in a specific and selective method for detecting the presence or absence of a bacterial species associated with respiratory disease.

### Disclosure of Invention

### Technical Problem

The present invention provides a primer set for amplifying target sequence(s) of nine respiratory disease-associated bacterial species according to claim 1.

The oligonucleotide primer set comprises an oligonucleotide set comprising an oligonucleotide consisting of at least 10 contiguous nucleotides of SEQ ID NO: 1 and an oligonucleotide consisting of at least 10 contiguous nucleotides of SEQ ID NO: 2; an oligonucleotide set comprising an oligonucleotide consisting of at least 10 contiguous nucleotides of SEQ ID NO: 3 and an oligonucleotide consisting of at least 10 contiguous nucleotides of SEQ ID NO: 4; an oligonucleotide set comprising an oligonucleotide consisting of at least 10 contiguous nucleotides of SEQ ID NO: 5 and an oligonucleotide consisting of at least 10 contiguous nucleotides of a sequence selected from the group consisting of SEQ ID NO: 6 and SEQ ID NO:7; and an oligonucleotide set comprising an oligonucleotide consisting of at least 10 contiguous nucleotides of SEQ ID NO: 8 and an oligonucleotide consisting of at least 10 contiguous nucleotides of SEQ ID NO: 9.

Also disclosed is a probe set for detecting one or more of nine respiratory disease-associated bacterial species. The probe set is specific to target sequence(s) amplified by the primer set.

The oligonucleotide probe set comprises at least one oligonucleotide probe selected from the group consisting of an oligonucleotide probe comprising an oligonucleotide consisting of at least 10 contiguous nucleotides of SEQ ID NO: 10 or a complement of the oligonucleotide; an oligonucleotide probe comprising an oligonucleotide consisting of at least 10 contiguous nucleotides of a sequence selected from the group consisting of SEQ ID NOS: 11-14 or a complement of the oligonucleotide; an oligonucleotide probe comprising an oligonucleotide consisting of at least 10 contiguous nucleotides of SEQ ID NO: 15 or a complement of the oligonucleotide; an oligonucleotide probe comprising an oligonucleotide consisting of at least 10 contiguous nucleotides of a sequence selected from the group consisting of SEQ ID NOS: 16-18 or a complement of the oligonucleotide; an oligonucleotide probe comprising an oligonucleotide consisting of at least 10 contiguous nucleotides of SEQ ID NO: 19 or a complement of the oligonucleotide; an oligonucleotide probe comprising an oligonucleotide consisting of at least 10 contiguous nucleotides of SEQ ID NO: 20 or a complement of the oligonucleotide; an oligonucleotide probe comprising an oligonucleotide consisting of at least 10 contiguous nucleotides of a sequence selected from the group consisting of SEQ ID NOS: 21-23 or a complement of the oligonucleotide; an oligonucleotide probe comprising an oligonucleotide consisting of at least 10 contiguous nucleotides of a sequence selected from the group consisting of SEQ ID NOS: 24-26 or a complement of the oligonucleotide; an oligonucleotide probe comprising an oligonucleotide consisting of at least 10 contiguous nucleotides of SEQ ID NO: 27 or a complement of the oligonucleotide; an oligonucleotide probe comprising an oligonucleotide consisting of at least 10 contiguous nucleotides of SEQ ID NO: 28 or a complement of the oligonucleotide; an oligonucleotide probe comprising an oligonucleotide consisting of at least 10 contiguous nucleotides of SEQ ID NO: 29 or a complement of the oligonucleotide; an oligonucleotide probe comprising an oligonucleotide consisting of at least 10 contiguous nucleotides of a sequence selected from the group consisting of SEQ ID NOS: 30-31 or a complement of the oligonucleotide; and an oligonucleotide probe comprising an oligonucleotide consisting of at least 10 contiguous nucleotides of a sequence selected from the group consisting of SEQ ID NOS: 32-35 or a complement of the oligonucleotide

Also disclosed is a microarray comprising the probe set and a method of detecting one or more of the nine respiratory disease-associated bacterial species using the probe set.

### Technical Solution

The present invention provides an oligonucleotide primer set for amplifying at least one target sequence of a 23S rRNA gene of at least one bacterial species selected from the group consisting of *Chlamydophila pneumoniae, Haemophilus influenzae, Mycoplasma pneumoniae, Klebsiella pneumoniae, Legionella pneumophila, Moraxella catarrhalis, Pseudomonas aeruginosa, Staphylococcus aureus,* and *Streptococcus pneumoniae.* The oligonucleotide primer set comprises an oligonucleotide set comprising an oligonucleotide consisting of at least 10 contiguous nucleotides of SEQ ID NO: 1 and an oligonucleotide consisting of at least 10 contiguous nucleotides of SEQ ID NO: 2; an oligonucleotide set comprising an oligonucleotide consisting of at least 10 contiguous nucleotides of SEQ ID NO: 3 and an oligonucleotide consisting of at least 10 contiguous nucleotides of SEQ ID NO: 4; an oligonucleotide set comprising an oligonucleotide consisting of at least 10 contiguous nucleotides of SEQ ID NO: 5 and an oligonucleotide consisting of at least 10 contiguous nucleotides of a sequence selected from the group consisting of SEQ ID NO: 6 and SEQ ID NO:7; and an oligonucleotide set comprising an oligonucleotide consisting of at least 10 contiguous nucleotides of SEQ ID NO: 8 and an oligonucleotide consisting of oat least 10 contiguous nucleotides of SEQ ID NO: 9.

The target sequence for the primer set of the invention can be at least one sequence selected from the group consisting of a nucleotide sequence corresponding to positions 124-365 (SEQ ID NO:A), a nucleotide sequence corresponding to positions 853-1353 (SEQ ID NO:B), a nucleotide sequence corresponding to positions 2483-2932 (SEQ ID NO:C), and a nucleotide sequence corresponding to positions 3041-3198 (SEQ ID NO:D) of bacterial 23s rRNA.

The primer set of the disclosure can be an oligonucleotide primer set for amplifying a nucleotide region corresponding to positions 124-365 of the 23S rRNA gene of at least one bacterial species selected from the group consisting of *Chlamydophila pneumoniae, Haemophilus influenzae, Mycoplasma pneumoniae, Klebsiella pneumoniae, Legionella pneumophila, Moraxella catarrhalis, Pseudomonas aeruginosa, Staphylococcus aureus,* and *Streptococcus pneumoniae,* comprising an oligonucleotide consisting of at least 10 contiguous nucleotides of SEQ ID NO: 1 and an oligonucleotide consisting of at least 10 contiguous nucleotides of SEQ ID NO: 2.

The primer set of the disclosure can be an oligonucleotide primer set for amplifying a nucleotide region corresponding to positions 853-1353 of the 23S rRNA gene of at least one bacterial species selected from the group consisting of *Chlamydophila pneumoniae, Haemophilus influenzae, Mycoplasma pneumoniae, Klebsiella pneumoniae, Legionella pneumophila, Moraxella catarrhalis, Pseudomonas aeruginosa, Staphylococcus aureus,* and *Streptococcus pneumoniae,* comprising an oligonucleotide consisting of at least 10 contiguous nucleotides of SEQ ID NO: 3 and an oligonucleotide consisting of at least 10 contiguous nucleotides of SEQ ID NO: 4.

The primer set of the disclosure can be an oligonucleotide primer set for amplifying a nucleotide region corresponding to positions 2483-2932 of the 23S rRNA gene of at least one bacterial species selected from the group consisting of *Chlamydophila pneumoniae, Haemophilus influenzae, Mycoplasma pneumoniae, Klebsiella pneumoniae, Legionella pneumophila, Moraxella catarrhalis, Pseudomonas aeruginosa, Staphylococcus aureus,* and *Streptococcus pneumoniae,* comprising an oligonucleotide set comprising an oligonucleotide consisting of at least 10 contiguous nucleotides of SEQ ID NO: 5 and an oligonucleotide consisting of at least 10 contiguous nucleotides of a sequence selected from the group consisting of SEQ ID NO: 6 and SEQ ID NO:7.

The primer set of the disclosure can be an oligonucleotide primer set for amplifying a nucleotide region corresponding to positions 3041-3198 of the 23S rRNA gene of at least one bacterial species selected from the group consisting of *Chlamydophila pneumoniae, Haemophilus influenza, Mycoplasma pneumoniae, Klebsiella pneumoniae, Legionella pneumophila, Moraxella catarrhalis, Pseudomonas aeruginosa, Staphylococcus aureus,* and *Streptococcus pneumoniae.* In an embodiment, the oligonucleotide primer set comprises an oligonucleotide set comprising an oligonucleotide consisting of at least 10 contiguous nucleotides of SEQ ID NO: 8 and an oligonucleotide consisting of at least 10 contiguous nucleotides of SEQ ID NO: 9.

The primer set of the disclosure can be an oligonucleotide primer set for amplifying nucleotide regions corresponding to positions 124-365, 853-1353, 2483-2932, and 3041-3198 of the 23S rRNA gene of at least one bacterial species selected from the group consisting of *Chlamydophila pneumoniae, Haemophilus influenza, Mycoplasma pneumoniae, Klebsiella pneumoniae, Legionella pneumophila, Moraxella catarrhalis, Pseudomonas aeruginosa, Staphylococcus aureus,* and *Streptococcus pneumoniae.* In the invention, the primer set comprises an oligonucleotide set comprising an oligonucleotide consisting of at least 10 contiguous nucleotides of SEQ ID NO: 1 and an oligonucleotide consisting of at least 10 contiguous nucleotides of SEQ ID NO: 2; an oligonucleotide set comprising an oligonucleotide consisting of at least 10 contiguous nucleotides of SEQ ID NO: 3 and an oligonucleotide consisting of at least 10 contiguous nucleotides of SEQ ID NO: 4; an oligonucleotide set comprising an oligonucleotide consisting of at least 10 contiguous nucleotides of SEQ ID NO: 5 and an oligonucleotide consisting of at least 10 contiguous nucleotides of a sequence selected from the group consisting of SEQ ID NO: 6 and SEQ ID NO:7; and an oligonucleotide set comprising an oligonucleotide consisting of at least 10 contiguous nucleotides of SEQ ID NO: 8 and an oligonucleotide consisting of oat least 10 contiguous nucleotides of SEQ ID NO: 9. In an embodiment, the primer set comprises an oligonucleotide set comprising an oligonucleotide consisting of SEQ ID NO: 1 and an oligonucleotide consisting of SEQ ID NO: 2; an oligonucleotide set comprising an oligonucleotide consisting of SEQ ID NO: 3 and an oligonucleotide consisting of SEQ ID NO: 4; an oligonucleotide set comprising an oligonucleotide consisting of SEQ ID NO: 5 and an oligonucleotide consisting of SEQ ID NO: 6 or SEQ ID NO:7; and an oligonucleotide set comprising an oligonucleotide consisting of SEQ ID NO: 8 and an oligonucleotide consisting of SEQ ID NO: 9.

The primer set of the invention was designed from target regions common to the 23S rRNA genes of the nine respiratory disease-associated bacterial species. The nine respiratory disease-associated bacterial species are *Chlamydophila pneumoniae, Haemophilus influenza, Klebsiella pneumoniae, Legionella pneumophila, Moraxella catarrhalis, Mycoplasma pneumoniae, Pseudomonas aeruginosa, Staphylococcus aureus,* and *Streptococcus pneumoniae.*

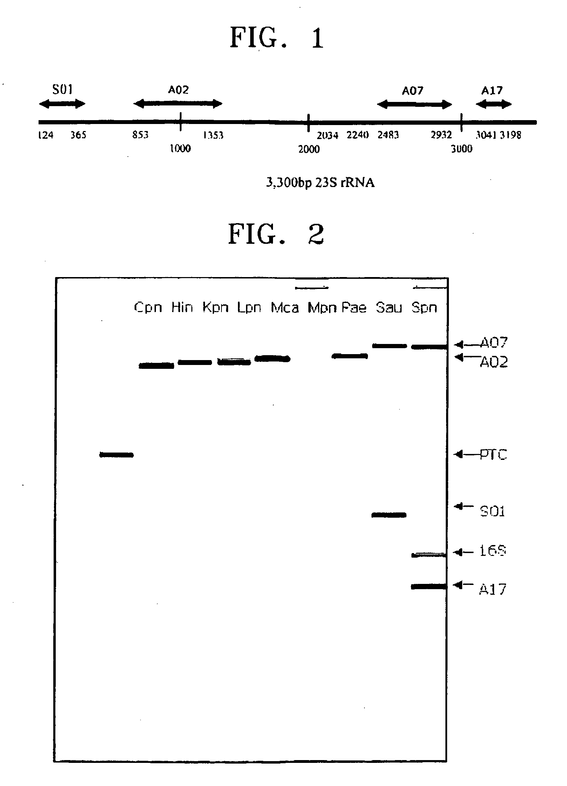

When performing PCR using the primer set of the present invention, a target sequence region sought to be amplified is selected from the nucleotide region corresponding to nucleotide positions 124-365 (represented by S01; SEQ ID NO:A) of the 23S rRNA gene, the nucleotide region corresponding to nucleotide positions 853-1353 (represented by A02; SEQ ID NO:B) of the 23S rRNA gene, the nucleotide region corresponding to nucleotide positions 2483-2932 (represented by A07; SEQ ID NO:C) of the 23S rRNA gene, and the nucleotide region corresponding to nucleotide positions 3041-3198 (represented by A17; SEQ ID NO:D) of the 23S rRNA gene. FIG. 1 is a diagram illustrating the nucleotide positions of the target sequences in the 23S rRNA gene.

The primer set of the invention was designed from the four target sequences common to the 23S rRNA genes of the nine respiratory disease-associated bacterial species. Exemplary examples of the primer set according to the present invention are presented in Table 1 below.

**Table 1: Examples of primer set of the present invention**

| Primer name | SEQ ID NO: | Remark |
|---|---|---|
| S01-F | 1 | Forward primer for S01 amplification |
| S01-R | 2 | Reverse primer for S01 amplification |
| A02-F | 3 | Forward primer for A02 amplification |
| A02-R | 4 | Reverse primer for A02 amplification |
| A07-F | 5 | Forward primer for A07 amplification |
| A07-R1 | 6 | Reverse primer for A07 amplification |
| A07-R2 | 7 | Reverse primer for A07 amplification |
| A17-F | 8 | Forward primer for A17 amplification |
| A17-R | 9 | Reverse primer for A17 amplification |

Also disclosed is an oligonucleotide probe set capable of hybridizing with at least one target sequence selected from the group consisting of nucleotide regions corresponding to positions 124-365, 853-1353, 2483-2932, and 3041-3198 of the 23S rRNA gene of at least one bacterial species selected from the group consisting of *Chlamydophila pneumoniae, Haemophilus influenzae, Mycoplasma pneumoniae, Klebsiella pneumoniae, Legionella pneumophila, Moraxella catarrhalis, Pseudomonas aeruginosa, Staphylococcus aureus,* and *Streptococcus pneumoniae*. The oligonucleotide probe set comprises at least one oligonucleotide probe selected from the group consisting of an oligonucleotide probe comprising an oligonucleotide consisting of at least 10 contiguous nucleotides of SEQ ID NO: 10 or a complement of the oligonucleotide; an oligonucleotide probe comprising an oligonucleotide consisting of at least 10 contiguous nucleotides of a sequence selected from the group consisting of SEQ ID NOS: 11-14 or a complement of the oligonucleotide; an oligonucleotide probe comprising an oligonucleotide consisting of at least 10 contiguous nucleotides of SEQ ID NO: 15 or a complement of the oligonucleotide; an oligonucleotide probe comprising an oligonucleotide consisting of at least 10 contiguous nucleotides of a sequence selected from the group consisting of SEQ ID NOS: 16-18 or a complement of the oligonucleotide; an oligonucleotide probe comprising an oligonucleotide consisting of at least 10 contiguous nucleotides of SEQ ID NO: 19 or a complement of the oligonucleotide; an oligonucleotide probe comprising an oligonucleotide consisting of at least 10 contiguous nucleotides of SEQ ID NO: 20 or a complement of the oligonucleotide; an oligonucleotide probe comprising an oligonucleotide consisting of at least 10 contiguous nucleotides of a sequence selected from the group consisting of SEQ ID NOS: 21-23 or a complement of the oligonucleotide; an oligonucleotide probe comprising an oligonucleotide consisting of at least 10 contiguous nucleotides of a sequence selected from the group consisting of SEQ ID NOS: 24-26 or a complement of the oligonucleotide; an oligonucleotide probe comprising an oligonucleotide consisting of at least 10 contiguous nucleotides of SEQ ID NO: 27 or a complement of the oligonucleotide; an oligonucleotide probe comprising an oligonucleotide consisting of at least 10 contiguous nucleotides of SEQ ID NO: 28 or a complement of the oligonucleotide; an oligonucleotide probe comprising an oligonucleotide consisting of at least 10 contiguous nucleotides of SEQ ID NO: 29 or a complement of the oligonucleotide; an oligonucleotide probe comprising an oligonucleotide consisting of at least 10 contiguous nucleotides of a sequence selected from the group consisting of SEQ ID NOS: 30-31 or a complement of the oligonucleotide; and an oligonucleotide probe comprising an oligonucleotide consisting of at least 10 contiguous nucleotides of a sequence selected from the group consisting of SEQ ID NOS: 32-35 or a complement of the oligonucleotide.

The probe set can be an oligonucleotide probe set capable of hybridizing with the nucleotide region corresponding to the positions 3041-3198 of the 23S rRNA gene of *Chlamydophila pneumoniae,* comprising an oligonucleotide consisting of at least 10 contiguous nucleotides of SEQ ID NO: 10 or a complement of the oligonucleotide.

The probe set can be an oligonucleotide probe set capable of hybridizing with at least one target sequence selected from the group consisting of the nucleotide regions corresponding to the positions 853-1353 and 2483-2932 of the 23S rRNA gene of *Haemophilus influenza.* In one aspect, the oligonucleotide probe set comprises at least one oligonucleotide probe selected from the group consisting of: an oligonucleotide probe comprising an oligonucleotide consisting of at least 10 contiguous nucleotides of a sequence selected from the group consisting of SEQ ID NOS: 11-14 or the complement of the oligonucleotide; and an oligonucleotide probe comprising an oligonucleotide consisting of at least 10 contiguous nucleotides of SEQ ID NO: 15 or the complement of the oligonucleotide. In another aspect, the oligonucleotide probe set comprises an oligonucleotide probe comprising an oligonucleotide consisting of at least 10 contiguous nucleotides of a sequence selected from the group consisting of SEQ ID NOS: 11-14 or the complement of the oligonucleotide; and an oligonucleotide probe comprising an oligonucleotide consisting of at least 10 contiguous nucleotides of SEQ ID NO: 15 or the complement of the oligonucleotide.

The probe set can be an oligonucleotide probe set capable of hybridizing with the nucleotide region corresponding to the positions 2483-2932 of the 23S rRNA gene of *Klebsiella pneumoniae,* comprising an oligonucleotide consisting of at least 10 contiguous nucleotides of a sequence selected from the group consisting of SEQ ID NOS: 16-18 or the complement of the oligonucleotide.

The probe set can be an oligonucleotide probe set capable of hybridizing with at least one target sequence selected from the group consisting of the nucleotide regions corresponding to the positions 853-1353 and 2483-2932 of the 23S rRNA gene of *Legionella pneumophila.* In one aspect, the oligonucleotide probe set comprises at least one oligonucleotide probe selected from the group consisting of an oligonucleotide probe comprising an oligonucleotide consisting of at least 10 contiguous nucleotides of SEQ ID NO: 19 or the complement of the oligonucleotide; and an oligonucleotide probe comprising an oligonucleotide consisting of at least 10 contiguous nucleotides of SEQ ID NO: 20 or the complement of the oligonucleotide. In another aspect, the oligonucleotide probe set comprises an oligonucleotide probe comprising an oligonucleotide consisting of at least 10 contiguous nucleotides of SEQ ID NO: 19 or the complement of the oligonucleotide; and an oligonucleotide probe comprising an oligonucleotide consisting of at least 10 contiguous nucleotides of SEQ ID NO: 20 or the complement of the oligonucleotide.

The probe set can be an oligonucleotide probe set capable of hybridizing with at least one target sequence selected from the group consisting of the nucleotide regions corresponding to the positions 853-1353 and 2483-2932 of the 23S rRNA gene of Moraxella catarrhalis. In an aspect, the oligonucleotide probe set comprises at least one oligonucleotide probe selected from the group consisting of an oligonucleotide probe comprising an oligonucleotide consisting of at least 10 contiguous nucleotides of a sequence selected from the group consisting of SEQ ID NOS: 21-23 or the complement of the oligonucleotide; and an oligonucleotide probe comprising an oligonucleotide consisting of at least 10 contiguous nucleotides of a sequence selected from the group consisting of SEQ ID NOS: 24-26 or the complement of the oligonucleotide. In an aspect, the oligonucleotide probe set comprises an oligonucleotide probe comprising an oligonucleotide consisting of at least 10 contiguous nucleotides of a sequence selected from the group consisting of SEQ ID NOS: 21-23 or the complement of the oligonucleotide; and an oligonucleotide probe comprising an oligonucleotide consisting of at least 10 contiguous nucleotides of a sequence selected from the group consisting of SEQ ID NOS: 24-26 or the complement of the oligonucleotide.

The probe set can be an oligonucleotide probe set capable of hybridizing with the nucleotide region corresponding to the positions 3041-3198 of the 23S rRNA gene of *Mycoplasma pneumoniae,* comprising an oligonucleotide consisting of at least 10 contiguous nucleotides of SEQ ID NO: 27 or the complement of the oligonucleotide.

The probe set can be an oligonucleotide probe set capable of hybridizing with at least one target sequence selected from the group consisting of the nucleotide regions corresponding to the positions 853-1353 and 2483-2932 of the 23S rRNA gene of Pseudomonas aeruginosa. In an aspect, the oligonucleotide probe set comprises an oligonucleotide probe selected from the group consisting of an oligonucleotide probe comprising an oligonucleotide consisting of at least 10 contiguous nucleotides of SEQ ID NO: 28 or the complement of the oligonucleotide; and an oligonucleotide probe comprising an oligonucleotide consisting of at least 10 contiguous nucleotides of SEQ ID NO: 29 or the complement of the oligonucleotide. In an aspect, the oligonucleotide probe set comprises an oligonucleotide probe comprising an oligonucleotide consisting of at least 10 contiguous nucleotides of SEQ ID NO: 28 or the complement of the oligonucleotide; and an oligonucleotide probe comprising an oligonucleotide consisting of at least 10 contiguous nucleotides of SEQ ID NO: 29 or the complement of the oligonucleotide.

The probe set can be an oligonucleotide probe set capable of hybridizing with the nucleotide region corresponding to the positions 124-365 of the 23S rRNA gene of *Staphylococcus aureus,* comprising an oligonucleotide consisting of at least 10 contiguous nucleotides of a sequence selected from the group consisting of SEQ ID NOS: 30 and 31 or the complement of the oligonucleotide.

The probe set can be an oligonucleotide probe set capable of hybridizing with the nucleotide region corresponding to the positions 2483-2932 of the 23S rRNA gene of *Streptococcus pneumoniae,* comprising an oligonucleotide consisting of at least 10 contiguous nucleotides of a sequence selected from the group consisting of SEQ ID NOS: 32-35 or the complement of the oligonucleotide.

The probe set can be an oligonucleotide probe set capable of hybridizing with a target sequence selected from the group consisting of the nucleotide regions corresponding to the positions 124-365, 853-1353, 2483-2932, and 3041-3198 of the 23S rRNA gene of a bacterial species selected from the group consisting of *Chlamydophila pneumoniae, Haemophilus influenza, Klebsiella pneumoniae, Legionella pneumophila, Moraxella catarrhalis, Mycoplasma pneumoniae, Pseudomonas aeruginosa, Staphylococcus aureus,* and *Streptococcus pneumoniae.* In one aspect, the oligonucleotide probe set comprises at least one oligonucleotide probe selected from the group consisting of: an oligonucleotide probe comprising an oligonucleotide consisting of SEQ ID NO: 10, or the complement thereof; an oligonucleotide probe comprising an oligonucleotide consisting of a sequence selected from the group consisting of SEQ ID NOS: 11-14, or the complement thereof; an oligonucleotide probe comprising an oligonucleotide consisting of SEQ ID NO: 15, or the complement thereof; an oligonucleotide probe comprising an oligonucleotide consisting of a sequence selected from the group consisting of SEQ ID NOS: 16-18, or the complement thereof; an oligonucleotide probe comprising an oligonucleotide consisting of SEQ ID NO: 19, or the complement thereof; an oligonucleotide probe comprising an oligonucleotide consisting of SEQ ID NO: 20, or the complement thereof; an oligonucleotide probe comprising an oligonucleotide consisting of a sequence selected from the group consisting of SEQ ID NOS: 21-23, or the complement thereof; an oligonucleotide probe comprising an oligonucleotide consisting of a sequence selected from the group consisting of SEQ ID NOS: 24-26, or the complement thereof; an oligonucleotide probe comprising an oligonucleotide consisting of SEQ ID NO: 27, or the complement thereof; an oligonucleotide probe comprising an oligonucleotide consisting of SEQ ID NO: 28, or the complement thereof; an oligonucleotide probe comprising an oligonucleotide consisting of SEQ ID NO: 29, or the complement thereof; an oligonucleotide probe comprising an oligonucleotide consisting of a sequence selected from the group consisting of SEQ ID NOS: 30 and 31, or the complement thereof; and an oligonucleotide probe comprising an oligonucleotide consisting of a sequence selected from the group consisting of SEQ ID NOS: 32-35, or the complement thereof. In another aspect, the oligonucleotide probe set comprises an oligonucleotide probe comprising an oligonucleotide consisting of SEQ ID NO: 10, or the complement thereof; an oligonucleotide probe comprising an oligonucleotide consisting of a sequence selected from the group consisting of SEQ ID NOS: 11-14, or the complement thereof; an oligonucleotide probe comprising an oligonucleotide consisting of SEQ ID NO: 15, or the complement thereof; an oligonucleotide probe comprising an oligonucleotide consisting of a sequence selected from the group consisting of SEQ ID NOS: 16-18, or the complement thereof; an oligonucleotide probe comprising an oligonucleotide consisting of SEQ ID NO: 19, or the complement thereof; an oligonucleotide probe comprising an oligonucleodde consisting of SEQ ID NO: 20, or the complement thereof; an oligonucleotide probe comprising an oligonucleotide consisting of a sequence selected from the group consisting of SEQ ID NOS: 21-23, or the complement thereof; an oligonucleotide probe comprising an oligonucleotide consisting of a sequence selected from the group consisting of SEQ ID NOS: 24-26, or the complement thereof; an oligonucleotide probe comprising an oligonucleotide consisting of SEQ ID NO: 27, or the complement thereof; an oligonucleotide probe comprising an oligonucleotide consisting of SEQ ID NO: 28, or the complement thereof; an oligonucleotide probe comprising an oligonucleotide consisting of SEQ ID NO: 29, or the complement thereof; an oligonucleotide probe comprising an oligonucleotide consisting of a sequence selected from the group consisting of SEQ ID NOS: 30 and 31, or the complement thereof; and an oligonucleotide probe comprising an oligonucleotide consisting of a sequence selected from the group consisting of SEQ ID NOS: 32-35, or the complement thereof.

The probe set specifically binds with PCR products amplified from target regions S01, A02, A07, and A17 of the 23S rRNA genes of the nine bacterial species obtained by PCR using the primer set of the invention. Thus, the probe set can be used to identify the nine bacterial species. The probe set was designed by comparing the target regions S01, A02, A07, and A17 of the 23S rRNA genes of the nine bacterial species and selecting sequence(s) specifically present in each bacterial species.

As used herein, the term 'probe' refers to a single-stranded nucleic acid sequence that can be base-paired with a complementary single-stranded target sequence to form a double-stranded molecule (hybrid).

As used herein, the term 'hybridization' refers to the hydrogen bonding between two complementary strands of nucleic acid to form a double-stranded molecule (hybrid).

As used herein, 'stringency' is the term used to describe a temperature and a solvent composition during hybridization and the subsequent processes. Under high stringency conditions, only highly complementary nucleic acid hybrids will be formed. Accordingly, the stringency of the hybridization assay conditions determines the amount of complementarity which should exist between two nucleic acid strands (probe and target) to form a hybrid. An example of a high stringency condition is a 0.12M phosphate buffer including equal moles of Na₂HPO₄ and NaH₂PO₄, 1 mM EDTA, and 0.02% sodium dodecylsulfate at 65 °C. Stringency is chosen to maximize the difference in stability between probe-target hybrids and probe-non-target hybrids. The present invention also provides a microarray comprising a substrate, wherein the oligonucleotide probe set according to the invention is immobilized thereon.

As used herein, the term 'microarray' refers to a high-density array of two or more groups of polynucleotides immobilized on a substrate. Here, each of the two or more groups of the polynucleotides is immobilized in a different predetermined region of the substrate. Microarrays are well known in the art. Examples of such microarrays are disclosed in U.S. Pat. No. 5,445,934 and No. 5,744,305 the disclosures of which are incorporated herein in their entireties by reference.

A method of detecting a respiratory disease-associated bacterial species selected from the group consisting of *Chlamydophila pneumoniae, Haemophilus influenza, Klebsiella pneumoniae, Legionella pneumophila, Moraxella catarrhalis, Mycoplasma pneumoniae, Pseudomonas aeruginosa, Staphylococcus aureus,* and *Streptococcus pneumoniae* is also provided. The method comprises contacting a sample to the oligonucleotide probe set according to the invention so that an oligonucleotide probe can hybridize with a target sequence present in the sample; and detecting a degree of hybridization between the oligonucleotide probe and the target sequence.

The sample comprises a PCR product. The PCR product is obtained by multiplex PCR using template nucleic acid obtained from a bacterial species selected from the group consisting of *Chlamydophila pneumoniae, Haemophilus influenza, Klebsiella pneumoniae, Legionella pneumophila, Moraxella catarrhalis, Mycoplasma pneumoniae, Pseudomonas aeruginosa, Staphylococcus aureus,* and *Streptococcus pneumoniae,* and an oligonucleotide primer set according to the invention. The template nucleic acid can be selected from the group consisting of chromosomal DNA, cDNA, and a fragment thereof.

In the method, the target sequence can be labeled with a detectable labeling material. For example, the labeling material can be a fluorescent material, a phosphorescent material, or a radioactive material. Preferably, the labeling material can be the fluorophores, Cy-5 or Cy-3.

In the method, the probe set can be immobilized on a microarray substrate.

In the method, hybridization between the target sequence and the oligonucleotide probe can be performed under a high stringency hybridization condition. For example, the high stringency hybridization condition can be a 0.12M phosphate buffer including equal moles of Na₂HPO₄ and NaH₂PO₄, 1 mM EDTA, and 0.02% sodium dode-cylsulfate and 65 °C.

In the method, 'PCR' refers to a polymerase chain reaction, a method for amplifying a target nucleic acid using a primer pair specifically binding with the target nucleic acid and a DNA polymerase. PCR is well known in the art. PCR can be performed using a commercially available kit. PCR can be classified into single PCR, i.e. the amplification of a single target sequence in a single PCR reaction, and multiplex PCR, i.e. the simultaneous amplification of multiple different target sequences in a single PCR reaction. Multiplex PCR is performed using a plurality of primer pairs, each of which is specific for a particular target sequence.

In the method, detection of the degree of hybridization between the oligonucleotide probe sequence and the target sequence can include labeling a PCR product (target sequence) with a detectable signal-emitting material; hybridizing the labeled PCR product with the oligonucleotide probe set; and detecting a signal generated from the hybridization product. Any detectable signal-emitting labeling material known in the art can be used. For exampled, the detectable signal-emitting material can be a material with a detectable optical property or an electrical signal-emitting material, but the present invention is not limited thereto. The material with a detectable optical property may be a fluorescent material or a phosphorescent material. The fluorescent material may be fluorescein, Cy-5, or Cy-3. The PCR product can be labeled with the detectable signal-emitting material before or after hybridization with the probe.

Detection of the hybrids does not require that the PCR product be labeled. For the instance in which the PCR product is unlabeled, hybridization between the PCR product and the oligonucleotide probe set can be detected by a difference in an electrical signal before and after hybridization. An example of a suitable electrical signal is capacitance, but the present invention is not limited thereto.

### Advantageous Effects

A nucleic acid primer set of the invention can amplify target sequence(s) derived from nine respiratory disease-associated bacterial species.

A probe set as disclosed here with is specific to a target sequence of a PCR product amplified using the primer set of the invention, and thus, can be used for detection of at least one of the nine respiratory disease-associated bacterial species.

A microarray as disclosed here with can be used for detection of at least one of the nine respiratory disease-associated bacterial species.

A detection method of the invention ensures high-efficiency and high-specificity of detection of the nine respiratory disease-associated bacterial species.

### Description of Drawings

The above and other features and advantages of the present invention will become more apparent by describing in detail exemplary embodiments thereof with reference to the attached drawings in which:
FIG. 1 is a diagram illustrating the positions of target sequences in the 23S rRNA gene; and
FIG. 2 shows electrophoretic results of polymerase chain reaction ('PCR') products obtained by PCR using four primer sets of the present invention.

### Best Mode

Hereinafter, the present invention will be described more specifically with reference to the following working examples. The following working examples are for illustrative purposes and are not intended to limit the scope of the invention.

### Examples

### Example 1: Selection of primers for amplifying target sequences commonly found in nine respiratory disease-associated bacterial species

In Example 1, target sequences common to the 23S rRNA genes of nine bacterial species, i.e., *Chlamydophila pneumoniae, Haemophilus influenzae, Mycoplasma pneumoniae, Klebsiella pneumoniae, Legionella pneumophila, Moraxella catarrhalis, Pseudomonos aeruginosa, Staphylococcus aureus,* and *Streptococcus pneumoniae* were selected, and primer sets capable of amplifying the target sequences were designed.

First, sequences of respiratory disease-associated bacteria were acquired by sequencing 23s rRNAs obtained from clinical isolates. The bacterial species, number of strains of each bacterial species, and the SEQ ID Nos. for the sequences are presented in Table 2 below.

**Table 2**

| Bacterial species | The number of strains | Examples of SEQ ID Nos: |
|---|---|---|
| *Chlamydophila pneumoniae* | 1 | 36, 37 |
| *Haemophilus influenzae,* | 50 | 38 to 45 |
| *Mycoplasma pneumoniae* | 1 | 46 |
| *Klebsiella pneumoniae* | 61 | 47 to 61 |
| *Legionella pneumophila* | 22 | 62 to 64 |
| *Moraxella catarrhalis* | 10 | 65 to 67 |
| *Pseudomonas aeruginosa* | 72 | 68 to 76 |
| *Staphylococcus aureus* | 79 | 77 to 83 |
| *Streptococcus pneumoniae* | 105 | 84 to 94 |
| Total | 401 | - |

Conserved regions of the 23S rRNA genes of the bacterial species were selected using the program DNASTAR.

An oligonucleotide primer set was designed for each of the four target sequences using DNASTAR: an oligonucleotide primer set consisting of SEQ ID NOS: 1 and 2, an oligonucleotide primer set consisting of SEQ ID NOS: 3 and 4, an oligonucleotide primer set consisting of SEQ ID NOS: 5 and 6, and an oligonucleotide primer set consisting of SEQ ID NOS: 8 and 9. Each of these oligonucleotide primer sets can amplify a target region in the 23S rRNA genes of one or more of the nine bacterial species.

### Example 2: Amplification of 23S rRNA genes of nine respiratory disease-associated bacterial species using primer sets of the present invention

The 23S rRNA genes of the nine respiratory disease-associated bacterial species were amplified using the four primer sets designed in Example 1.

First, amplification using polymerase chain reaction ('PCR') was performed using only a single set of the primers designed in Example 1 ('single PCR') to determine if each target sequence was specifically amplified. In the various single PCR, genomic DNA from 47 control bacterial species and 9 test bacterial species was used as template DNA. The control and test bacterial species used are listed in Table 4 below. Each single PCR was performed using 20 □ of a PCR solution containing 2 □ of a genomic DNA (extracted using a G-SPIN genomic DNA extraction kit; iNtRON ), 1.5 mM MgCl₂, 250 mM of each dNTP, 10 mM tris-HCl (pH 9.0), 1 unit of Taq polymerase, and about 2 pmol of each primer, for 29 minutes and 5 seconds, as follows: 25 cycles of denaturation at 95 °C for 10 seconds, annealing at 60 °C for 10 seconds, and extension at 60 °C for 13 seconds.

The PCR products were identified by electrophoresis. The results for the 9 test species are presented in Table 3 below. Expected PCR products are amplified in a control PCR using gDNA from one of 47 control species (data not shown).

Next, multiplex PCR was performed using the four primer sets designed in Example 1 simultaneously to amplify a genomic DNA from one of the nine respiratory disease-associated bacterial species. The products from the multiplex PCR were identified by electrophoresis on an agarose gel.

### (1) Preparation of bacterial cultures

Cultured isolates of the nine respiratory disease-associated bacterial species from the Asian-Pacific Research Foundation for Infectious Diseases (ARFID) were used.

### (2) Multiplex PCR

The PCR mix for the multiplex PCR was made up to a total volume of 50 D , containing 10.5 □ distilled water, 7.5 □ 10x buffer (750mM Tris-HCl (pH9), 150mM Ammonium Sulfate(NH₄)₂SO₄ 25mM MgCl₂, 1mg/ml BSA), 1 □ 200 µ M dNTP (each), 20 □ 400 nM end-labeled primer (each, Bioneer, Korea), 5 □ extracted genomic DNA, and 1 □ Taq polymerase (5 units). Human MODY exon 9 ('e9') DNA was used as a positive control.

The multiplex PCR was performed as follows: initial denaturation at 95 °C for one minute; 25 cycles of denaturation at 95 °C for 5 seconds, annealing at 62 °C for 13 seconds, and extension at 72 °C for 15 seconds; and extension at 72 °C for one minute.

The single PCR results and the multiplex PCR results for the 9 test bacterial species are presented in Table 3 below. Abbreviations used for the nine bacterial species in Table 3 are defined in Table 4 below. In Table 3,'PTC' is a positive control, and a primer set targeting 16S rRNA (16S-F and 16S-R) was also included as a control.

**Table 3**

| | Primer ID | Cpn | Hin | Kpn | Lpn | Mca | Mpn | Pae | Sau | Spn |
|---|---|---|---|---|---|---|---|---|---|---|
| The number of mismatched bases between primer and target sequence | S01-F | 8 | 8 | 8 | 9 | 8 | 8 | 9 | 0 | 8 |
| | S01-R | 9 | 8 | 10 | 10 | 10 | 8 | 11 | 0 | 10 |
| | A02-F | 5 | 0 | 0 | 0 | 0 | 7 | 0 | 5 | 5 |
| | A02-R | 3 | 0 | 0 | 0 | 1 | 6 | 0 | 1 | 1 |
| | A07-F | 0 | 0 | 0 | 0 | 0 | 3 | 0 | 0 | 0 |
| | A07-R | 5 | 0 | 0 | 0 | 0 | 2 | 0 | 0 | 0 |
| | A17-F | 0 | 0 | 0 | 0 | 0 | 0 | 0 | 0 | 1 |
| | A17-R | 0 | 0 | 0 | 0 | 0 | 0 | 0 | 2 | 0 |
| | 16S-F | 0 | 0 | 0 | 0 | 0 | 0 | 0 | 0 | 0 |
| | 16S-R | 0 | 0 | 0 | 0 | 0 | 0 | 0 | 0 | 0 |
| Single PCR results | S01 | X | X | X | X | X | X | X | O | X |
| | A02 | O | O | O | O | O | X | O | X | X |
| | A07 | O | O | O | O | O | X | O | O | O |
| | A17 | O | O | O | O | O | O | O | O | O |
| | 16S | O | O | O | O | O | O | O | O | O |
| Multiplex PCR results | S01 | X | X | X | X | X | X | X | O | X |
| | A02 | X | O | O | O | O | X | O | X | X |
| | A07 | X | O | O | O | O | X | O | O | O |
| | A17 | O | O | O | O | O | O | O | X | O |
| | 16S | O | O | O | O | O | O | O | O | O |
| | PTC | O | O | O | O | O | O | O | O | O |

| | | | | | | | | | | |
|---|---|---|---|---|---|---|---|---|---|---|
| O: band detection; X: no band detection | | | | | | | | | | |

The single PCR results in Table 3 show that not all four target sequences were present in each of the test bacterial species. Further, the multiplex PCR results in Table 3 show that the target sequence of each of the four primer sets could be identified by multiplex PCR using the four primer sets, although not all four target sequences were present in each of the test bacterial species. This shows that, in general, the target sequence specificity of any one of the four primer sets was not affected by the presence of the other primer sets during the multiplex PCR. However, amplification of *Chlamydophila pneumoniae* using the primer sets A02-F and R and A07-F and R, *Staphylococcus aureus* using the primer set A17-F and R, and *Streptococcus pneumoniae* using the primer set A02-F and R, produced weak gel bands in the single PCR, that were not seen in the multiplex PCR.

FIG. 2 shows the result of electrophoretic analysis of PCR products obtained by multiplex PCR using the four primer sets of the present invention simultaneously with each of the nine test bacterial species.

### Example 3: Detection of nine respiratory disease-associated bacterial species using microarrays

In Example 3, multiplex PCR products obtained as in Example 2 were allowed to hybridize with probes immobilized on microarrays and the degree of probe-target hybridization was determined to detect the presence of any PCR product amplified from a specific bacterial species.

Sample preparation and multiplex PCR were performed in the same manner as in Example 2 except that 5'-ends of all forward and reverse primers were labeled with Cy-3. PCR products were detected using microarrays as follows.

401 strains of the nine bacterial species were used as a test group and 226 strains of 47 species of 10 other bacterial genera were used as a control group.

The bacterial strains used as the test group and the control group are summarized in Table 4 below.

**Table 4**

| | Genus | Species | Abbreviation | The number of strains |
|---|---|---|---|---|
| Test group | *Streptococcus* | *Streptococcus pneumoniae* | Spn | 105 |
| | *Staphylococcus* | *Staphylococcus aureus* | Sau | 79 |
| | *Klebsiella* | *Klebsiella pneumoniae* | Kpn | 61 |
| | *Pseudomonas* | *Pseudomonas aeruginosa* | Pae | 72 |
| | *Haemophilus* | *Haemophilus influenza* | Hin | 50 |
| | *Legionella* | *Legionella pneumophila* | Lpn | 22 |
| | *Moraxella* | *Moraxella catarrhalis* | Mca | 10 |
| | *Mycoplasma* | *Mycoplasma pneumoniae* | Mpn | 1 |
| | *Chlamydophila* | *Chlamydophila pneumoniae* | Cpn | 1 |
| Total | 9 | 9 | | 401 |
| Control group | *Acinetobacter* | *Acinetobacter baumannii* | Aba | 9 |
| | | *Acinetobacter calcoaceticus* | Aca | 1 |
| | | *Acinetobacter Iwoffii* | Alw | 1 |
| | *Bacillus* | *Bacillus subtilis* | Bsu | 1 |
| | *Bordetella* | *Bordetella ansorpii* | Ban | 1 |
| | | *Bordetella avium* | Bav | 1 |
| | | *Bordetella bronchiseptica* | Bbr | 1 |
| | *Citrobacter* | *Citrobacter freundii* | Cfr | 6 |
| | *Enterobacter* | *Enterobacter aerogenes* | Eae | 9 |
| | | *Enterobacter cloacae* | Ecl | 6 |
| | *Enterococcus* | *Enterococcus faecalis* | Efs | 11 |
| | | *Enterococcus faecium* | Efm | 11 |
| | *Escherichia* | *Escherichia coli* | Eco | 20 |
| | *Gemella* | *Gemella melitensis* | Gme | 1 |
| | *Haemophilus* | *Haemophilus aprophilus* | Hap | 1 |
| | *Klebsiella* | *Klebsiella oxytoca* | Kox | 12 |
| | *Moraxella* | *Moraxella nonliquefaciens* | Mon | |
| Control group | *Morganella* | *Morganella morganii* | Mmo | 7 |
| | *Proteus* | *Proteus mirabi*/*is* | Pmi | 8 |
| | | *Proteus vulgaris* | Pvu | 5 |
| | *Pseudomonas* | *Pseudomonas fluorescens* | Pfl | 9 |
| | | *Pseudomonas putida* | Ppu | 1 |
| | | *Pseudomonas stutzeri* | Pst | 1 |
| | *Salmonella* | *Salmonella typhi* | Sty | 10 |
| | | *Salmonella murium* | Stym | 7 |
| | *Shigella* | *Shigella boydii* | Sbo | 2 |
| | | *Shigella flexneri* | Sfl | 9 |
| | | *Shigella sonnei* | Sso | 6 |
| | *Staphylococcus* | *Staphylococcus conii* | Sco | 2 |
| | | *Staphylococcus epidermis* | Sep | 7 |
| | | *Staphylococcus galinarium* | Sga | 1 |
| | | *Staphylococcus haemolyticus* | Sha | 1 |
| | | *Staphylococcus hominis* | Sho | 3 |
| | | *Staphylococcus intermedius* | Sin | 2 |
| | | *Staphylococcus lentus* | Sle | 3 |
| | | *Staphylococcus xylosus* | Sxy | 1 |
| | | Coagulase negative *staphylococci* | CNS | 20 |
| | *Streptococcus* | *Streptococcus agalactiae* (Group B) | Sag | 1 |
| | | *Streptococcus disqalactiae* | Sdy | 1 |
| | | *Streptococcus gordonii* | Sgo | 1 |
| | | *Streptococcus intermedius*/*milleri* | st. in | 1 |
| | | *Streptococcus mitis* | Smi | 6 |
| | | *Streptococcus oralis* | Sor | 11 |
| | | *Streptococcus pyogenes* | Spy | 1 |
| | | *Streptococcus salivarius subsp. thermophilus* | Ssa | 1 |
| | | *Streptococcus sanguinis* | Ssan | 1 |
| | | *Streptococcus suis* | Ssu | 1 |
| Total | 18 | 47 | | 223 |

### (1) Probe design

**Table 5.**

| Probe name | Target bacteria species | SEQ ID NO | Target region of 23S rRNA |
|---|---|---|---|
| A17-Chl.pneumo-re | *Chlamydophila pneumoniae* | 10 | 3041-3198 (A017) |
| A02-Hae.influe | *Haemophilus influenzae* | 11 | 853-1353 (A02) |
| A02-Hae.intlue1-re | *Haemophilus influenzae* | 12 | 853-1353 (A02) |
| A02-H.inf2-I | *Haemophilus influenzae* | 13 | 853-1353 (A02) |
| A02-H.inf2-A | *Haemophilus influenzae* | 14 | 853-1353 (A02) |
| A07-Hae.influe | *Haemophilus influenzae* | 15 | 2483-2932 (A07) |
| A07-Kle.pneumo1-re | *Klebsielta pneumoniae* | 16 | 2483-2932 (A07) |
| A07-Kle.pneumo3-re | *Klebsiella pneumoniae* | 17 | 2483-2932 (A07) |
| A07-Kle.pneumo-re | *Klebsiella pneumoniae* | 18 | 2483-2932 (A07) |
| A02-Leg.pneumo | *Legionella pneumophila* | 19 | 853-1353 (A02) |
| A07-Leg.pneumo2 | *Legionella pneumophila* | 20 | 2483-2932 (A07) |
| A02-Mor.catarr1-re | *Moraxella catarrhalis* | 21 | 853-1353 (A02) |
| A02-Mor.cat2-C | *Moraxella catarrhalis* | 22 | 853-1353 (A02) |
| A02-Mor.cat2-N | *Moraxella catarrhalis* | 23 | 853-1353 (A02) |
| A07-Mor.catarr-re | *Moraxella catarrhalis* | 24 | 2483-2932 (A07) |
| A07-Mor.cat2-C | *Moraxella calarrhalis* | 25 | 2483-2932 (A07) |
| A07-Mor.cat2-N | *Moraxella catarrhalis* | 26 | 2483-2932 (A07) |
| A17-Myc.pneumo | *Mycoplasma pneumoniae* | 27 | 3041-3198 (A17) |
| A02-Pse.aerugi | *Pseudomonas aeruginosa* | 28 | 853-1353 (A02) |
| A07-Pse.aerugi-re | *Pseudomonas aeruginosa* | 29 | 2483-2932 (A07) |
| S01_Sta.aureu1 | *Staphylococcus aureus* | 30 | 124-365 (S01) |
| S01_Sta.aureu2 | *Staphylococcus aureus* | 31 | 124-365 (S01) |
| A07-Str.pneumo2-re | *Streptococcus pneumoniae* | 32 | 2483-2932(A07) |
| A07-Str.pneumo3 | *Streptococcus pneumoniae* | 33 | 2483-2932(A07) |
| A07-Str.pneumo3-O-re | *Streptococcus pneumoniae* | 34 | 2483-2932(A07) |
| A07-Str.pneumo3-P-re | *Streptococcus pneumoniae* | 35 | 2483-2932(A07) |

Probes were selected from the PCR-amplified regions of the bacterial genomes using the program, DNASTAR. Probe information is summarized in Table 5.

### (2) Manufacturing of probe-immobilized microarrays

Wafers were spin-coated with a solution of GAPTES (γ-aminopropyltriethoxy silane) (20%(v/v)) or GAPDES (γ -aminopropyldiethoxysilane) (20%(v/v)) in ethanol. The spin coating was performed using a spin coater (Model CEE 70, CEE) as follows: initial coating at a rate of 500 rpm/10 sec and main coating at a rate of 2000 rpm/10 sec. After the spin coating was completed, the wafers were placed in a Teflon wafer carrier and cured at 120 °C for 40 minutes. The cured wafers were immersed in water for 10 minutes, ultrasonically washed for 15 minutes, immersed in water for 10 minutes, and dried. The drying was performed using a spin-drier. All the experiments were conducted in a clean room class 1000 where most dust particles had been sufficiently removed.

A probe set including probes consisting of sequences as set forth in SEQ ID NOS: 10-35 was immobilized on the amino-activated wafers using a spotting method to thereby obtain microarrays.

### (3) Detection

The PCR products were added directly to the microarrays. The microarrays were incubated at 42 °C for one hour so that probe-target hybridization occurred. The microarray was then washed with a washing buffer. Fluorescence intensity from hybridized PCR products was measured using a GenePix Scanner (Molecular Device, U.S.A.). The measured fluorescence intensity from a hybrid formed with a particular probe on the microarray is summarized in Table 6 below.

**Table 6.**

| Num bers | Probe ID | Cpn | Hin | Kpn | Lpn | Mca | Mpn | Pae | Sau | Spn |
|---|---|---|---|---|---|---|---|---|---|---|
| 1 | pcr_probe4 | 6543 9 | 6535 1 | 6543 1 | 6533 4 | 6544 5 | 6544 5 | 6543 8 | 5416 5 | 6294 7 |
| 2 | spike_probe1 | 5968 | 5331 | 1014 1 | 4961 | 5562 | 5202 | 6666 | 3798 | 4446 |
| 3 | (-) | 109 | 84 | 89 | 85 | 99 | 106 | 102 | 90 | 96 |
| 4 | A02-IC1 | 2551 | 6528 1 | 2319 4 | 6532 7 | 6141 4 | 2376 | 6543 4 | 1081 3 | 1595 |
| 5 | A07-IC2 | 8275 | 5114 8 | 4192 6 | 5662 0 | 2393 8 | 1079 3 | 4363 1 | 3600 | 5321 4 |
| 6 | A07-IC3 | 2157 6 | 6358 1 | 6539 7 | 4921 2 | 3122 3 | 1746 3 | 6260 0 | 3316 | 5485 |
| 7 | A17-IC1 | 4361 | 3048 | 8117 | 1025 | 651 | 3339 | 3996 | 1232 | 5285 0 |
| 8 | A17-IC2 | 1722 7 | 2750 4 | 1866 4 | 3561 4 | 1988 1 | 6017 | 2933 7 | 1602 | 9037 |
| 9 | A17-IC3 | 4293 4 | 311 | 811 | 352 | 216 | 381 | 311 | 259 | 228 |
| 10 | A17-Chl.pneum o24543 -re | 2454 3 | 583 | 766 | 588 | 1911 | 764 | 580 | 352 | 1621 |
| 11 | A02-Hae.influe | 3036 | 6535 0 | 482 | 658 | 376 | 2650 | 694 | 818 | 1967 |
| 12 | A07-Hae.influe | 199 | 6535 3 | 1064 | 941 | 2400 9 | 172 | 3913 | 845 | 632 |
| 13 | A02-Hae.influe 1-r | 1923 | 6533 9 | 3128 | 586 | 5365 | 1733 | 751 | 639 | 1224 |
| 14 | A07-Kle.pneum o1-r | 143 | 1017 | 3439 9 | 5176 | 151 | 208 | 1072 | 4581 | 1115 4 |
| 15 | A07-Kle.pneum o3-r | 132 | 163 | 4865 1 | 137 | 133 | 146 | 157 | 139 | 158 |
| 16 | A07-Kle.pneum o-re | 155 | 273 | 5237 5 | 139 | 659 | 154 | 156 | 148 | 143 |
| 17 | A02-Leg.pneum o | 386 | 1020 5 | 560 | 6529 0 | 2827 8 | 242 | 2853 | 159 | 331 |
| 18 | A07-Leg.pneum o2 | 134 | 129 | 106 | 5130 6 | 123 | 140 | 140 | 111 | 136 |
| 19 | A02-Mor.catarr 1-r | 283 | 190 | 192 | 159 | 6543 5 | 209 | 154 | 139 | 159 |
| 20 | A07-Mor.catarr -re | 156 | 177 | 154 | 776 | 6543 5 | 168 | 158 | 137 | 176 |
| 21 | A17-Myc.pneu mo | 573 | 858 | 767 | 550 | 2483 | 6544 1 | 489 | 299 | 365 |
| 22 | A02-Pse.aerugi | 144 | 138 | 109 | 126 | 121 | 151 | 4638 1 | 124 | 157 |
| 23 | A07-Pse.aerugire | 181 | 158 | 275 | 4019 4 | 5333 | 176 | 2899 3 | 147 | 171 |
| 24 | S01-IC1 | 596 | 404 | 659 | 751 | 325 | 472 | 478 | 5778 3 | 519 |
| 25 | S01_Sta.aureu1 | 953 | 595 | 797 | 515 | 404 | 829 | 728 | 6543 5 | 676 |
| 26 | S01_Sta.aureu2 | 943 | 591 | 845 | 499 | 447 | 877 | 702 | 6541 6 | 638 |
| 27 | A07-Str.pneum o | 1086 4 | 1100 6 | 1484 8 | 9588 | 6890 | 9306 | 9166 | 5435 | 6542 6 |
| 28 | A07-Str.pneum o2-r | 238 | 121 | 155 | 99 | 117 | 171 | 154 | 3056 | 6542 7 |
| 29 | A07-Str.pneum o3 | 282 | 187 | 265 | 160 | 161 | 198 | 204 | 200 | 6454 1 |
| 30 | A07-Str.pneum o3-O | 183 | 138 | 150 | 84 | 122 | 154 | 140 | 130 | 5012 8 |
| 31 | A07-Str.pneum o201 3-P | 201 | 117 | 132 | 338 | 118 | 169 | 136 | 116 | 6349 2 |

As shown in Table 6, the nine bacterial species were detected with high sensitivity using the probes designed as disclosed here with. Probes corresponding to numbers 1-9, and 24 given in Table 6 represent various control probes. A probe corresponding number 1 is a positive PCR probe indicating that a PCR is successfully conducted. A probe corresponding number 2 is a positive microarray probe indicating that a hybridization in successfully conducted. A probe corresponding number 3 is a negative probe. Probes corresponding numbers 4-10 and 31 are positive probes indicating that each target region corresponding to each region is successfully amplified.

The results of Table 6 also reveal that the probes designed as disclosed here with had 99.8 or higher % specificity and 100% sensitivity for the nine target bacterial species among 640 strains of 56 bacterial species as shown in Table 7 to 16. The experimental data obtained by conducting a hybridization on a microarray were confirmed by culturing experiment.

**Table 7.**

| S.pneumoniae | | Culture | |
|---|---|---|---|
| | | + | - |
| Microarray | + | 104 | 0 |
| | - | 0 | 518 |
| Sensitivity | Specificity | 100% | 100% |

**Table 8.**

| H.influenzae | | Culture | |
|---|---|---|---|
| | | + | - |
| Microarray | + | 48 | 0 |
| | - | 0 | 574 |
| Sensitivity | Specificity | 100% | 100% |

**Table 9.**

| S.aureus | | Culture | |
|---|---|---|---|
| | | + | - |
| Microarray | + | 78 | 0 |
| | - | 0 | 544 |
| Sensitivity | Specificity | 100% | 100% |

**Table 10.**

| P.aeruginosa | | Culture | |
|---|---|---|---|
| | | + | - |
| Microarray | + | 72 | 0 |
| | - | 0 | 550 |
| Sensitivity | Specificity | 100% | 100% |

**Table 11.**

| K. pneumoniae | | Culture | |
|---|---|---|---|
| | | + | - |
| Microarray | + | 61 | 1 |
| | - | 0 | 561 |
| Sensitivity | Specificity | 100% | 99.8% |

**Table 12.**

| M.catarrhalis | | Culture | |
|---|---|---|---|
| | | + | - |
| Microarray | + | 10 | 1 |
| | - | 0 | 612 |
| Sensitivity | Specificity | 100% | 99.8% |

**Table 13.**

| C.pneumoniae | | Culture | |
|---|---|---|---|
| | | + | - |
| Microarray | + | 1 | 0 |
| | - | 0 | 621 |
| Sensitivity | Specificity | 100% | 100% |

**Table 14.**

| L.pneumophila | | Culture | |
|---|---|---|---|
| | | + | - |
| Microarray | + | 23 | 0 |
| | - | 0 | 599 |
| Sensitivity | Specificity | 100% | 100% |

**Table 15.**

| Total (9 bacterial species) | | Culture | |
|---|---|---|---|
| | | + | - |
| Microarray | + | 402 | 2 |
| | - | 0 | 218 |
| Sensitivity | Specificity | 100% | 99.1% |

**Table 16.**

| M. pneumoniae | | Culture | |
|---|---|---|---|
| | | + | - |
| Microarray | + | 5 | 0 |
| | - | 0 | 617 |
| Sensitivity | Specificity | 100% | 100% |

The terminology used herein is for the purpose of describing particular embodiments only and is not intended to be limiting of the invention. The terms 'a' and 'an' do not denote a limitation of quantity, but rather denote the presence of at least one of the referenced item. The term 'or' means 'and/or'. The terms 'comprising', 'having', 'including', and 'containing' are to be construed as open-ended terms (i.e., meaning 'including, but not limited to').

Recitation of ranges of values are merely intended to serve as a shorthand method of referring individually to each separate value failing within the range, unless otherwise indicated herein, and each separate value is incorporated into the specification as if it were individually recited herein. The endpoints of all ranges are included within the range and independently combinable.

All methods described herein can be performed in a suitable order unless otherwise indicated herein or otherwise clearly contradicted by context. The use of any and all examples, or exemplary language (e.g., 'such as'), is intended merely to better illustrate the invention and does not pose a limitation on the scope of the invention unless otherwise claimed. No language in the specification should be construed as indicating any non-claimed element as essential to the practice of the invention as used herein. Unless defined otherwise, technical and scientific terms used herein have the same meaning as is commonly understood by one of skill in the art to which this invention belongs.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above- described elements in all possible variations thereof is encompassed by the invention unless otherwise indicated herein or otherwise clearly contradicted by context. While the present invention has been particularly shown and described with reference to exemplary embodiments thereof, it will be understood by those of ordinary skill in the art that various changes in form and details may be made therein without departing from the scope of the present invention as defined by the following claims.
## Claims

1. An oligonucleotide primer set for amplifying at least one target sequence of a 23 S rRNA gene of at least one bacterial species selected from the group consisting of Chlamydophila pneumoniae, Haemophilus influenzae, Mycoplasma pneumoniae, Klebsiella pneumoniae, Legionella pneumophila, Moraxella catarrhalis, Pseudomonas aeruginosa, Staphylococcus aureus, and Streptococcus pneumoniae, comprising: the oligonucleotide set comprising the oligonucleotide consisting of at least 10 contiguous nucleotides of SEQ ID NO: 1 and the oligonucleotide consisting of at least 10 contiguous nucleotides of SEQ ID NO: 2; the oligonucleotide set comprising the oligonucleotide consisting of at least 10 contiguous nucleotides of SEQ ID NO: 3 and the oligonucleotide consisting of at least 10 contiguous nucleotides of SEQ ID NO: 4; the oligonucleotide set comprising the oligonucleotide consisting of at least 10 contiguous nucleotides of SEQ ID NO: 5 and the oligonucleotide consisting of at least 10 contiguous nucleotides of a sequence selected from the group consisting of SEQ ID NOS: 6 and 7; and the oligonucleotide set comprising the oligonucleotide consisting of at least 10 contiguous nucleotides of SEQ ID NO: 8 and the oligonucleotide consisting of at least 10 contiguous nucleotides of SEQ ID NO: 9.

2. The oligonucleotide primer set of claim 1, which comprises an oligonucleotide set comprising an oligonucleotide consisting of SEQ ID NO: 1 and an oligonucleotide consisting of SEQ ID NO: 2; an oligonucleotide set comprising an oligonucleotide consisting of SEQ ID NO: 3 and an oligonucleotide consisting of SEQ ID NO: 4; an oligonucleotide set comprising an oligonucleotide consisting of SEQ ID NO: 5 and an oligonucleotide consisting of SEQ ID NO: 6 or SEQ ID NO:7; and an oligonucleotide set comprising an oligonucleotide consisting of SEQ ID NO: 8 and an oligonucleotide consisting of SEQ ID NO: 9.

3. A method of detecting a bacterial species selected from the group consisting of Chlamydophila pneumoniae, Haemophilus influenza, Klebsiella pneumoniae, Legionella pneumophila, Moraxella catarrhalis, Mycoplasma pneumoniae, Pseudomonas aeruginosa, Staphylococcus aureus, and Streptococcus pneumoniae, the method comprising:
a) performing a multiplex PCR using template nucleic acid obtained from a bacterial species selected from the group consisting of Chlamydophila pneumoniae, Haemophilus influenza, Klebsiella pneumoniae, Legionella pneumophila, Moraxella catarrhalis, Mycoplasma pneumoniae, Pseudomonas aeruginosa, Staphylococcus aureus, and Streptococcus pneumoniae, and an oligonucleotide primer set comprising the oligonucleotide set of claim 1 or 2;
b) obtaining a sample comprising the PCR product of step a);
c) contacting the sample to an oligonucleotide probe set so that an oligonucleotide probe hybridizes with a target sequence present in the sample; and
d) detecting a degree of hybridization between the oligonucleotide probe and the target sequence, wherein the oligonucleotide probe set comprises at least one oligonucleotide probe selected from the group consisting of:
an oligonucleotide probe comprising an oligonucleotide consisting of at least 10 contiguous nucleotides of SEQ ID NO: 10 or a complement of the oligonucleotide;
an oligonucleotide probe comprising an oligonucleotide consisting of at least 10 contiguous nucleotides of a sequence selected from the group consisting of SEQ ID NOS: 11-14 or a complement of the oligonucleotide;
an oligonucleotide probe comprising an oligonucleotide consisting of at least 10 contiguous nucleotides of SEQ ID NO: 15 or a complement of the oligonucleotide;
an oligonucleotide probe comprising an oligonucleotide consisting of at least 10 contiguous nucleotides of a sequence selected from the group consisting of SEQ ID NOS: 16-18 or a complement of the oligonucleotide;
an oligonucleotide probe comprising an oligonucleotide consisting of at least 10 contiguous nucleotides of SEQ ID NO: 19 or a complement of the oligonucleotide;
an oligonucleotide probe comprising an oligonucleotide consisting of at least 10 contiguous nucleotides of SEQ ID NO: 20 or a complement of the oligonucleotide;
an oligonucleotide probe comprising an oligonucleotide consisting of at least 10 contiguous nucleotides of a sequence selected from the group consisting of SEQ ID NOS: 21-23 or a complement of the oligonucleotide;
an oligonucleotide probe comprising an oligonucleotide consisting of at least 10 contiguous nucleotides of a sequence selected from the group consisting of SEQ ID NOS: 24-26 or a complement of the oligonucleotide;
an oligonucleotide probe comprising an oligonucleotide consisting of at least 10 contiguous nucleotides of SEQ ID NO: 27 or a complement of the oligonucleotide;
an oligonucleotide probe comprising an oligonucleotide consisting of at least 10 contiguous nucleotides of SEQ ID NO: 28 or a complement of the oligonucleotide;
an oligonucleotide probe comprising an oligonucleotide consisting of at least 10 contiguous nucleotides of SEQ ID NO: 29 or a complement of the oligonucleotide; an oligonucleotide probe comprising an oligonucleotide consisting of at least 10 contiguous nucleotides of a sequence selected from the group consisting of SEQ ID NOS: 30 and 31 or a complement of the oligonucleotide;
and an oligonucleotide probe comprising an oligonucleotide consisting of at least 10 contiguous nucleotides of a sequence selected from the group consisting of SEQ ID NOS: 32-35 or a complement of the oligonucleotide.

4. The method of claim 3, wherein the oligonucleotide probe set comprises an oligonucleotide probe selected from the group consisting of:
the oligonucleotide probe comprising the oligonucleotide consisting of SEQ ID NO: 10, or the complement thereof;
the oligonucleotide probe comprising the oligonucleotide consisting of a sequence selected from the group consisting of SEQ ID NOS: 11-14, or the complement thereof;
the oligonucleotide probe comprising the oligonucleotide consisting of SEQ ID NO: 15, or the complement thereof; the oligonucleotide probe comprising the oligonucleotide consisting of a sequence selected from the group consisting of SEQ ID NOS: 16-18, or the complement thereof;
the oligonucleotide probe comprising the oligonucleotide consisting of SEQ ID NO: 19, or the complement thereof;
the oligonucleotide probe comprising the oligonucleotide consisting of SEQ ID NO: 20, or the complement thereof;
the oligonucleotide probe comprising the oligonucleotide consisting of a sequence selected from the group consisting of SEQ ID NOS: 21-23, or the complement thereof;
the oligonucleotide probe comprising the oligonucleotide consisting of a sequence selected from the group consisting of SEQ ID NOS: 24-26, or the complement thereof;
the oligonucleotide probe comprising the oligonucleotide consisting of SEQ ID NO: 27, or the complement thereof;
the oligonucleotide probe comprising the oligonucleotide consisting of SEQ ID NO: 28, or the complement thereof;
the oligonucleotide probe comprising the oligonucleotide consisting of SEQ ID NO: 29, or the complement thereof;
the oligonucleotide probe comprising the oligonucleotide consisting of a sequence selected from the group consisting of SEQ ID NOS: 30 and 31, or the complement thereof; and
the oligonucleotide probe comprising the oligonucleotide consisting of a sequence selected from the group consisting of SEQ ID NOS: 32-35, or the complement thereof.

5. The method of claim 3, wherein the oligonucleotide probe set includes probes consisting of the sequences as set forth in SEQ ID NOS: 10-35.

6. The method of any one of claims 3 to 5, wherein the target sequence is labeled with a detectable labeling material.

7. The method of claim 6, wherein the labeling material is a fluorescent material, a phosphorescent material, or a radioactive material.

8. The method of any one of claims 3 to 7, wherein the oligonucleotide probe set is immobilized on a microarray substrate.

## Patentansprüche

1. Oligonucleotidprimersatz zum Amplifizieren mindestens einer Zielsequenz eines 23S rRNA-Gens von mindestens einer Bakterienspezies, ausgewählt aus der Gruppe, bestehend aus Chlamydophila pneumoniae, Haemophilus influenzae, Mycoplasma pneumoniae, Klebsiella pneumoniae, Legionella pneumophila, Moraxella catarrhalis, Pseudomonas aeruginosa, Staphylococcus aureus und Streptococcus pneumoniae, umfassend: den Oligonucleotidsatz, umfassend das Oligonucleotid, bestehend aus mindestens 10 zusammenhängenden Nucleotiden der SEQ ID NO: 1, und das Oligonucleotid, bestehend aus mindestens 10 zusammenhängenden Nucleotiden der SEQ ID NO: 2; den Oligonucleotidsatz, umfassend das Oligonucleotid, bestehend aus mindestens 10 zusammenhängenden Nucleotiden der SEQ ID NO: 3, und das Oligonucleotid, bestehend aus mindestens 10 zusammenhängenden Nucleotiden der SEQ ID NO: 4; den Oligonucleotidsatz, umfassend das Oligonucleotid, bestehend aus mindestens 10 zusammenhängenden Nucleotiden der SEQ ID NO: 5, und das Oligonucleotid, bestehend aus mindestens 10 zusammenhängenden Nucleotiden einer Sequenz, ausgewählt aus der Gruppe, bestehend aus SEQ ID NO: 6 und 7; und den Oligonucleotidsatz, umfassend das Oligonucleotid, bestehend aus mindestens 10 zusammenhängenden Nucleotiden der SEQ ID NO: 8, und das Oligonucleotid, bestehend aus mindestens 10 zusammenhängenden Nucleotiden der SEQ ID NO: 9.

2. Oligonucleotidprimersatz nach Anspruch 1, welcher einen Oligonucleotidsatz, umfassend ein Oligonucleotid, bestehend aus SEQ ID NO: 1, und ein Oligonucleotid, bestehend aus SEQ ID NO: 2; einen Oligonucleotidsatz, umfassend ein Oligonucleotid, bestehend aus SEQ ID NO: 3, und ein Oligonucleotid, bestehend aus SEQ ID NO: 4; einen Oligonucleotidsatz, umfassend ein Oligonucleotid, bestehend aus SEQ ID NO: 5, und ein Oligonucleotid, bestehend aus SEQ ID NO: 6 oder SEQ ID NO: 7; und einen Oligonucleotidsatz, umfassend ein Oligonucleotid, bestehend aus SEQ ID NO: 8, und ein Oligonucleotid, bestehend aus SEQ ID NO: 9, umfasst.

3. Verfahren zum Nachweisen einer Bakterienspezies, ausgewählt aus der Gruppe, bestehend aus Chlamydophila pneumoniae, Haemophilus influenzae, Klebsiella pneumoniae, Legionella pneumophila, Moraxella catarrhalis, Mycoplasma pneumoniae, Pseudomonas aeruginosa, Staphylococcus aureus und Streptococcus pneumoniae, wobei das Verfahren umfasst:
a) Durchführen einer Multiplex-PCR unter Verwendung von Matrizen-Nucleinsäure, erhalten von einer Bakterienspezies, ausgewählt aus der Gruppe, bestehend aus Chlamydophila pneumoniae, Haemophilus influenzae, Klebsiella pneumoniae, Legionella pneumophila, Moraxella catarrhalis, Mycoplasma pneumoniae, Pseudomonas aeruginosa, Staphylococcus aureus und Streptococcus pneumoniae, und eines Oligonucleotidprimersatzes, umfassend den Oligonucleotidsatz nach Anspruch 1 oder 2;
b) Erhalten einer Probe, umfassend das PCR-Produkt von Schritt a);
c) In-Kontakt-Bringen der Probe mit einem Oligonucleotidsondensatz, so dass eine Oligonucleotidsonde mit einer in der Probe vorhandenen Zielsequenz hybridisiert; und
d) Nachweisen eines Hybridisierungsgrades zwischen der Oligonucleotidsonde und der Zielsequenz, wobei der Oligonucleotidsondensatz mindestens eine Oligonucleotidsonde umfasst, ausgewählt aus der Gruppe, bestehend aus:
einer Oligonucleotidsonde, umfassend ein Oligonucleotid, bestehend aus mindestens 10 zusammenhängenden Nucleotiden der SEQ ID NO: 10, oder ein Komplement des Oligonucleotids;
einer Oligonucleotidsonde, umfassend ein Oligonucleotid, bestehend aus mindestens 10 zusammenhängenden Nucleotiden einer Sequenz, ausgewählt aus der Gruppe, bestehend aus SEQ ID NO: 11 - 14, oder ein Komplement des Oligonucleotids;
einer Oligonucleotidsonde, umfassend ein Oligonucleotid, bestehend aus mindestens 10 zusammenhängenden Nucleotiden der SEQ ID NO: 15, oder ein Komplement des Oligonucleotids;
einer Oligonucleotidsonde, umfassend ein Oligonucleotid, bestehend aus mindestens 10 zusammenhängenden Nucleotiden einer Sequenz, ausgewählt aus der Gruppe, bestehend aus SEQ ID NO: 16 - 18, oder ein Komplement des Oligonucleotids;
einer Oligonucleotidsonde, umfassend ein Oligonucleotid, bestehend aus mindestens 10 zusammenhängenden Nucleotiden der SEQ ID NO: 19, oder ein Komplement des Oligonucleotids;
einer Oligonucleotidsonde, umfassend ein Oligonucleotid, bestehend aus mindestens 10 zusammenhängenden Nucleotiden der SEQ ID NO: 20, oder ein Komplement des Oligonucleotids;
einer Oligonucleotidsonde, umfassend ein Oligonucleotid, bestehend aus mindestens 10 zusammenhängenden Nucleotiden einer Sequenz, ausgewählt aus der Gruppe, bestehend aus SEQ ID NO: 21 - 23, oder ein Komplement des Oligonucleotids;
einer Oligonucleotidsonde, umfassend ein Oligonucleotid, bestehend aus mindestens 10 zusammenhängenden Nucleotiden einer Sequenz, ausgewählt aus der Gruppe, bestehend aus SEQ ID NO: 24 - 26, oder ein Komplement des Oligonucleotids;
einer Oligonucleotidsonde, umfassend ein Oligonucleotid, bestehend aus mindestens 10 zusammenhängenden Nucleotiden der SEQ ID NO: 27, oder ein Komplement des Oligonucleotids;
einer Oligonucleotidsonde, umfassend ein Oligonucleotid, bestehend aus mindestens 10 zusammenhängenden Nucleotiden der SEQ ID NO: 28, oder ein Komplement des Oligonucleotids;
einer Oligonucleotidsonde, umfassend ein Oligonucleotid, bestehend aus mindestens 10 zusammenhängenden Nucleotiden der SEQ ID NO: 29, oder ein Komplement des Oligonucleotids; einer Oligonucleotidsonde, umfassend ein Oligonucleotid, bestehend aus mindestens 10 zusammenhängenden Nucleotiden einer Sequenz, ausgewählt aus der Gruppe, bestehend aus SEQ ID NO: 30 und 31, oder ein Komplement des Oligonucleotids;
und einer Oligonucleotidsonde, umfassend ein Oligonucleotid, bestehend aus mindestens 10 zusammenhängenden Nucleotiden einer Sequenz, ausgewählt aus der Gruppe, bestehend aus SEQ ID NO: 32 - 35, oder ein Komplement des Oligonucleotids.

4. Verfahren nach Anspruch 3, wobei der Oligonucleotidsondensatz eine Oligonucleotidsonde umfasst, ausgewählt aus der Gruppe, bestehend aus:
der Oligonucleotidsonde, umfassend das Oligonucleotid, bestehend aus SEQ ID NO: 10, oder das Komplement davon;
der Oligonucleotidsonde, umfassend das Oligonucleotid, bestehend aus einer Sequenz, ausgewählt aus der Gruppe, bestehend aus SEQ ID NO: 11 - 14, oder das Komplement davon;
der Oligonucleotidsonde, umfassend das Oligonucleotid, bestehend aus SEQ ID NO: 15, oder das Komplement davon; der Oligonucleotidsonde, umfassend das Oligonucleotid, bestehend aus einer Sequenz, ausgewählt aus der Gruppe, bestehend aus SEQ ID NO: 16 - 18, oder das Komplement davon;
der Oligonucleotidsonde, umfassend das Oligonucleotid, bestehend aus SEQ ID NO: 19, oder das Komplement davon;
der Oligonucleotidsonde, umfassend das Oligonucleotid, bestehend aus SEQ ID NO: 20, oder das Komplement davon;
der Oligonucleotidsonde, umfassend das Oligonucleotid, bestehend aus einer Sequenz, ausgewählt aus der Gruppe, bestehend aus SEQ ID NO: 21 - 23, oder das Komplement davon;
der Oligonucleotidsonde, umfassend das Oligonucleotid, bestehend aus einer Sequenz, ausgewählt aus der Gruppe, bestehend aus SEQ ID NO: 24 - 26, oder das Komplement davon;
der Oligonucleotidsonde, umfassend das Oligonucleotid, bestehend aus SEQ ID NO: 27, oder das Komplement davon;
der Oligonucleotidsonde, umfassend das Oligonucleotid, bestehend aus SEQ ID NO: 28, oder das Komplement davon;
der Oligonucleotidsonde, umfassend das Oligonucleotid, bestehend aus SEQ ID NO: 29, oder das Komplement davon;
der Oligonucleotidsonde, umfassend das Oligonucleotid, bestehend aus einer Sequenz, ausgewählt aus der Gruppe, bestehend aus SEQ ID NO: 30 und 31, oder das Komplement davon; und
der Oligonucleotidsonde, umfassend das Oligonucleotid, bestehend aus einer Sequenz, ausgewählt aus der Gruppe, bestehend aus SEQ ID NO: 32 - 35, oder das Komplement davon.

5. Verfahren nach Anspruch 3, wobei der Oligonucleotidsondensatz Sonden einschließt, die aus den in SEQ ID NO: 10 - 35 dargelegten Sequenzen bestehen.

6. Verfahren nach einem der Ansprüche 3 bis 5, wobei die Zielsequenz mit einem nachweisbaren Markierungsmaterial markiert ist.

7. Verfahren nach Anspruch 6, wobei es sich bei dem Markierungsmaterial um ein fluoreszierendes Material, ein phosphoreszierendes Material oder ein radioaktives Material handelt.

8. Verfahren nach einem der Ansprüche 3 bis 7, wobei der Oligonucleotidsondensatz auf einem Mikroarray-Substrat immobilisiert ist.

## Revendications

1. Jeu d'amorces oligonucléotidiques pour amplifier au moins une séquence cible d'un gène d'ARNr 23S d'au moins une espèce bactérienne choisie dans le groupe constitué par Chlamydophila pneumoniae, Haemophilus influenzae, Mycoplasma pneumoniae, Klebsiella pneumoniae, Legionella pneumophila, Moraxella catarrhalis, Pseudomonas aeruginosa, Staphylococcus aureus, et Streptococcus pneumoniae, comprenant : le jeu d'oligonucléotides comprenant l'oligonucléotide constitué d'au moins 10 nucléotides contigus de SEQ ID NO: 1 et l'oligonucléotide constitué d'au moins 10 nucléotides contigus de SEQ ID NO: 2 ; le jeu d'oligonucléotides comprenant l'oligonucléotide constitué d'au moins 10 nucléotides contigus de SEQ ID NO: 3 et l'oligonucléotide constitué d'au moins 10 nucléotides contigus de SEQ ID NO: 4 ; le jeu d'oligonucléotides comprenant l'oligonucléotide constitué d'au moins 10 nucléotides contigus de SEQ ID NO: 5 et l'oligonucléotide constitué d'au moins 10 nucléotides contigus d'une séquence choisie dans le groupe constitué des SEQ ID NOS: 6 et 7 ; et le jeu d'oligonucléotides comprenant l'oligonucléotide constitué d'au moins 10 nucléotides contigus de SEQ ID NO: 8 et l'oligonucléotide constitué d'au moins 10 nucléotides contigus de SEQ ID NO: 9.

2. Jeu d'amorces oligonucléotidiques selon la revendication 1, qui comprend un jeu d'oligonucléotides comprenant un oligonucléotide constitué de SEQ ID NO: 1 et un oligonucléotide constitué de SEQ ID NO: 2 ; un jeu d'oligonucléotides comprenant un oligonucléotide constitué de SEQ ID NO: 3 et un oligonucléotide constitué de SEQ ID NO: 4 ; un jeu d'oligonucléotides comprenant un oligonucléotide constitué de SEQ ID NO: 5 et un oligonucléotide constitué de SEQ ID NO: 6 ou de SEQ ID NO: 7 ; et un jeu d'oligonucléotides comprenant un oligonucléotide constitué de SEQ ID NO: 8 et un oligonucléotide constitué de SEQ ID NO: 9.

3. Procédé de détection d'une espèce bactérienne choisie dans le groupe constitué par Chlamydophila pneumoniae, Haemophilus influenza, Klebsiella pneumoniae, Legionella pneumophila, Moraxella catarrhalis, Mycoplasma pneumoniae, Pseudomonas aeruginosa, Staphylococcus aureus, et Streptococcus pneumoniae, le procédé comprenant :
a) la réalisation d'une PCR multiplex à l'aide d'un acide nucléique matrice obtenu à partir d'une espèce bactérienne choisie dans le groupe constitué par Chlamydophila pneumoniae, Haemophilus influenza, Klebsiella pneumoniae, Legionella pneumophila, Moraxella catarrhalis, Mycoplasma pneumoniae, Pseudomonas aeruginosa, Staphylococcus aureus, et Streptococcus pneumoniae, et d'un jeu d'amorces oligonucléotidiques comprenant le jeu d'oligonucléotides selon la revendication 1 ou 2 ;
b) l'obtention d'un échantillon comprenant le produit de PCR de l'étape a) ;
c) la mise en contact de l'échantillon avec un jeu de sondes oligonucléotidiques de sorte qu'une sonde oligonucléotidique s'hybride avec une séquence cible présente dans l'échantillon ; et
d) la détection d'un degré d'hybridation entre la sonde oligonucléotidique et la séquence cible, où le jeu de sondes oligonucléotidiques comprend au moins une sonde oligonucléotidique choisie dans le groupe constitué par :
une sonde oligonucléotidique comprenant un oligonucléotide constitué d'au moins 10 nucléotides contigus de SEQ ID NO: 10 ou un complément de l'oligonucléotide ;
une sonde oligonucléotidique comprenant un oligonucléotide constitué d'au moins 10 nucléotides contigus d'une séquence choisie dans le groupe constitué par les SEQ ID NOS: 11-14 ou un complément de l'oligonucléotide ;
une sonde oligonucléotidique comprenant un oligonucléotide constitué d'au moins 10 nucléotides contigus de SEQ ID NO: 15 ou un complément de l'oligonucléotide ;
une sonde oligonucléotidique comprenant un oligonucléotide constitué d'au moins 10 nucléotides contigus d'une séquence choisie dans le groupe constitué par les SEQ ID NOS: 16-18 ou un complément de l'oligonucléotide ;
une sonde oligonucléotidique comprenant un oligonucléotide constitué d'au moins 10 nucléotides contigus de SEQ ID NO: 19 ou un complément de l'oligonucléotide ;
une sonde oligonucléotidique comprenant un oligonucléotide constitué d'au moins 10 nucléotides contigus de SEQ ID NO: 20 ou un complément de l'oligonucléotide ;
une sonde oligonucléotidique comprenant un oligonucléotide constitué d'au moins 10 nucléotides contigus d'une séquence choisie dans le groupe constitué par les SEQ ID NOS: 21-23 ou un complément de l'oligonucléotide ;
une sonde oligonucléotidique comprenant un oligonucléotide constitué d'au moins 10 nucléotides contigus d'une séquence choisie dans le groupe constitué par les SEQ ID NOS: 24-26 ou un complément de l'oligonucléotide ;
une sonde oligonucléotidique comprenant un oligonucléotide constitué d'au moins 10 nucléotides contigus de SEQ ID NO: 27 ou un complément de l'oligonucléotide ;
une sonde oligonucléotidique comprenant un oligonucléotide constitué d'au moins 10 nucléotides contigus de SEQ ID NO: 28 ou un complément de l'oligonucléotide ;
une sonde oligonucléotidique comprenant un oligonucléotide constitué d'au moins 10 nucléotides contigus de SEQ ID NO: 29 ou un complément de l'oligonucléotide ;
une sonde oligonucléotidique comprenant un oligonucléotide constitué d'au moins 10 nucléotides contigus d'une séquence choisie dans le groupe constitué par les SEQ ID NOS: 30 et 31 ou un complément de l'oligonucléotide ;
et une sonde oligonucléotidique comprenant un oligonucléotide constitué d'au moins 10 nucléotides contigus d'une séquence choisie dans le groupe constitué par les SEQ ID NOS: 32-35 ou un complément de l'oligonucléotide.

4. Procédé selon la revendication 3, dans lequel le jeu de sondes oligonucléotidiques comprend une sonde oligonucléotidique choisie dans le groupe constitué par :
la sonde oligonucléotidique comprenant l'oligonucléotide constitué de SEQ ID NO: 10, ou son complément ;
la sonde oligonucléotidique comprenant l'oligonucléotide constitué d'une séquence choisie dans le groupe constitué par les SEQ ID NOS: 11-14, ou son complément ;
la sonde oligonucléotidique comprenant l'oligonucléotide constitué de SEQ ID NO: 15, ou son complément ;
la sonde oligonucléotidique comprenant l'oligonucléotide constitué d'une séquence choisie dans le groupe constitué par les SEQ ID NOS: 16-18, ou son complément ;
la sonde oligonucléotidique comprenant l'oligonucléotide constitué de SEQ ID NO: 19, ou son complément ;
la sonde oligonucléotidique comprenant l'oligonucléotide constitué de SEQ ID NO: 20, ou son complément ;
la sonde oligonucléotidique comprenant l'oligonucléotide constitué d'une séquence choisie dans le groupe constitué par les SEQ ID NOS: 21-23, ou son complément ;
la sonde oligonucléotidique comprenant l'oligonucléotide constitué d'une séquence choisie dans le groupe constitué par les SEQ ID NOS: 24-26, ou son complément ;
la sonde oligonucléotidique comprenant l'oligonucléotide constitué de SEQ ID NO: 27, ou son complément ;
la sonde oligonucléotidique comprenant l'oligonucléotide constitué de SEQ ID NO: 28, ou son complément ;
la sonde oligonucléotidique comprenant l'oligonucléotide constitué de SEQ ID NO: 29, ou son complément ;
la sonde oligonucléotidique comprenant l'oligonucléotide constitué d'une séquence choisie dans le groupe constitué par les SEQ ID NOS: 30 et 31, ou son complément ; et
la sonde oligonucléotidique comprenant l'oligonucléotide constitué d'une séquence choisie dans le groupe constitué par les SEQ ID NOS: 32-35, ou son complément.

5. Procédé selon la revendication 3, dans lequel le jeu de sondes oligonucléotidiques inclut des sondes constituées des séquences telles que présentées dans les SEQ ID NOS: 10-35.

6. Procédé selon l'une quelconque des revendications 3 à 5, dans lequel la séquence cible est marquée avec un matériau de marquage détectable.

7. Procédé selon la revendication 6, dans lequel le matériau de marquage est un matériau fluorescent, un matériau phosphorescent, ou un matériau radioactif.

8. Procédé selon l'une quelconque des revendications 3 à 7, dans lequel le jeu de sondes oligonucléotidiques est immobilisé sur un substrat à microréseau.
